# EUROPEAN PATENT APPLICATION

(11) **EP 4 851 304 A1**
(43) Date of publication of application: **09.12.2026**
(21) Application number: 25181195.6
(22) Date of filing: 05.06.2025
(51) Int. Cl.: C12Q 1/6848

(54) **DNA AMPLIFICATION AND PRODUCTION METHODS**

(71) Applicant: 4basebio UK Ltd, Cambridge CB24 5QE (GB)
(72) Inventor: LANCKRIET, Heikki, Cambridge CB24 5QE (GB); WALKER, Amy, Cambridge CB24 5QE (GB); PAVLICKOVA, Milena, Cambridge CB24 5QE (GB); BRUCE, Caitlyn, Cambridge CB24 5QE (GB)
(74) Representative: Boult Wade Tennant LLP

(57) **Abstract**

The invention relates to methods for amplifying a DNA template molecule by rolling circle amplification in the presence of a primase/polymerase and a single-stranded DNA binding protein that enhances the efficiency of amplification, as well as related methods for producing a linear DNA product of increased resistance to nuclease digestion. Further disclosed are linear DNA products produced by the methods described herein and uses thereof.

## Description

### TECHNICAL FIELD

The invention relates to methods for amplifying a DNA template molecule in the presence of a single-stranded DNA binding protein. The invention also relates to methods for producing a linear DNA product of increased resistance to nuclease digestion. The invention also relates to linear DNA products produced by the methods described herein and uses thereof.

### BACKGROUND

A variety of methods have been developed to produce stable synthetic DNA. Larger DNA molecules may be produced using a variety of amplification technologies e.g. rolling circle amplification (RCA). Methods have also been developed to improve the stability of DNA molecules by providing resistance to nuclease digestion. This may be accomplished by producing closed linear DNA molecules. For example, WO2010/086626 describes a method for producing a closed linear DNA by utilizing a protelomerase. However, this method is limited in that the action of protelomerase produces the same sequence at both ends of the closed linear DNA molecule. Alternative methods are described in WO2022/229460 and WO2023/006978. The methods described in WO2022/229460 include methods for the production of linear double-stranded DNA products that comprise amplification of a DNA template molecule comprising at least one cleavable sequence in the presence of at least one type of a protected nucleotide to generate a double-stranded DNA product; and contacting the double-stranded DNA product with an enzyme that cleaves the cleavable sequence to generate the linear double-stranded DNA product that has increased resistance to nucleases. The methods described in WO2023/006978 include methods for producing a DNA product with enhanced resistance to nuclease digestion based on ligating adaptor molecules to a double-stranded DNA molecule.

In the context of synthetic DNA that can be used in therapy, a need exists for methods which produce synthetic DNA of high stability, homogeneity and/or purity.

### DESCRIPTION

The inventors have developed methods for amplifying a DNA template molecule. The methods of the invention comprise rolling circle amplification in the presence of a single-stranded DNA binding protein. The single-stranded binding protein may enhance the efficiency of amplification. The methods of the invention may comprise rolling circle amplification in the presence of a single-stranded DNA binding protein and a primase/polymerase. The single-stranded binding protein may enhance the efficiency of amplification.

The inventors have also developed methods for producing a linear deoxyribonucleic acid (DNA) product of increased resistance to nuclease (e.g. exonuclease) digestion. The methods for producing a linear DNA product of increased resistance to nuclease digestion typically incorporate the methods for amplifying a DNA template molecule. The methods of the invention may produce the linear DNA product in a single reaction volume (or single contiguous aqueous volume). The invention also relates to linear DNA products produced by the methods described herein and uses thereof. The invention addresses the need identified by the inventors for synthetic DNA production methods that produce homogenous and/or stable linear DNA products. The DNA products produced by the methods of the invention may have high purity.

The invention provides a method for amplifying a DNA template molecule, wherein the method comprises amplifying a DNA template molecule to generate a double-stranded DNA molecule, wherein the DNA template molecule is amplified by rolling circle amplification in the presence of (i) a single-stranded DNA binding protein, and (ii) a primase/polymerase.

The invention provides a method for amplifying a DNA template molecule, wherein the method comprises amplifying a DNA template molecule to generate a double-stranded DNA molecule, wherein the DNA template molecule is amplified by rolling circle amplification in the presence of (i) a single-stranded DNA binding protein that enhances the efficiency of amplification, and (ii) a primase/polymerase.

The inventors found that rolling circle amplification in the presence of (i) a single-stranded DNA binding protein that enhances the efficiency of amplification, and (ii) a primase/polymerase produces a stable and homogenous amplification product. Preferably, the amplification product is a double-stranded linear DNA product. The inventors found that rolling circle amplification in the presence of (i) a single-stranded DNA binding protein that enhances the efficiency of amplification, and (ii) a primase/polymerase produces minimal (if any) amounts of a single stranded DNA product. The methods of the invention improve the stability, homogeneity and purity of the DNA product.

The DNA template molecule may comprise at least one endonuclease target sequence so that the amplification product (e.g. the double-stranded DNA molecule) may be further processed to produce a linear DNA product. Thus, the invention provides a method for producing a linear DNA product, wherein the method comprises:
(a) amplifying a DNA template molecule comprising at least one endonuclease target sequence to generate a double-stranded DNA molecule, wherein the DNA template molecule is amplified by rolling circle amplification in the presence of (i) a single-stranded DNA binding protein, and (ii) a primase/polymerase; and
(b) contacting the double-stranded DNA molecule with an endonuclease to generate the linear DNA product.

The invention provides a method for producing a linear DNA product, wherein the method comprises:
(a) amplifying a DNA template molecule comprising at least one endonuclease target sequence to generate a double-stranded DNA molecule, wherein the DNA template molecule is amplified by rolling circle amplification in the presence of (i) a single-stranded DNA binding protein that enhances the efficiency of amplification, and (ii) a primase/polymerase; and
(b) contacting the double-stranded DNA molecule with an endonuclease to generate the linear DNA product.

Preferably, the linear DNA product comprises a single DNA cassette. That is to say that the linear DNA product does not comprise tandem repeats of the DNA cassette (i.e. is not a concatemer).

The amplification product (e.g. the double-stranded DNA product) may be further processed to produce a linear DNA product of enhanced or improved resistance to nuclease (e.g. exonuclease) digestion. The linear DNA product of enhanced or improved resistance to nuclease (e.g. exonuclease) digestion may be a closed linear DNA product. That is to say that the linear DNA product may comprise a hairpin adaptor at each end. The linear DNA product of enhanced or improved resistance to nuclease (e.g. exonuclease) digestion may be a partially closed DNA product. That is to say that the linear DNA product may comprise a hairpin adaptor at the first end and an adaptor comprising nuclease-resistant nucleotide(s) at the second end. The linear DNA product of enhanced or improved resistance to nuclease (e.g. exonuclease) digestion may be a linear DNA product. That is to say that the linear DNA product may comprise an adaptor that comprises nuclease-resistant nucleotide(s) at each end of the linear DNA product. Alternatively, the linear DNA product may comprise nuclease-resistant nucleotide(s) at different location(s) in sense and/or antisense strands.

Thus, the invention provides a method for producing a closed linear DNA product, wherein the method comprises:
(a) amplifying a DNA template molecule comprising at least one endonuclease target sequence to generate a double-stranded DNA molecule, wherein the DNA template molecule is amplified by rolling circle amplification in the presence of (i) a single-stranded DNA binding protein, and (ii) a primase/polymerase;
(b) contacting the double-stranded DNA molecule with an endonuclease, a ligase and first and second adaptor molecules to form a single contiguous aqueous volume; and
(c) incubating the single contiguous aqueous volume to generate the closed linear DNA product,
wherein the closed linear DNA product comprises a linear double-stranded region, wherein the linear double-stranded region comprises a linear portion of the double-stranded DNA molecule, and wherein the linear double-stranded region is closed at a first end by the first adaptor molecule and closed at a second end by the second adaptor molecule.

The invention provides a method for producing a closed linear DNA product, wherein the method comprises:
(a) amplifying a DNA template molecule comprising at least one endonuclease target sequence to generate a double-stranded DNA molecule, wherein the DNA template molecule is amplified by rolling circle amplification in the presence of (i) a single-stranded DNA binding protein that enhances the efficiency of amplification, and (ii) a primase/polymerase;
(b) contacting the double-stranded DNA molecule with an endonuclease, a ligase and first and second adaptor molecules to form a single contiguous aqueous volume; and
(c) incubating the single contiguous aqueous volume to generate the closed linear DNA product,
wherein the closed linear DNA product comprises a linear double-stranded region, wherein the linear double-stranded region comprises a linear portion of the double-stranded DNA molecule, and wherein the linear double-stranded region is closed at a first end by the first adaptor molecule and closed at a second end by the second adaptor molecule.

The invention also provides a method for producing a linear DNA product, wherein the method comprises:
(a) amplifying a DNA template molecule comprising at least one endonuclease target sequence to generate a double-stranded DNA molecule, wherein the DNA template molecule is amplified by rolling circle amplification in the presence of (i) a single-stranded DNA binding protein, and (ii) a primase/polymerase;
(b) contacting the double-stranded DNA molecule with an endonuclease, a ligase and first and second adaptor molecules to form a single contiguous aqueous volume; and
(c) incubating the single contiguous aqueous volume to generate the linear DNA product,
wherein the linear DNA product comprises a linear double-stranded region, wherein the linear double-stranded region comprises a linear portion of the double-stranded DNA molecule, and wherein the first adaptor molecule is ligated to a first end of the linear double-stranded region and the second adaptor molecule is ligated to a second end of the linear double-stranded region, and wherein the first and second adaptor molecules are nucleic acid molecules that comprise one or more nuclease-resistant nucleotides.

The invention also provides a method for producing a linear DNA product, wherein the method comprises:
(a) amplifying a DNA template molecule comprising at least one endonuclease target sequence to generate a double-stranded DNA molecule, wherein the DNA template molecule is amplified by rolling circle amplification in the presence of (i) a single-stranded DNA binding protein that enhances the efficiency of amplification, and (ii) a primase/polymerase;
(b) contacting the double-stranded DNA molecule with an endonuclease, a ligase and first and second adaptor molecules to form a single contiguous aqueous volume; and
(c) incubating the single contiguous aqueous volume to generate the linear DNA product,
wherein the linear DNA product comprises a linear double-stranded region, wherein the linear double-stranded region comprises a linear portion of the double-stranded DNA molecule, and wherein the first adaptor molecule is ligated to a first end of the linear double-stranded region and the second adaptor molecule is ligated to a second end of the linear double-stranded region, and wherein the first and second adaptor molecules are nucleic acid molecules that comprise one or more nuclease-resistant nucleotides.

The invention also provides a method for producing a partially closed DNA product, wherein the method comprises:
(a) amplifying a DNA template molecule comprising at least one endonuclease target sequence to generate a double-stranded DNA molecule, wherein the DNA template molecule is amplified by rolling circle amplification in the presence of (i) a single-stranded DNA binding protein, and (ii) a primase/polymerase;
(b) contacting the double-stranded DNA molecule with an endonuclease, a ligase and first and second adaptor molecules to form a single contiguous aqueous volume; and
(c) incubating the single contiguous aqueous volume to generate the partially closed linear DNA product, wherein the partially closed linear DNA product comprises a linear double-stranded region,
wherein the linear double-stranded region comprises a linear portion of the double-stranded DNA molecule, and wherein the first adaptor molecule is ligated to a first end of the linear double-stranded region and the second adaptor molecule is ligated to a second end of the linear double-stranded region, and wherein the first adaptor molecule is a nucleic acid molecule that comprise one or more nuclease-resistant nucleotides, and wherein the linear double-stranded region is closed at the second end by the second adaptor molecule.

The invention also provides a method for producing a partially closed DNA product, wherein the method comprises:
(a) amplifying a DNA template molecule comprising at least one endonuclease target sequence to generate a double-stranded DNA molecule, wherein the DNA template molecule is amplified by rolling circle amplification in the presence of (i) a single-stranded DNA binding protein that enhances the efficiency of amplification, and (ii) a primase/polymerase;
(b) contacting the double-stranded DNA molecule with an endonuclease, a ligase and first and second adaptor molecules to form a single contiguous aqueous volume; and
(c) incubating the single contiguous aqueous volume to generate the partially closed linear DNA product, wherein the partially closed linear DNA product comprises a linear double-stranded region,
wherein the linear double-stranded region comprises a linear portion of the double-stranded DNA molecule, and wherein the first adaptor molecule is ligated to a first end of the linear double-stranded region and the second adaptor molecule is ligated to a second end of the linear double-stranded region, and wherein the first adaptor molecule is a nucleic acid molecule that comprise one or more nuclease-resistant nucleotides, and wherein the linear double-stranded region is closed at the second end by the second adaptor molecule.

The invention also provides a method for producing a linear double-stranded DNA product, wherein the method comprises:
(a) rolling circle amplification of a DNA template molecule comprising at least one endonuclease target sequence in the presence of: (i) at least one type of a nuclease-resistant nucleotide; and (ii) a single-stranded DNA binding protein, to generate a double-stranded DNA product,
(b) contacting the double-stranded DNA product with at least one endonuclease to generate the linear double-stranded DNA product, wherein the linear double-stranded DNA product has increased resistance to nuclease digestion.

Preferably, in step (a) a primase/polymerase is also present.

The invention also provides a method for producing a linear double-stranded DNA product, wherein the method comprises:
(a) rolling circle amplification of a DNA template molecule comprising at least one endonuclease target sequence in the presence of: (i) at least one type of a nuclease-resistant nucleotide; and (ii) a single-stranded DNA binding protein that enhances the efficiency of amplification, to generate a double-stranded DNA product,
(b) contacting the double-stranded DNA product with at least one endonuclease to generate the linear double-stranded DNA product, wherein the linear double-stranded DNA product has increased resistance to nuclease digestion.

The expressions "increased resistance to nuclease digestion" or "enhanced resistance to nuclease digestion" are intended to encompass linear DNA products which may have partial or full resistance to nuclease digestion (e.g. exonuclease digestion). The enhanced resistance to nuclease (e.g. exonuclease) digestion may extend the life of the linear DNA product in a cell (i.e. the linear DNA product has enhanced resistance to intracellular exonucleases) and in a cell-free system (i.e. the linear DNA product has enhanced resistance to extracellular exonucleases). The enhanced resistance to exonuclease digestion may be provided by appending (e.g. ligating) a first adaptor molecule to a first end of the linear double-stranded region and a second adaptor to a second end of the linear double stranded region. In this regard, the entire content of WO2023/006978 A1 is incorporated herein by reference. The enhanced resistance to exonuclease digestion may be provided by incorporating (exo)nuclease-resistant nucleotides into the double-stranded DNA molecule during RCA. In this regard, the entire content of WO2022/229460 A1 is incorporated herein by reference.

Without wishing to be bound by a particular theory, it is believed that the improved stability, homogeneity and/or purity of the DNA product produced by the methods of the invention is the result of the combination of (1) a rolling circle amplification, which, preferably, uses a highly processive (i.e. strand displacing) DNA polymerase, (2) a single-stranded DNA binding protein that enhances the efficiency of amplification; and (3) (optionally) a primase/polymerase.

The expression "single-stranded DNA binding protein that enhances the efficiency of amplification" as used herein is intended to encompass any single-stranded DNA binding proteins, which function includes one or more of: (1) helix destabilization, (2) reduction of the formation of secondary DNA structures, (3) prevention of the degradation of ssDNA by nucleases, (4) prevention of the inhibition of amplification by template DNA contaminants, (5) improvement of the efficiency of DNA amplification by DNA polymerase, (6) stabilization of single-stranded regions of DNA for site-specific mutagenesis, (7) facilitation of completion of restriction enzyme digestion, (8) improvement of the efficiency of DNA synthesis by DNA polymerase, and (9) enhancement of the fidelity of DNA polymerase. Single-stranded DNA binding proteins (SSB) are canonically part of the DNA replication process in viruses, prokaryotes and eukaryotes and stabilize ssDNA regions during replication process. They are also loaded onto DNA independently of any other DNA replication machinery and are eventually displaced by replicative polymerase as it traverses DNA strand. Once displaced, soluble SSB is then available for further binding with ssDNA strands. The inventors found that presence of SSB proteins during the DNA amplification (by RCA) promotes existence of DNA template as interspersed ssDNA and SSB-bound ssDNA regions which are easily accessible to both priming and DNA amplification machinery. In particular, the inventors discovered that the single-stranded DNA binding protein used during amplification transiently binds ssDNA template and minimizes the formation of highly stable ssDNA secondary structures that could hinder DNA amplification. By way of explanation, in most amplification approaches, double stranded DNA template molecule is first converted into ssDNA through a denaturation step. ssDNA is the template that enables both priming and DNA amplification machinery to bind and is therefore one of the key limiting factors in DNA amplification origin formation. However, the ssDNA form is naturally unstable and partially reverses back into dsDNA (renaturation) as the DNA amplification reaction progresses. Renaturation of ssDNA can take place in form of ssDNA self-annealing which can form small loop domains or non-B DNA structures such as G-quadruplexes, Z-DNA or H-DNA structures which are highly stable, inaccessible and can be difficult to melt by DNA polymerase strand displacement. Large GC-rich constructs, constructs with repeated sequences, constructs with homopolymer regions and other highly complex regions are the constructs that are most likely to form the non-B DNA structures in a DNA template. Formation of such secondary structures on the template can result in DNA polymerase stalling and skipping regions of the template or completely aborting the replication of the current template strand. Similarly, canonical dsDNA duplexes can start reforming which prevents efficient priming and replication machinery loading. Thus, the use of the single-stranded DNA binding protein in the methods of the invention addresses this issue by transiently binding to the ssDNA template and minimizing the formation of highly stable ssDNA secondary structures that could hinder DNA amplification.

The single-stranded DNA binding protein may be bacteriophage T4 Gene 32 Protein (gp32), *Thermus thermophilus* SSB protein (TthSSB), *Thermus aquaticus* SSB protein (TaqSSB), *E.coli* SSB (EcoSSB), and/or Bacteriophage Phi29 protein 5 (p5) protein.

Preferably, the single-stranded DNA binding protein is bacteriophage T4 Gene 32 Protein (gp32).

Bacteriophage T4 Gene 32 Protein is a single-stranded DNA (ssDNA) binding protein required for bacteriophage T4 replication and repair. It cooperatively binds to and stabilizes transiently formed regions of ssDNA and plays an important structural role during T4 phage replication. It also has been used to stabilize and mark regions of ssDNA for electron microscopic examination of intracellular DNA structures. The bacteriophage T4 Gene 32 Protein has a UniProt accession number P03695.

The single-stranded DNA binding protein may have a sequence of at least 80%, at least 85%, at least 90%, at least 95%, at least 97%, at least 98%, or at least 99% identity to SEQ ID NO: 5. The single-stranded DNA binding protein may have a sequence of SEQ ID NO: 5.

The expression "enhances the efficiency of amplification" as used herein is understood to mean an improvement to one or more steps and/or events taking places during amplification of a DNA template molecule, which overall results in a more efficient amplification of the DNA template molecule. The expression "more efficient" may mean that more DNA product is produced, or that amplification requires less of the DNA template molecule, or that amplification uses less reagents, or that amplification is performed over a shorter period of time / lower temperature, or that less contaminants are produced (e.g. single stranded DNA). For example, the expression "enhances the efficiency of amplification" may mean one or more of (1) an improvement in helix destabilization, (2) reduction of the formation of secondary DNA structures, (3) prevention of the degradation of ssDNA by nucleases, (4) prevention of the inhibition of amplification by template DNA contaminants, (5) improvement of the efficiency of DNA amplification by DNA polymerase, (6) stabilization of single-stranded regions of DNA for site-specific mutagenesis, (7) facilitation of completion of restriction enzyme digestion, (8) improvement of the efficiency of DNA synthesis by DNA polymerase, and (9) enhancement of the fidelity of DNA polymerase.

The inventors discovered that the single-stranded DNA binding protein at a concentration of less than 50 ng/µL is particularly successful in producing the linear DNA product. Thus, the single-stranded DNA binding protein may be at a concentration of less than 50 ng/µL. The single-stranded DNA binding protein may be a concentration of between 1 ng/µL and less than 50 ng/µL. The single-stranded DNA binding protein may be at a concentration of between 4 ng/µL and 50 ng/µL, between 4 ng/µL and 40 ng/µL, between 4 ng/µL and 30 ng/µL, between 4 ng/µL and 25 ng/µL, or between 10 ng/µL and 25 ng/µL. The single-stranded DNA binding protein may be at a concentration of between 4 ng/µL and 50 ng/µL, between 4 ng/µL and 25 ng/µL, or between 4 ng/µL and 30 ng/µL. Preferably, the single-stranded DNA binding protein may be at a concentration of between 4 ng/µL and 25 ng/µL. The single-stranded DNA binding protein may be at a concentration of about 25 ng/µL.

The term "about" as used herein for numerical data refers to a value within 10% of the underlying parameter (i.e., plus or minus 10%), and use of the term "about" at the beginning of a string of values modifies each of the values (i.e., "about 1, 2 and 3" refers to about 1, about 2 and about 3). For example, a concentration of "about 25 ng/µL" may include concentrations between (and including) 22.5 ng/µL and 27.5 ng/µL.

In the embodiments in which the single-stranded DNA binding protein is bacteriophage T4 Gene 32 Protein (gp32), the bacteriophage T4 Gene 32 Protein may be at a concentration of less than 50 ng/µL. The bacteriophage T4 Gene 32 Protein may be a concentration of between 1 ng/µL and less than 50 ng/µL. The bacteriophage T4 Gene 32 Protein may be at a concentration of between 4 ng/µL and 40 ng/µL, between 4 ng/µL and 30 ng/µL, between 4 ng/µL and 25 ng/µL, or between 10 ng/µL and 25 ng/µL. The bacteriophage T4 Gene 32 Protein may be at a concentration of between 4 ng/µL and 50 ng/µL, or between 4 ng/µL and 30 ng/µL. The bacteriophage T4 Gene 32 Protein may be at a concentration of about 25 ng/µL. Preferably, the bacteriophage T4 Gene 32 Protein is at a concentration of 25 ng/µL. Preferably, the bacteriophage T4 Gene 32 Protein is at a concentration of between 4 ng/µL and 25 ng/µL.

Thus, the invention provides a method for amplifying a DNA template molecule, wherein the method comprises amplifying a DNA template molecule to generate a double-stranded DNA molecule, wherein the DNA template molecule is amplified by rolling circle amplification in the presence of (i) bacteriophage T4 Gene 32 Protein, and (ii) a primase/polymerase.

The invention provides a method for amplifying a DNA template molecule, wherein the method comprises amplifying a DNA template molecule to generate a double-stranded DNA molecule, wherein the DNA template molecule is amplified by rolling circle amplification in the presence of (i) bacteriophage T4 Gene 32 Protein at a concentration of less than 50 ng/µL, and (ii) a primase/polymerase.

The invention provides a method for amplifying a DNA template molecule, wherein the method comprises amplifying a DNA template molecule to generate a double-stranded DNA molecule, wherein the DNA template molecule is amplified by rolling circle amplification in the presence of (i) bacteriophage T4 Gene 32 Protein at a concentration of between 4 ng/µL and less than 50 ng/µL, and (ii) a primase/polymerase.

The invention provides a method for amplifying a DNA template molecule, wherein the method comprises amplifying a DNA template molecule to generate a double-stranded DNA molecule, wherein the DNA template molecule is amplified by rolling circle amplification in the presence of (i) bacteriophage T4 Gene 32 Protein at a concentration of between 4 ng/µL and 25 ng/µL, and (ii) a primase/polymerase.

The invention provides a method for amplifying a DNA template molecule, wherein the method comprises amplifying a DNA template molecule to generate a double-stranded DNA molecule, wherein the DNA template molecule is amplified by rolling circle amplification in the presence of (i) bacteriophage T4 Gene 32 Protein at a concentration of 25 ng/µL, and (ii) a primase/polymerase.

The expression "primase/polymerase" as used herein is intended to encompass enzymes which have the activity of both primase and DNA polymerase. The expression "primase/polymerase" is intended to encompass enzymes that can start *in vitro* RNA/DNA synthesis and/or DNA extension. The primase/polymerase does not require the use of a primer in an amplification reaction. For example, the primase/polymerase may be TthPrimPol. The TthPrimPol protein comes from Thermophilic bacterium *T. Thermophilus.* This protein has DNA-dependent activity to synthesize DNA Primer (Primase), and its recognition sequence is 3'- GTCC-5'. In the presence of magnesium ions, it can use ATP or dATP as the coenzyme to synthesize 7-9 nucleotide DNA Primer. TthPrimPol is described in detail in Picher et al. (Picher et al., (2016) "TruePrime is a novel method for whole-genome amplification from single cells based on TthPrimPol", Nature Communications, 7(1):13296), which is incorporated herein by reference in its entirety. The primase/polymerase may be the PrimPol described in WO2025/012454, which is incorporated herein by reference in its entirety. The primase/polymerase may have a sequence of at least 80%, at least 85%, at least 90%, at least 95%, at least 97%, at least 98%, or at least 99% identity to SEQ ID NO: 1. The primase/polymerase may have a sequence of at least 80%, at least 85%, at least 90%, at least 95%, at least 97%, at least 98%, or at least 99% identity to SEQ ID NO: 2. The primase/polymerase may have a sequence of SEQ ID NO:1. The primase/polymerase may have a sequence of SEQ ID NO: 2. The primase/polymerase may have a sequence of at least 80%, at least 85%, at least 90%, at least 95%, at least 97%, at least 98%, or at least 99% identity to SEQ ID NO: 3. The primase/polymerase may have a sequence of at least 80%, at least 85%, at least 90%, at least 95%, at least 97%, at least 98%, or at least 99% identity to SEQ ID NO: 4. The primase/polymerase may have a sequence of SEQ ID NO: 3. The primase/polymerase may have a sequence of SEQ ID NO: 4. The expression "primase/polymerase" may interchangeably be used with the expressions "polymerase that has primase activity" or "DNA primase".

Preferably, the primase/polymerase is TthPrimPol.

The step of amplification may be performed in the presence of a DNA polymerase. Preferably, the DNA polymerase is a highly processive (i.e. strand displacing) DNA polymerase. The inventors discovered that the combination of a primase/polymerase, single-stranded DNA binding protein and a highly processive DNA polymerase is particularly beneficial in the context of the methods of the present invention as this combination delivers the most stable and homogenous linear DNA product. In other words, the purity of the final reaction is the highest.

The expression "highly processive DNA polymerase" as used herein is intended to encompass DNA polymerases which bind and extend a DNA template molecule with longer binding events as compared to standard DNA polymerases. The expression "highly processive DNA polymerase" is intended to encompass strand displacing DNA polymerases. For example, a highly processive DNA polymerase may be a Phi29 DNA polymerase, or a chimeric form of Phi29 DNA polymerase (i.e. QualiPhi DNA polymerase).

The DNA polymerase may be a wild-type DNA polymerase, or a chimeric form of a wild-type DNA polymerase.

The DNA polymerase may be Phi29 DNA polymerase (e.g. wild-type Phi29 DNA polymerase) or a chimeric form of Phi29 DNA polymerase (e.g. QualiPhi DNA polymerase). Preferably, the DNA polymerase is QualiPhi DNA polymerase. The DNA polymerase may not be *Bsu* DNA polymerase.

Phi29 DNA polymerase is the replicative polymerase from the *Bacillus subtilis* phage phi29 (Φ29). Phi29 DNA polymerase has exceptional strand displacement and processive synthesis properties. Phi29 DNA polymerase has an inherent 3'→5' proofreading exonuclease activity. QualiPhi DNA Polymerase is a highly processive chimeric form of Phi29 DNA polymerase, engineered for enhanced sensitivity and efficiency of amplification.

Thus, the invention provides a method for amplifying a DNA template molecule, wherein the method comprises amplifying a DNA template molecule to generate a double-stranded DNA molecule, wherein the DNA template molecule is amplified by rolling circle amplification in the presence of (i) a single-stranded DNA binding protein that enhances the efficiency of amplification, (ii) a primase/polymerase; and (iii) a DNA polymerase.

The invention provides a method for amplifying a DNA template molecule, wherein the method comprises amplifying a DNA template molecule to generate a double-stranded DNA molecule, wherein the DNA template molecule is amplified by rolling circle amplification in the presence of (i) a single-stranded DNA binding protein that enhances the efficiency of amplification, (ii) TthPrimPol; and (iii) a DNA polymerase.

The invention provides a method for amplifying a DNA template molecule, wherein the method comprises amplifying a DNA template molecule to generate a double-stranded DNA molecule, wherein the DNA template molecule is amplified by rolling circle amplification in the presence of (i) a single-stranded DNA binding protein that enhances the efficiency of amplification, (ii) a primase/polymerase; and (iii) a highly processive DNA polymerase (e.g. QualiPhi DNA polymerase).

The invention provides a method for amplifying a DNA template molecule, wherein the method comprises amplifying a DNA template molecule to generate a double-stranded DNA molecule, wherein the DNA template molecule is amplified by rolling circle amplification in the presence of (i) a single-stranded DNA binding protein that enhances the efficiency of amplification, (ii) TthPrimPol; and (iii) a highly processive DNA polymerase (e.g. QualiPhi DNA polymerase).

The invention provides a method for amplifying a DNA template molecule, wherein the method comprises amplifying a DNA template molecule to generate a double-stranded DNA molecule, wherein the DNA template molecule is amplified by rolling circle amplification in the presence of (i) bacteriophage T4 Gene 32 Protein, (ii) a primase/polymerase, and (iii) a DNA polymerase.

The invention provides a method for amplifying a DNA template molecule, wherein the method comprises amplifying a DNA template molecule to generate a double-stranded DNA molecule, wherein the DNA template molecule is amplified by rolling circle amplification in the presence of (i) bacteriophage T4 Gene 32 Protein, (ii) TthPrimPol, and (iii) a DNA polymerase.

The invention provides a method for amplifying a DNA template molecule, wherein the method comprises amplifying a DNA template molecule to generate a double-stranded DNA molecule, wherein the DNA template molecule is amplified by rolling circle amplification in the presence of (i) bacteriophage T4 Gene 32 Protein at a concentration of 25 ng/µL, (ii) a primase/polymerase, and (iii) a DNA polymerase.

The invention provides a method for amplifying a DNA template molecule, wherein the method comprises amplifying a DNA template molecule to generate a double-stranded DNA molecule, wherein the DNA template molecule is amplified by rolling circle amplification in the presence of (i) bacteriophage T4 Gene 32 Protein at a concentration of between 4 ng/µL and less than 50 ng/µL, (ii) a primase/polymerase, and (iii) a DNA polymerase.

The invention provides a method for amplifying a DNA template molecule, wherein the method comprises amplifying a DNA template molecule to generate a double-stranded DNA molecule, wherein the DNA template molecule is amplified by rolling circle amplification in the presence of (i) bacteriophage T4 Gene 32 Protein at a concentration of between 4 ng/µL and 25 ng/µL, (ii) a primase/polymerase, and (iii) a DNA polymerase.

The invention provides a method for amplifying a DNA template molecule, wherein the method comprises amplifying a DNA template molecule to generate a double-stranded DNA molecule, wherein the DNA template molecule is amplified by rolling circle amplification in the presence of (i) bacteriophage T4 Gene 32 Protein at a concentration of less than 50 ng/µL, (ii) a primase/polymerase, and (iii) a DNA polymerase.

The invention provides a method for amplifying a DNA template molecule, wherein the method comprises amplifying a DNA template molecule to generate a double-stranded DNA molecule, wherein the DNA template molecule is amplified by rolling circle amplification in the presence of (i) bacteriophage T4 Gene 32 Protein at a concentration of 25 ng/µL, (ii) TthPrimPol, and (iii) a DNA polymerase.

The invention provides a method for amplifying a DNA template molecule, wherein the method comprises amplifying a DNA template molecule to generate a double-stranded DNA molecule, wherein the DNA template molecule is amplified by rolling circle amplification in the presence of (i) bacteriophage T4 Gene 32 Protein at a concentration of 4 ng/µL and less than 50 ng/µL, (ii) TthPrimPol, and (iii) a DNA polymerase.

The invention provides a method for amplifying a DNA template molecule, wherein the method comprises amplifying a DNA template molecule to generate a double-stranded DNA molecule, wherein the DNA template molecule is amplified by rolling circle amplification in the presence of (i) bacteriophage T4 Gene 32 Protein at a concentration of 4 ng/µL and 25 ng/µL, (ii) TthPrimPol, and (iii) a DNA polymerase.

The invention provides a method for amplifying a DNA template molecule, wherein the method comprises amplifying a DNA template molecule to generate a double-stranded DNA molecule, wherein the DNA template molecule is amplified by rolling circle amplification in the presence of (i) bacteriophage T4 Gene 32 Protein at a concentration of less than 50 ng/µL, (ii) TthPrimPol, and (iii) a DNA polymerase.

The invention provides a method for amplifying a DNA template molecule, wherein the method comprises amplifying a DNA template molecule to generate a double-stranded DNA molecule, wherein the DNA template molecule is amplified by rolling circle amplification in the presence of (i) bacteriophage T4 Gene 32 Protein, (ii) a primase/polymerase, and (iii) a highly processive DNA polymerase (e.g. QualiPhi DNA polymerase).

The invention provides a method for amplifying a DNA template molecule, wherein the method comprises amplifying a DNA template molecule to generate a double-stranded DNA molecule, wherein the DNA template molecule is amplified by rolling circle amplification in the presence of (i) bacteriophage T4 Gene 32 Protein, (ii) TthPrimPol, and (iii) a highly processive DNA polymerase (e.g. QualiPhi DNA polymerase).

The invention provides a method for amplifying a DNA template molecule, wherein the method comprises amplifying a DNA template molecule to generate a double-stranded DNA molecule, wherein the DNA template molecule is amplified by rolling circle amplification in the presence of (i) bacteriophage T4 Gene 32 Protein at a concentration of 25 ng/µL, (ii) a primase/polymerase, and (iii) a highly processive DNA polymerase (e.g. QualiPhi DNA polymerase).

The invention provides a method for amplifying a DNA template molecule, wherein the method comprises amplifying a DNA template molecule to generate a double-stranded DNA molecule, wherein the DNA template molecule is amplified by rolling circle amplification in the presence of (i) bacteriophage T4 Gene 32 Protein at a concentration of 4 ng/µL and less than 50 ng/µL, (ii) a primase/polymerase, and (iii) a highly processive DNA polymerase (e.g. QualiPhi DNA polymerase).

The invention provides a method for amplifying a DNA template molecule, wherein the method comprises amplifying a DNA template molecule to generate a double-stranded DNA molecule, wherein the DNA template molecule is amplified by rolling circle amplification in the presence of (i) bacteriophage T T4 Gene 32 Protein at a concentration of 4 ng/µL and 25 ng/µL, (ii) a primase/polymerase, and (iii) a highly processive DNA polymerase (e.g. QualiPhi DNA polymerase).

The invention provides a method for amplifying a DNA template molecule, wherein the method comprises amplifying a DNA template molecule to generate a double-stranded DNA molecule, wherein the DNA template molecule is amplified by rolling circle amplification in the presence of (i) bacteriophage T4 Gene 32 Protein at a concentration of less than 50 ng/µL, (ii) a primase/polymerase, and (iii) a highly processive DNA polymerase (e.g. QualiPhi DNA polymerase).

The invention provides a method for amplifying a DNA template molecule, wherein the method comprises amplifying a DNA template molecule to generate a double-stranded DNA molecule, wherein the DNA template molecule is amplified by rolling circle amplification in the presence of (i) bacteriophage T4 Gene 32 Protein at a concentration of 25 ng/µL, (ii) TthPrimPol, and (iii) a highly processive DNA polymerase (e.g. QualiPhi DNA polymerase).

The invention provides a method for amplifying a DNA template molecule, wherein the method comprises amplifying a DNA template molecule to generate a double-stranded DNA molecule, wherein the DNA template molecule is amplified by rolling circle amplification in the presence of (i) bacteriophage T4 Gene 32 Protein at a concentration of 4 ng/µL and less than 50 ng/µL, (ii) TthPrimPol, and (iii) a highly processive DNA polymerase (e.g. QualiPhi DNA polymerase).

The invention provides a method for amplifying a DNA template molecule, wherein the method comprises amplifying a DNA template molecule to generate a double-stranded DNA molecule, wherein the DNA template molecule is amplified by rolling circle amplification in the presence of (i) bacteriophage T4 Gene 32 Protein at a concentration of 4 ng/µL and 25 ng/µL, (ii) TthPrimPol, and (iii) a highly processive DNA polymerase (e.g. QualiPhi DNA polymerase).

The invention provides a method for amplifying a DNA template molecule, wherein the method comprises amplifying a DNA template molecule to generate a double-stranded DNA molecule, wherein the DNA template molecule is amplified by rolling circle amplification in the presence of (i) bacteriophage T4 Gene 32 Protein at a concentration of less than 50 ng/µL, (ii) TthPrimPol, and (iii) a highly processive DNA polymerase (e.g. QualiPhi DNA polymerase).

The invention provides a method for producing a linear deoxyribonucleic acid (DNA) product, wherein the method comprises:
(a) amplifying a DNA template molecule comprising at least one endonuclease target sequence to generate a double-stranded DNA molecule, wherein the DNA template molecule is amplified by rolling circle amplification in the presence of (i) a single-stranded DNA binding protein that enhances the efficiency of amplification, (ii) a primase/polymerase, and (iii) a DNA polymerase; and
(b) contacting the double-stranded DNA molecule with an endonuclease to generate the linear DNA product.

The invention provides a method for producing a closed linear deoxyribonucleic acid (DNA) product, wherein the method comprises:
(a) amplifying a DNA template molecule comprising at least one endonuclease target sequence to generate a double-stranded DNA molecule, wherein the DNA template molecule is amplified by rolling circle amplification in the presence of (i) a single-stranded DNA binding protein that enhances the efficiency of amplification, (ii) a primase/polymerase, and (iii) a DNA polymerase;
(b) contacting the double-stranded DNA molecule with an endonuclease, a ligase and first and second adaptor molecules to form a single contiguous aqueous volume; and
(c) incubating the single contiguous aqueous volume to generate the closed linear DNA product,
wherein the closed linear DNA product comprises a linear double-stranded region, wherein the linear double-stranded region comprises a linear portion of the double-stranded DNA molecule, and wherein the linear double-stranded region is closed at a first end by the first adaptor molecule and closed at a second end by the second adaptor molecule.

The invention provides a method for producing a linear deoxyribonucleic acid (DNA) product, wherein the method comprises:
(a) amplifying a DNA template molecule comprising at least one endonuclease target sequence to generate a double-stranded DNA molecule, wherein the DNA template molecule is amplified by rolling circle amplification in the presence of (i) a single-stranded DNA binding protein that enhances the efficiency of amplification, (ii) a primase/polymerase, and (iii) a DNA polymerase;
(b) contacting the double-stranded DNA molecule with an endonuclease, a ligase and first and second adaptor molecules to form a single contiguous aqueous volume; and
(c) incubating the single contiguous aqueous volume to generate the linear DNA product,
wherein the linear DNA product comprises a linear double-stranded region, wherein the linear double-stranded region comprises a linear portion of the double-stranded DNA molecule, and wherein the first adaptor molecule is ligated to a first end of the linear double-stranded region and the second adaptor molecule is ligated to a second end of the linear double-stranded region, and wherein the first and second adaptor molecules are nucleic acid molecules that comprise one or more nuclease-resistant nucleotides.

The invention provides a method for producing a partially closed deoxyribonucleic acid (DNA) product, wherein the method comprises:
(a) amplifying a DNA template molecule comprising at least one endonuclease target sequence to generate a double-stranded DNA molecule, wherein the DNA template molecule is amplified by rolling circle amplification in the presence of (i) a single-stranded DNA binding protein that enhances the efficiency of amplification, (ii) a primase/polymerase, and (iii) a DNA polymerase;
(b) contacting the double-stranded DNA molecule with an endonuclease, a ligase and first and second adaptor molecules to form a single contiguous aqueous volume; and
(c) incubating the single contiguous aqueous volume to generate the partially closed linear DNA product, wherein the partially closed linear DNA product comprises a linear double-stranded region,
wherein the linear double-stranded region comprises a linear portion of the double-stranded DNA molecule, and wherein the first adaptor molecule is ligated to a first end of the linear double-stranded region and the second adaptor molecule is ligated to a second end of the linear double-stranded region, and wherein the first adaptor molecule is a nucleic acid molecule that comprise one or more nuclease-resistant nucleotides, and wherein the linear double-stranded region is closed at the second end by the second adaptor molecule.

The invention provides a method for producing a linear double-stranded DNA product, wherein the method comprises:
(a) rolling circle amplification of a DNA template molecule comprising at least one endonuclease target sequence in the presence of: 1) at least one type of a nuclease-resistant nucleotide; 2) a single-stranded DNA binding protein that enhances the efficiency of amplification, and 3) a DNA polymerase, to generate a double-stranded DNA product,
(b) contacting the double-stranded DNA product with at least one endonuclease to generate the linear double-stranded DNA product, wherein the linear double-stranded DNA product has increased resistance to nuclease digestion.

Preferably, in step (a) a primase/polymerase is also present.

Preferably, the single-stranded DNA binding protein is bacteriophage T4 Gene 32 Protein, the primase/polymerase is TthPrimPol, and the DNA polymerase is QualiPhi DNA polymerase or the Phi29 DNA polymerase. More preferably, the single stranded DNA binding protein is bacteriophage T4 Gene 32 Protein at a concentration of between 4 ng/µL and less than 50 ng/µL, the primase/polymerase is TthPrimPol, and the DNA polymerase is QualiPhi DNA polymerase or the Phi29 DNA polymerase. More preferably still, the single stranded DNA binding protein is bacteriophage T4 Gene 32 Protein at a concentration of 25 ng/µL, the primase/polymerase is TthPrimPol, and the DNA polymerase is QualiPhi DNA polymerase or the Phi29 DNA polymerase.

The methods may comprise a step of providing a template molecule before the step of amplifying the DNA template molecule.

In the methods, the step of amplification may be carried out at a temperature of between 20°C and 40°C, or between 25°C and 35°C, preferably about 30°C. The step of amplification may be carried out for at least 10 hours, at least 12 hours, at least 14 hours, at least 16 hours, at least 18 hours, or at least 20 hours, preferably about 20 hours. The step of amplification may be carried out for less than 24 hours, or less than 22 hours, preferably about 20 hours.

The methods of the invention also contemplate the use of primers instead of or in addition to the primase/polymerase.

The invention provides a method of amplifying of a DNA template molecule, wherein the method comprises amplifying a DNA template molecule to generate a double-stranded DNA molecule, wherein the DNA template molecule is amplified by rolling circle amplification in the presence of (i) a single-stranded DNA binding protein that enhances the efficiency of amplification, and (ii) primers.

The invention provides a method for producing a linear deoxyribonucleic acid (DNA) product, wherein the method comprises:
(a) amplifying a DNA template molecule comprising at least one endonuclease target sequence to generate a double-stranded DNA molecule, wherein the DNA template molecule is amplified by rolling circle amplification in the presence of (i) a single-stranded DNA binding protein that enhances the efficiency of amplification, and (ii) primers; and
(b) contacting the double-stranded DNA molecule with an endonuclease to generate the linear DNA product.

The invention provides a method for producing a closed linear deoxyribonucleic acid (DNA) product, wherein the method comprises:
(a) amplifying a DNA template molecule comprising at least one endonuclease target sequence to generate a double-stranded DNA molecule, wherein the DNA template molecule is amplified by rolling circle amplification in the presence of (i) a single-stranded DNA binding protein that enhances the efficiency of amplification, and (ii) primers;
(b) contacting the double-stranded DNA molecule with an endonuclease, a ligase and first and second adaptor molecules to form a single contiguous aqueous volume; and
(c) incubating the single contiguous aqueous volume to generate the closed linear DNA product,
wherein the closed linear DNA product comprises a linear double-stranded region, wherein the linear double-stranded region comprises a linear portion of the double-stranded DNA molecule, and wherein the linear double-stranded region is closed at a first end by the first adaptor molecule and closed at a second end by the second adaptor molecule.

The invention provides a method for producing a linear deoxyribonucleic acid (DNA) product, wherein the method comprises:
(a) amplifying a DNA template molecule comprising at least one endonuclease target sequence to generate a double-stranded DNA molecule, wherein the DNA template molecule is amplified by rolling circle amplification in the presence of (i) a single-stranded DNA binding protein that enhances the efficiency of amplification, and (ii) primers;
(b) contacting the double-stranded DNA molecule with an endonuclease, a ligase and first and second adaptor molecules to form a single contiguous aqueous volume; and
(c) incubating the single contiguous aqueous volume to generate the linear DNA product,
wherein the linear DNA product comprises a linear double-stranded region, wherein the linear double-stranded region comprises a linear portion of the double-stranded DNA molecule, and wherein the first adaptor molecule is ligated to a first end of the linear double-stranded region and the second adaptor molecule is ligated to a second end of the linear double-stranded region, and wherein the first and second adaptor molecules are nucleic acid molecules that comprise one or more nuclease-resistant nucleotides.

The invention provides a method for producing a partially closed deoxyribonucleic acid (DNA) product, wherein the method comprises:
(a) amplifying a DNA template molecule comprising at least one endonuclease target sequence to generate a double-stranded DNA molecule, wherein the DNA template molecule is amplified by rolling circle amplification in the presence of (i) a single-stranded DNA binding protein that enhances the efficiency of amplification, and (ii) primers;
(b) contacting the double-stranded DNA molecule with an endonuclease, a ligase and first and second adaptor molecules to form a single contiguous aqueous volume; and
(c) incubating the single contiguous aqueous volume to generate the partially closed linear DNA product, wherein the partially closed linear DNA product comprises a linear double-stranded region,
wherein the linear double-stranded region comprises a linear portion of the double-stranded DNA molecule, and wherein the first adaptor molecule is ligated to a first end of the linear double-stranded region and the second adaptor molecule is ligated to a second end of the linear double-stranded region, and wherein the first adaptor molecule is a nucleic acid molecule that comprise one or more nuclease-resistant nucleotides, and wherein the linear double-stranded region is closed at the second end by the second adaptor molecule.

Preferably, the step of amplification is performed in the presence of a DNA polymerase. Preferably, the DNA polymerase is QualiPhi DNA polymerase.

### 1. DNA cassette

The linear DNA product produced by the methods of the invention may comprise a DNA cassette. The linear DNA product produced by the methods of the invention may comprise a single DNA cassette. The DNA cassette may be difficult to completely and/or faithfully amplify from a single DNA template molecule. DNA cassettes may be difficult to completely and/or faithfully amplify from a single DNA template molecule due to their size and/or sequence composition (e.g. high GC content and/or presence of repeat regions such as palindromic repeats).

The DNA cassette may be formed of a single cassette sequence. The DNA cassette may be formed of at least two cassette sequences. The DNA cassette may be formed of a first cassette sequence and a second cassette sequence. The DNA cassette may be formed of at least 3, at least 4, at least 5, at least 6, at least 7, at least 8, at least 9, at least 10, at least 15, at least 20, at least 30, at least 40, at least 50, at least 60, at least 70, at least 80, at least 90 or at least 100 cassette sequences. The DNA cassette may comprise cassette sequences that are each amplified from separate DNA template molecules (each of which comprises a cassette sequence). Each of the double-stranded DNA molecules (i.e. the amplified DNA products) may comprise a cassette sequence. Each of the linear portions of the double-stranded DNA molecules (i.e. the amplified DNA products) may comprise a cassette sequence. The linear double-stranded region (or the linear DNA product) may comprise the DNA cassette (i.e. the complete DNA cassette). Alternatively, one or more cassette sequences (or portions thereof) may be provided by the first adaptor molecule and/or the second adaptor molecule.

Each of the cassette sequences may provide a different sequence of the DNA cassette. Alternatively, at least 2, at least 3, at least 4, at least 5, at least 6, at least 7, at least 8, at least 9, at least 10, at least 15, at least 20 or at least 25 cassette sequences may provide the same sequence of the DNA cassette. The DNA cassette may be formed of at least 2, at least 3, at least 4, at least 5, at least 6, at least 7, at least 8, at least 9, or at least 10, at least 15, at least 20 or at least 25 repeats of the same sequence.

In the methods, the first DNA template molecule may comprise a first cassette sequence; and the second DNA template molecule may comprise a second cassette sequence. The linear double-stranded region may comprise the DNA cassette (i.e. the complete DNA cassette).

In the methods, the first DNA template molecule, the first double-stranded DNA molecule (i.e. the first amplified DNA product) and the linear portion of the first double-stranded DNA molecule (i.e. the first amplified DNA product) may each comprise a first cassette sequence; and the second DNA template molecule, the second double-stranded DNA molecule (i.e. the second amplified DNA product) and the linear portion of the second double-stranded DNA molecule (i.e. the second amplified DNA product) may each comprise a second cassette sequence. The linear double-stranded region may comprise the DNA cassette (i.e. the complete DNA cassette).

The skilled person would be aware of a range of options for the composition of the DNA cassette and sequence components that might be included in such a cassette. Various non-limiting options for the cassette are described herein.

The DNA cassette may be at least 1, at least 2, at least 3, at least 4, at least 5, at least 6, at least 7, at least 8, at least 9, at least 10, at least 11, at least 12, at least 13, at least 14, at least 15, at least 20, at least 25, at least 30, at least 35, at least 40, at least 45, at least 50 or at least 55 kilobases (kb) in length. The DNA cassette may be 1-55, 2-50, 3-45, 4-40, 5-35, 6-30, 7-25, 8-20, 9-15, 10-14 or 11-13 kb in length.

Each cassette sequence may be at least 500 bp, at least 1 kb, at least 2 kb, at least 3 kb, at least 4 kb, at least 5 kb, at least 6 kb, at least 7 kb, at least 8 kb, at least 9 kb, at least 10 kb, at least 15 kb, at least 20 kb or at least 25 kb. Each cassette sequence may be 500 bp to 25 kb, 1-20 kb, 2-15 kb, 3-10 kb, 4-9 kb, 5-8 kb or 6-7 kb.

The DNA cassette may be formed of at least 3, at least 4, at least 5, at least 6, at least 7, at least 8, at least 9, or at least 10 cassette sequences. Each cassette sequence may be at least 500 bp, at least 1 kb, at least 2 kb, at least 3 kb, at least 4 kb, at least 5 kb, at least 6 kb, at least 7 kb, at least 8 kb, at least 9 kb, at least 10 kb, at least 15 kb, at least 20 kb or at least 25 kb. Each cassette sequence may be 500 bp to 25 kb, 1-20 kb, 2-15 kb, 3-10 kb, 4-9 kb, 5-8 kb or 6-7 kb.

The DNA cassette (or a cassette sequence) may comprise a repeated sequence. Repeated sequence may refer to a sequence in which two or more identical or extremely similar nucleotide sequences are repeated. The repeated sequence may be a tandem repeat or interspersed repeat. The repeated sequence may be a direct repeat or inverted repeat (e.g. a palindromic repeat such as an inverted terminal repeat (ITR)). Tandem repeats are repeated sequences which are directly adjacent to each other. Interspersed repeats are repeated sequences with intervening sequences, or repeated sequences that are non-adjacent. Direct repeats occur when a nucleotide sequence is repeated with the same directionality. The DNA cassette may comprise a long terminal repeat (LTR). Inverted repeats occur when a nucleotide sequence is repeated in the inverse direction. When there are no nucleotides separating the inverted repeat, the sequence is called a palindromic repeat. The repeated sequence may be at least 2, at least 5, at least 10, at least 25, at least 50, at least 75, at least 100, at least 250, at least 500, at least 750 or at least 1000 nucleotides long. Preferably, the repeated sequence is at least 100 nucleotides long. The repeated sequence may be 2-1000 nucleotides long, 3-500 nucleotides long, 4-250 nucleotides long, 5-100 nucleotides long, 10-1000 nucleotides long, 15-500 nucleotides long, 20-250 nucleotides long, 25-100 nucleotides long. Preferably, the repeated sequence is 4-250 nucleotides long.

The DNA cassette may comprise inverted terminal repeat sequences upstream and downstream of a coding region. The inverted terminal repeat sequences may be symmetrical (i.e. have the same symmetrical three-dimensional organization with respect to each other) or asymmetrical (i.e. have different three-dimensional organization with respect to each other). The inverted terminal repeat sequences may be from the same or different (viral) serotypes. An inverted terminal repeat sequence may comprise a terminal resolution site and a Rep binding site.

The DNA cassette (or a cassette sequence) may comprise a homopolymeric sequence. The DNA cassette (or a cassette sequence) may comprise a homopolymeric sequence at a 5'-end or a 3'-end or both a 5'-end and a 3'-end. The homopolymeric sequence may be a polyA, a polyC, a polyG, or a polyT sequence. The homopolymeric sequence may be between 3-200 nucleotides in length. The homopolymeric sequence may be used to facilitate purification of the linear DNA product, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides. The homopolymeric sequence may be formed (in the DNA cassette) by at least two cassette sequences.

The DNA cassette (or a cassette sequence) may comprise a coding sequence. The coding sequence may be at least 250, at least 500, at least 750, at least 1000, at least 1500, at least 2000, at least 3000, at least 4000, at least 5000, at least 7500, at least 10000, at least 15000 or at least 20000 base pairs (bp) in length. The coding sequence may be 250 to 20000, 500 to 15000, 750 to 10000, 1000 to 7500, 1500 to 5000, or 2000 to 4000 base pairs (bp) in length.

The coding sequence may be formed by at least 2, at least 3, at least 4, at least 5, at least 6, at least at least 7, at least 8, at least 9, at least 10, at least 15, at least 20 or at least 25 cassette sequences.

The DNA cassette (or a cassette sequence) may comprise a promoter (or a portion thereof) and a coding sequence. The promoter (or portion thereof) may be operably linked to the coding sequence. The cassette may comprise a promoter, a coding sequence and a 3' untranslated region e.g. a poly(A) tail. The cassette may comprise a promoter, a coding sequence, a 3' untranslated region e.g. a poly(A) tail, and a translational termination sequence. The cassette may comprise a promoter, a ribosome binding site, a coding sequence and a 3' untranslated region e.g. a poly(A) tail. The cassette may comprise a promoter, a ribosome binding site, a coding sequence, a 3' untranslated region e.g. a poly(A) tail, and a translational termination sequence. The cassette may comprise one or more of: a promoter; a ribosome binding site; a coding sequence; a 3' untranslated region e.g. a poly(A) tail; and a translational termination sequence.

The DNA cassette (or a cassette sequence) may comprise a promoter. The promoter may be an inducible or constitutive promoter. The promoter may be a non-specific promoter. The promoter may be a CMV promoter, a CAG promoter, a Rous sarcoma virus (RSV) promoter, an simian virus 40 (SV40) promoter, a mammalian elongation factor 1α (EF1α) promoter, a MPSV (Moloney Murine Sarcoma Virus) promoter, a Human Ubiquitin C (UBC) promoter and/or a human phosphoglycerate kinase hPGK promoter. The promoter may be a tissue-specific promoter. The promoter may be a thyroxine binding globulin (TBG) promoter, a glial fibrillary acidic protein (GFAP) promoter and/or an actin promoter.

The DNA cassette may further comprise an enhancer. The enhancer may be a CMV enhancer, an SV40 enhancer, a hTERT enhancer, an HIV enhancer, an LTR enhancer and/or a GATA enhancer.

The DNA cassette may comprise an insulator. The insulator may be a cis-regulatory element. The insulator may be at least 300, at least 400, at least 500, at least 1000, at least 1500 or at least 2000 nucleotides in length. The insulator may function either as an enhancer-blocker or a barrier, or both.

The DNA cassette may be an expression cassette. The DNA cassette may be a eukaryotic expression cassette e.g. a mammalian expression cassette or a plant expression cassette. The DNA cassette may be a prokaryotic expression cassette e.g. a bacterial expression cassette. The DNA cassette may be a viral expression cassette.

The DNA cassette may further comprise a reporter gene. The reporter gene may be an eGFP reporter gene, a luciferase reporter gene, or a β-glucuronidase reporter gene.

The DNA cassette may additionally comprise a sequence aiding protein expression, e.g. a cap-independent translation element such as an internal ribosome entry site (IRES).

The DNA cassette may comprise the sequence of a gene. The DNA cassette may comprise the sequence for at least two, three, four, or five genes.

The DNA cassette may comprise a sequence encoding an antigen. The DNA cassette may comprise sequences encoding at least 2, at least 3, at least 4, at least 5, at least 6, at least 7, at least 8, at least 9, at least 10, at least 15, at least 20, at least 30, at least 40, at least 50, at least 60, at least 70, at least 80, at least 90 or at least 100 antigens. The antigen(s) may be (a) self-antigen(s). The antigen(s) may be (a) neoantigen(s).

The DNA cassette may comprise (or encode) a repair template (or editing template). The repair template (or editing template) may be for use in CRISPR-Cas mediated homology directed repair (HDR). The DNA cassette may encode a CRISPR guide RNA.

The DNA cassette may further comprise a LoxP sequence, preferably two LoxP sequences. The two LoxP sequences may be oriented in the same direction. In this case, the DNA sequence between the two LoxP sequences may be excised as a circular loop of DNA. The two LoxP sequences may be oriented in opposite directions. In this case, the DNA sequence between the two LoxP sequences may be inverted. Preferably, the two LoxP sequences are in the same orientation (i.e. the same direction) in the cassette. One LoxP sequence may be at a first end of the cassette and the other LoxP sequence may be at a second end of the cassette. One LoxP sequence may be upstream and the other LoxP sequence may be downstream of the other sequence components of the cassette.

The DNA cassette may be a functional cassette i.e. it may have all sequence elements required for expression of a coding sequence.

Any of the sequence components described above in relation to the DNA cassette may be comprised in a single cassette sequence or split across two or more cassette sequences. For example, a coding sequence may be comprised in a single cassette sequence or split across two or more cassette sequences. A promoter may be comprised in a single cassette sequence or split across two or more cassette sequences.

The first cassette sequence may comprise a promoter and the second cassette sequence may comprise a coding sequence. The first cassette sequence may comprise an enhancer and the second cassette sequence may comprise a promoter and a coding sequence. The first cassette sequence may comprise a first gene coding sequence and a first regulatory sequence, and the second cassette sequence may comprise a second gene coding sequence and a second regulatory sequence. The first cassette sequence may comprise a first homology arm, the second cassette sequence may comprise a coding sequence and the third cassette sequence may comprise a second homology arm. The first cassette sequence may comprise a first ITR region, the second cassette sequence may comprise a coding sequence and the third cassette sequence may comprise a second ITR region. The first cassette sequence may comprise a first ITR region, the second cassette sequence may comprise a first coding sequence, the third cassette sequence may comprise a second coding sequence and the fourth cassette sequence may comprise a second ITR region. The first cassette sequence may comprise a first homology arm, the second cassette sequence may comprise a first coding sequence, the third cassette sequence may comprise a second coding sequence and the fourth cassette sequence may comprise a second homology arm. The first cassette sequence may comprise a first homology arm, the second cassette sequence may comprise a regulatory sequence, the third cassette sequence may comprise a linker block (non-RCA product), the fourth cassette sequence may comprise a coding sequence, and the fifth cassette sequence may comprise a second homology arm.

As described herein, the DNA template molecules (each comprising a cassette sequence) may be amplified separately. The separate amplification reactions may introduce different modifications into the amplified DNA products. For example, one amplification reaction may be performed in the presence of one or more reagents that are not included in the other amplification reaction(s). The one or more reagents may be an enzyme (e.g. methyltransferase), one or more modified nucleotides (e.g. nuclease-resistant nucleotides, methylated nucleotides, labelled nucleotides such as Cy3 or Cy5 labelled nucleotides, fluorescein labelled nucleotides, digoxygenin labelled nucleotides, biotin labelled nucleotides, fluorescent dye labelled nucleotides and/or modified nucleotides used for click chemistry), one or more nucleotide analogues (e.g. fluoro bases, deoxyUridine, zeatin riboside, nictotinamide and/or adenine dinucleotide), one or more fluorescently-conjugated nucleotides, and/or one or more non-hydrolyzable nucleotides.

At least one of the cassette sequences may contain one or more modifications that are not present in any of the other cassette sequences of the DNA cassette. The one or more modifications may be selected from methylation, a label such as Cy3 or Cy5, fluorescein, digoxygenin, biotin, a fluorescent dye, and/or modifications used for click chemistry.

### 2. Amplifying DNA template molecules to generate double-stranded DNA molecules

The methods comprise amplifying a DNA template molecule to generate double-stranded DNA molecules (i.e. an amplified DNA product). The DNA template molecule may comprise at least one endonuclease target sequence.

The methods may comprise amplifying DNA template molecules (e.g. first and second DNA template molecules) to generate double-stranded DNA molecules (i.e. amplified DNA products) (e.g. first and second double-stranded DNA molecules (i.e. amplified DNA products)). The DNA template molecules may comprise at least one endonuclease target sequence.

The double-stranded DNA molecule may be an amplified DNA product. The first double-stranded DNA molecule may be a first amplified DNA product. The second double-stranded DNA molecule may be a second amplified DNA product. Thus, the methods may comprise amplifying DNA template molecules (e.g. first and second DNA template molecules) to generate amplified DNA products (e.g. first and second amplified DNA products), wherein the DNA template molecules comprise at least one endonuclease target sequence.

### DNA template molecule

The DNA template molecule may comprise a sequence of the DNA cassette (i.e. a cassette sequence).

If more than one DNA template molecule is used, each DNA template molecule may comprise a sequence of the DNA cassette (i.e. a cassette sequence). For example, the first DNA template molecule may comprise a first cassette sequence and the second DNA template molecule may comprise a second cassette sequence.

The DNA template molecule(s) may (each) comprise at least one endonuclease target sequence. Thus, each DNA template molecule may comprise at least one endonuclease target sequence. Preferably, the DNA template molecule comprises at least two endonuclease target sequences. If more than one DNA template molecule is used, preferably, each DNA template molecule comprises at least two endonuclease target sequences. The endonuclease target sequences may be the same or different. Preferably, the at least one endonuclease target sequence is a restriction endonuclease target sequence. Different restriction endonuclease target sequences would be known to the skilled person. The endonuclease target sequence may be a Type IIS restriction endonuclease target sequence. For example, the restriction endonuclease target sequence may be a Bbsl, Bsal, BsmBl, BspQl, BtgZl, Esp3I, Sapl, Aarl, Acc36I, AclWI, Acul, Ajul, Alol, Alw26I, Alwl, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse1I, Bse3DI, BseGI, BseMI, BseMll, BseNI, BseRI, BseXl, Bsgl, BsIFI, BsmAl, BsmFl, Bsml, Bso31I, BspCNI, BspMI, BspPI, BspQl, BspTNI, BsrDl, Bsrl, Bst6I, BstF5I, BstMAI, BstV1I, BstV2I, Bsul, BtgZl, BtsCl, Btsl-v2, BtsMutl, Bvel, Csel, CspCl, Eam1104I, Earl, Ecil, Eco31I, Eco57I, Esp3I, Faql, Faul, Fokl, Gsul, Hgal, Hphl, HpyAV, Lgul, Lmnl, Lsp1109I, Lwel, Mboll, Mlyl, Mmel, Mnll, Mva1269I, NmeAlll, PaqCl, PciSl, Pctl, Plel, Ppsl, Psrl, Schl, SfaNI, Taqll, TspDTI and/or TspGWI target sequence. Preferably, the endonuclease target sequence is Bsal. The at least one endonuclease target sequence may be a native endonuclease sequence (i.e. an endonuclease sequence present in the DNA template molecule(s)). Alternatively, the at least one endonuclease target sequence may be introduced to the DNA template molecule prior to the amplification of the DNA template molecule.

The DNA template molecule used in the methods may be single-stranded or double-stranded. A single stranded DNA template molecule may be generated by denaturing a double stranded DNA template molecule. Thus, the methods may further comprise denaturing a double stranded DNA template molecule to generate a single stranded DNA template molecule. Denaturation may be achieved by any means known in the art including heat and/or chemical treatment (including organic solvents such as DMSO). Preferably, the DNA template molecule is double-stranded. Preferably, the DNA template molecule is single stranded after denaturation. Preferably, the single-stranded DNA binding protein stabilizes the single stranded DNA template molecule during amplification.

The DNA template molecule may be a natural circular DNA molecule. For example, the DNA template molecule may be (i) a plasmid, (ii) a minicircle, (iii) a cosmid, (iv) a bacterial artificial chromosome (BAC), or (v) a molecular inversion probe (MIP). The DNA template molecule may be an enzymatically produced circular DNA molecule. For example, the DNA template molecule may be (i) a circular DNA molecule obtained from a recombinase reaction, preferably a Cre recombinase reaction, or (ii) a circular DNA molecule obtained from a ligase reaction, preferably using the golden gate assembly. The DNA template molecule may be an enzymatically produced covalently-closed linear DNA molecule. For example, the DNA template molecule may be (i) a DNA molecule processed with TelN protelomerase; or (ii) a DNA molecule generated by ligation of the DNA ends with an adaptor.

The DNA template molecule may comprise an element that is double-stranded and an element that is single-stranded. For example, the template DNA molecule may comprise a double-stranded DNA and a single-stranded hairpin loop.

The DNA template molecule may be linear. If the DNA template molecule is linear, prior to amplification (e.g. rolling circle amplification), the DNA template molecule may be circularized to produce a DNA template molecule suitable for use in the methods described herein.

### Amplification

Amplification is performed in the presence of a single-stranded DNA binding protein that enhances the efficiency of amplification.

The expression "single-stranded DNA binding protein that enhances the efficiency of amplification" as used herein is intended to encompass any single-stranded DNA binding proteins, which function includes one or more of: (1) helix destabilization, (2) reduction of the formation of secondary DNA structures, (3) prevention of the degradation of ssDNA by nucleases, (4) prevention of the inhibition of amplification by template DNA contaminants, (5) improvement of the efficiency of DNA amplification by DNA polymerase, (6) stabilization of single-stranded regions of DNA for site-specific mutagenesis, (7) facilitation of completion of restriction enzyme digestion, (8) improvement of the efficiency of DNA synthesis by DNA polymerase, and (9) enhancement of the fidelity of DNA polymerase. Single-stranded DNA binding proteins (SSB) are canonically part of the DNA replication process in viruses, prokaryotes and eukaryotes and stabilize ssDNA regions during replication process. They are also loaded onto DNA independently of any other DNA replication machinery and are eventually displaced by replicative polymerase as it traverses DNA strand. Once displaced, soluble SSB is then available for further binding with ssDNA strands. The inventors found that presence of SSB proteins during the DNA amplification (by RCA) promotes existence of DNA template as interspersed ssDNA and SSB-bound ssDNA regions which are easily accessible to both priming and DNA amplification machinery. In particular, the inventors discovered that the single-stranded DNA binding protein used during amplification transiently binds ssDNA template and minimizes the formation of highly stable ssDNA secondary structures that could hinder DNA amplification. By way of explanation, in most amplification approaches, double stranded DNA template molecule is first converted into ssDNA through a denaturation step. ssDNA is the template that enables both priming and DNA amplification machinery to bind and is therefore one of the key limiting factors in DNA amplification origin formation. However, the ssDNA form is naturally unstable and partially reverses back into dsDNA (renaturation) as the DNA amplification reaction progresses. Renaturation of ssDNA can take place in form of ssDNA self-annealing which can form small loop domains or non-B DNA structures such as G-quadruplexes, Z-DNA or H-DNA structures which are highly stable, inaccessible and can be difficult to melt by DNA polymerase strand displacement. Large GC-rich constructs, constructs with repeated sequences, constructs with homopolymer regions and other highly complex regions are the constructs that are most likely to form the non-B DNA structures in a DNA template. Formation of such secondary structures on the template can result in DNA polymerase stalling and skipping regions of the template or completely aborting the replication of the current template strand. Thus, the use of the single-stranded DNA binding protein in the methods of the invention addresses this issue by transiently binding to the ssDNA template and minimizing the formation of highly stable ssDNA secondary structures that could hinder DNA amplification.

The single-stranded DNA binding protein may be bacteriophage T4 Gene 32 Protein (gp32), *Thermus thermophilus* SSB protein (TthSSB), *Thermus aquaticus* SSB protein (TaqSSB), and/or *E.coli* SSB (EcoSSB).

Preferably, the single-stranded DNA binding protein is bacteriophage T4 gene 32 protein (gp32).

Bacteriophage T4 Gene 32 Protein is a single-stranded DNA (ssDNA) binding protein required for bacteriophage T4 replication and repair. It cooperatively binds to and stabilizes transiently formed regions of ssDNA and plays an important structural role during T4 phage replication. It also has been used to stabilize and mark regions of ssDNA for electron microscopic examination of intracellular DNA structures. The bacteriophage T4 Gene 32 Protein has a UniProt accession number P03695.

The single-stranded DNA binding protein that enhances the efficiency of amplification may have a sequence of at least 80%, at least 85%, at least 90%, at least 95%, at least 97%, at least 98%, or at least 99% identity to SEQ ID NO: 5. The single-stranded DNA binding protein that enhances the efficiency of amplification may have a sequence of SEQ ID NO: 5.

The expression "enhances the efficiency of amplification" as used herein is understood to mean an improvement to one or more steps and/or events taking places during amplification of a DNA template molecule, which overall results in a more efficient amplification of the DNA template molecule. The expression "more efficient" may mean that more DNA product is produced, or that amplification requires less of the DNA template molecule, or that amplification uses less reagents, or that amplification is performed over a shorter period of time / lower temperature, or that less contaminants are produced (e.g. single stranded DNA). For example, the expression "enhances the efficiency of amplification" may mean one or more of (1) an improvement in helix destabilization, (2) reduction of the formation of secondary DNA structures, (3) prevention of the degradation of ssDNA by nucleases, (4) prevention of the inhibition of amplification by template DNA contaminants, (5) improvement of the efficiency of DNA amplification by DNA polymerase, (6) stabilization of single-stranded regions of DNA for site-specific mutagenesis, (7) facilitation of completion of restriction enzyme digestion, (8) improvement of the efficiency of DNA synthesis by DNA polymerase, and (9) enhancement of the fidelity of DNA polymerase.

The inventors discovered that the single-stranded DNA binding protein at a concentration of less than 50 ng/µL is particularly successful in producing the linear DNA product. Thus, the single-stranded DNA binding protein may be at a concentration of less than 50 ng/µL. The single-stranded DNA binding protein may be a concentration of between 1 ng/µL and less than 50 ng/µL. The single-stranded DNA binding protein may be at a concentration of between 4 ng/µL and 40 ng/µL, between 4 ng/µL and 30 ng/µL, between 4 ng/µL and 25 ng/µL, or between 10 ng/µL and 25 ng/µL. The single-stranded DNA binding protein may be at a concentration of between 4 ng/µL and 50 ng/µL, or between 4 ng/µL and 30 ng/µL. The single-stranded DNA binding protein may be at a concentration of about 25 ng/µL.

The term "about" as used herein for numerical data refers to a value within 10% of the underlying parameter (i.e., plus or minus 10%), and use of the term "about" at the beginning of a string of values modifies each of the values (i.e., "about 1, 2 and 3" refers to about 1, about 2 and about 3). For example, a concentration of "about 25 ng/µL" may include concentrations between 22.5 ng/µL and 27.5 ng/µL.

Preferably, the single-stranded DNA binding protein is at a concentration of 25 ng/µL.

In the embodiments in which the single-stranded DNA binding protein is bacteriophage T4 Gene 32 Protein (gp32). The bacteriophage T4 Gene 32 Protein may be at a concentration of less than 50 ng/µL. The bacteriophage T4 Gene 32 Protein may be a concentration of between 1 ng/µL and less than 50 ng/µL. The bacteriophage T4 Gene 32 Protein may be at a concentration of between 4 ng/µL and 40 ng/µL, between 4 ng/µL and 30 ng/µL, between 4 ng/µL and 25 ng/µL, or between 10 ng/µL and 25 ng/µL. The bacteriophage T4 Gene 32 Protein may be at a concentration of between 4 ng/µL and 50 ng/µL, or between 4 ng/µL and 30 ng/µL. The bacteriophage T4 Gene 32 Protein may be at a concentration of about 25 ng/µL. Preferably, the bacteriophage T4 Gene 32 Protein is at a concentration of 25 ng/µL.

Amplification (i.e. the step(s) of amplifying) may be performed: using a primer specific for the DNA template molecule; using a pair of primers specific for the DNA template molecule; or in the presence of a primase/polymerase (such as *Thermus thermophilus (Tth*) PrimPol (TthPrimPol)). Preferably, amplification is performed in the presence of a primase/polymerase.

The expression "primase/polymerase" as used herein is intended to encompass enzymes which have the activity of both primase and DNA polymerase. The expression "primase/polymerase" is intended to encompass enzymes that can start *in vitro* RNA/DNA synthesis and/or DNA extension. The primase/polymerase does not require the use of a primer in an amplification reaction. For example, the primase/polymerase may be TthPrimPol. TheTthPrimPol protein comes from Thermophilic bacterium *T. Thermophilus.* This protein has DNA-dependent activity to synthesize DNA Primer (Primase), and its recognition sequence is 3'- GTCC-5'. In the presence of magnesium ions, it can use ATP or dATP as the coenzyme to synthesize 7-9 nucleotide DNA Primer. TthPrimPol is described in detail in Picher et al. (Picher et al., (2016) "TruePrime is a novel method for whole-genome amplification from single cells based on *Tth*PrimPol", Nature Communications, 7(1):13296), which is incorporated herein by reference in its entirety. The primase/polymerase may be the PrimPol described in WO2025/012454, which is incorporated herein by reference in its entirety. The primase/polymerase may have a sequence of at least 80%, at least 85%, at least 90%, at least 95%, at least 97%, at least 98%, or at least 99% identity to SEQ ID NO: 1. The primase/polymerase may have a sequence of at least 80%, at least 85%, at least 90%, at least 95%, at least 97%, at least 98%, or at least 99% identity to SEQ ID NO: 2. The primase/polymerase may have a sequence of SEQ ID NO:1. The primase/polymerase may have a sequence of SEQ ID NO: 2. The expression "primase/polymerase" may interchangeably be used with the expressions "polymerase that has primase activity" or "DNA primase".

Preferably, the primase/polymerase is *Tth*PrimPol.

Primers specific for the DNA template molecule include single-stranded nucleic acids that are complementary to a target sequence in the DNA template molecule. A first primer of a set of primers may be complementary to the beginning of a target sequence in the DNA template molecule and a second primer of the set of primers may be complementary to the end of the target sequence in the DNA template molecule. In the case of a double-stranded DNA template, a first primer may be complementary to a first strand and a second primer may be complementary to a second strand.

Amplification (i.e. the step(s) of amplifying) may comprise *in vitro* or *in vivo* amplification. Preferably, the step(s) of amplifying is/are steps of *in vitro* amplification. Preferably, the step(s) of amplifying are performed by rolling circle amplification (RCA). Other amplification methods are also contemplated, for example, Multiple Annealing and Looping Based Amplification Cycles (MALBAC), polymerase chain reaction (PCR), nucleic acid sequence-based amplification (NASBA), loop-mediated isothermal amplification (LAMP), helicase-dependent amplification (HDA), multiple displacement amplification (MDA) or recombinase polymerase amplification (RPA).

Rolling circle amplification may be performed without any primers (i.e. in the presence of a primase/polymerase), or in the presence of a primer or multiple primers. For example, the primer may be a synthetic primer. The primers may be primers specific for a target sequence and/or random primers. Rolling circle amplification may be performed in the presence of a primase/polymerase. The primase/polymerase may be *Thermus thermophilus* (*Tth*) PrimPol (*Tth*PrimPol). Preferably, if the rolling circle amplification is performed without any primers, it is performed in the presence of a primase/polymerase, such as *Tth*PrimPol. If the rolling circle amplification is performed with a primase/polymerase, it may also be performed in the presence of one or more primers (such as synthetic primers). Thus, rolling circle amplification may be performed with primers and with a primase/polymerase.

Amplification may be performed with a strand-displacing polymerase (i.e. highly processive DNA polymerase) e.g. Phi29 DNA polymerase or a mutant thereof which retains functionality (e.g. QualiPhi^{®}, a chimeric form of Phi29 DNA polymerase from 4basebio).

Amplification may be performed in the presence of nuclease-resistant nucleotide triphosphates, such as phosphorothioated nucleotide triphosphates. The incorporation of nuclease-resistant nucleotide triphosphates may render the amplified DNA product resistant to exonuclease digestion. The ratio of nuclease-resistant nucleotides to total nucleotides used in the methods described herein may be at least 0.0001, at least 0.0025, at least 0.01, at least 0.025, at least 0.05, at least 0.075, at least 0.10, at least 0.12 at least 0.15, at least 0.25, at least 0.35, at least 0.5, or at least 0.75. The ratio of the nuclease-resistant nucleotides to total nucleotides used in the methods described herein may be less than 1, less than 0.9, less than, 0.8, less than 0.65, less than 0.5, less than 0.4, less than 0.3, less than 0.2, less than 0.1, less than 0.075, or less than 0.05. The ratio of the nuclease-resistant nucleotides to total nucleotides may be between 0.0001-1, between 0.0025-0.75, between 0.025-0.65, between 0.025-0.15, or between 0.25-0.50. Preferably, a ratio of the nuclease-resistant nucleotides to total nucleotides is between 0.025-0.15.

The step of amplification may be carried out at a temperature of between 20°C and 40°C, or between 25°C and 35°C, preferably about 30°C. The step of amplification may be carried out for at least 10 hours, at least 12 hours, at least 14 hours, at least 16 hours, at least 18 hours, or at least 20 hours, preferably about 20 hours. The step of amplification may be carried out for less than 24 hours, or less than 22 hours, preferably about 20 hours.

### Double-stranded DNA molecules (amplified DNA product(s))

The double-stranded DNA molecule (i.e. the amplified DNA product) may comprise a sequence of the DNA cassette (i.e. a cassette sequence).

Each double-stranded DNA molecule (i.e. each amplified DNA product) may comprise a sequence of the DNA cassette (i.e. a cassette sequence). For example, the first double-stranded DNA molecule (i.e. the first amplified DNA product) may comprise a first cassette sequence and the second double-stranded DNA molecule (i.e. the second amplified DNA product) may comprise a second cassette sequence.

The double-stranded DNA molecule(s) (i.e. the amplified DNA product(s)) is/are preferably produced by rolling circle amplification.

The double-stranded DNA molecule(s) (i.e. the amplified DNA product(s)) may be circular or branched.

The double-stranded DNA molecule(s) (i.e. the amplified DNA product(s)) is/are preferably concatemers (e.g. the first double-stranded DNA molecule (i.e. the first amplified DNA product) is a first concatemer and the second double-stranded DNA molecule (i.e. the second amplified DNA product) is a second concatemer). The concatemers may be generated by rolling circle amplification. The double-stranded DNA molecule(s) (i.e. the amplified DNA product(s)) may each comprise two or more copies of the same cassette sequence. Each double-stranded DNA molecule (i.e. each amplified DNA product) may be a concatemer comprising multiple copies of same cassette sequence.

The amplified DNA product(s) may each comprise double-stranded regions and single-stranded regions. Preferably, the amplified DNA product(s) is/are double-stranded DNA molecules.

The double-stranded DNA molecule (i.e. each amplified DNA product) may each comprise at least one endonuclease target sequence. Preferably, the double-stranded DNA molecule (i.e. the amplified DNA product) comprises at least two endonuclease target sequences. The endonuclease target sequences may be the same or different. Preferably, the at least one endonuclease target sequence is a restriction endonuclease target sequence. Different restriction endonuclease target sequences are known to the skilled person. The endonuclease target sequence may be a Type IIS restriction endonuclease target sequence. For example, the restriction endonuclease target sequence may be a Bbsl, Bsal, BsmBI, BspQl, BtgZl, Esp3I, Sapl, Aarl, Acc36I, AclWI, Acul, Ajul, Alol, Alw26I, Alwl, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse1I, Bse3DI, BseGI, BseMI, BseMll, BseNI, BseRl, BseXI, Bsgl, BsIFI, BsmAl, BsmFl, Bsml, Bso31I, BspCNI, BspMI, BspPI, BspQl, BspTNI, BsrDl, Bsrl, Bst6I, BstF5I, BstMAI, BstV1I, BstV2I, Bsul, BtgZl, BtsCl, Btsl-v2, BtsMutl, Bvel, Csel, CspCl, Eam1104I, Earl, Ecil, Eco31I, Eco57I, Esp3I, Faql, Faul, Fokl, Gsul, Hgal, Hphl, HpyAV, Lgul, Lmnl, Lsp1109I, Lwel, Mboll, Mlyl, Mmel, Mnll, Mva1269I, NmeAlll, PaqCl, PciSI, Pctl, Plel, Ppsl, Psrl, Schl, SfaNI, Taqll, TspDTI and/or TspGWI target sequence. Preferably, the endonuclease target sequence if Bsal. The at least one endonuclease target sequence may be a native endonuclease target sequence (i.e. an endonuclease target sequence present in the DNA template molecule(s)). Alternatively, the at least one endonuclease target sequence may be introduced into the DNA template molecule prior to the amplification of the DNA template molecule.

Each double-stranded DNA molecule (i.e. each amplified DNA product) may each comprise at least one endonuclease target sequence. Preferably, each double-stranded DNA molecule (i.e. each amplified DNA product) comprises at least two endonuclease target sequences. The endonuclease target sequences may be the same or different. Preferably, the at least one endonuclease target sequence is a restriction endonuclease target sequence. Different restriction endonuclease target sequences are known to the skilled person. The endonuclease target sequence may be a Type IIS restriction endonuclease target sequence. For example, the restriction endonuclease target sequence may be a Bbsl, Bsal, BsmBI, BspQl, BtgZl, Esp3I, Sapl, Aarl, Acc36I, AclWI, Acul, Ajul, Alol, Alw26I, Alwl, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse1I, Bse3DI, BseGI, BseMI, BseMll, BseNI, BseRl, BseXI, Bsgl, BsIFI, BsmAl, BsmFl, Bsml, Bso31I, BspCNI, BspMI, BspPI, BspQl, BspTNI, BsrDl, Bsrl, Bst6I, BstF5I, BstMAI, BstV1I, BstV2I, Bsul, BtgZl, BtsCl, Btsl-v2, BtsMutl, Bvel, Csel, CspCl, Eam1104I, Earl, Ecil, Eco31I, Eco57I, Esp3I, Faql, Faul, Fokl, Gsul, Hgal, Hphl, HpyAV, Lgul, Lmnl, Lsp1109I, Lwel, Mboll, Mlyl, Mmel, Mnll, Mva1269I, NmeAlll, PaqCl, PciSI, Pctl, Plel, Ppsl, Psrl, Schl, SfaNl, Taqll, TspDTI and/or TspGWI target sequence. The at least one endonuclease target sequence may be a native endonuclease target sequence (i.e. an endonuclease target sequence present in the DNA template molecule(s)). Alternatively, the at least one endonuclease target sequence may be introduced into the DNA template molecule prior to the amplification of the DNA template molecule.

The double-stranded DNA molecule(s) (i.e. the amplified DNA product(s)) may comprise a spacer.

The spacer may be at least 10, at least 20, at least 30, at least 40, at least 50, at least 60, at least 70, at least 80, at least 90, at least 100, at least 125, at least 150, at least 175, or at least 200 base pairs long. The spacer may improve an amplification yield of the double-stranded DNA molecule(s) (i.e. the amplified DNA product(s)). The spacer may improve ligation efficiency of first and second adaptor molecules to the linear double-stranded region. The spacer may improve cell transfection yields.

### Forming and incubating a single contiguous aqueous volume

The methods may comprise: forming a single contiguous aqueous volume comprising a double-stranded DNA molecule (i.e. an amplified DNA product), an endonuclease, a ligase and an adaptor molecule; and incubating the single contiguous aqueous volume to generate the linear DNA product, wherein the linear DNA product comprises a linear double-stranded region, and wherein an adaptor molecule is ligated to an end of the linear double-stranded region.

The methods may comprise: forming a single contiguous aqueous volume comprising a double-stranded DNA molecule (i.e. an amplified DNA product), an endonuclease, a ligase and first and second adaptor molecules; and incubating the single contiguous aqueous volume to generate the linear DNA product, wherein the linear DNA product comprises a linear double-stranded region, and wherein a first adaptor molecule is ligated to a first end of the linear double-stranded region and a second adaptor molecule is ligated to a second end of the linear double-stranded region.

The methods may comprise: forming a single contiguous aqueous volume comprising at least two double-stranded DNA molecules (i.e. at least two amplified DNA products) (e.g. the first double-stranded DNA molecule (i.e. the first amplified DNA product) and the second double-stranded DNA molecule (i.e. the second amplified DNA product)), an endonuclease, a ligase and an adaptor molecule; and incubating the single contiguous aqueous volume to generate the linear DNA product, wherein the linear DNA product comprises a linear double-stranded region, wherein the linear double-stranded region comprises a linear portion of each of the double-stranded DNA molecules (i.e. the amplified DNA products) (e.g. a linear portion of the first double-stranded DNA molecule (i.e. the amplified DNA product) and a linear portion of the second double-stranded DNA molecule (i.e. amplified DNA product)) and wherein an adaptor molecule is ligated to an end of the linear double-stranded region.

The methods may comprise: forming a single contiguous aqueous volume comprising at least two double-stranded DNA molecules (i.e. at least two amplified DNA products) (e.g. the first double-stranded DNA molecule (i.e. the first amplified DNA product) and the second double-stranded DNA molecule (i.e. the second amplified DNA product)), an endonuclease, a ligase and first and second adaptor molecules; and incubating the single contiguous aqueous volume to generate the linear DNA product, wherein the linear DNA product comprises a linear double-stranded region, wherein the linear double-stranded region comprises a linear portion of each of the double-stranded DNA molecules (i.e. the amplified DNA products) (e.g. a linear portion of the first double-stranded DNA molecule (i.e. the amplified DNA product) and a linear portion of the second double-stranded DNA molecule (i.e. amplified DNA product)) and wherein a first adaptor molecule is ligated to a first end of the linear double-stranded region and a second adaptor molecule is ligated to a second end of the linear double-stranded region.

### Linear portion(s) of the double-stranded DNA molecule(s) (i.e. the amplified DNA product(s)) and the linear double-stranded region

The linear portion of a double-stranded DNA molecule (i.e. an amplified DNA product) may comprise a sequence of the DNA cassette (i.e. a cassette sequence).

The double-stranded DNA molecule (i.e. each amplified DNA product) may be digested by an endonuclease to generate a linear portion of the double-stranded DNA molecule. The term "single cassette sequence" as used herein is intended to encompass a product that does not comprise or consist of a plurality of cassette sequences. That is to say that the digested product (i.e. the linear portion of the double-stranded DNA molecule (i.e. the amplified DNA product)) comprises only a single cassette sequence, which may for example comprise a single coding sequence of a gene of interest. The single cassette sequence may not comprise or consist of a plurality of tandem repeat sequences, and/or concatemeric DNA. The term "single cassette sequence" as used herein is intended to encompass a single copy of a sequence of the DNA cassette, for example, a single copy of the coding sequence or a single copy of a promoter sequence. Thus, the "single cassette sequence" may not encompass a cassette sequence that comprises or consists of multiple copies of the same DNA sequence linked in series.

The linear portion(s) of the double-stranded DNA molecule(s) (i.e. the amplified DNA product(s)) may have low dispersity (i.e. be substantially monodisperse). As used herein, the terms "low dispersity" and "monodisperse" are intended to encompass a collection of copies of digested DNA product of substantially the same size, i.e. the same length of the polynucleotide chain. That is to say, each linear portion of a double-stranded DNA molecule (i.e. an amplified DNA product) may vary in size (i.e. number of bases in the polynucleotide chain) from the other linear portions from the same double-stranded DNA molecule (i.e. amplified DNA product) by less than 10%, preferably by less than 5%.

The linear portion of the double-stranded DNA molecule (i.e. amplified DNA product) may be at least 50, at least 100, at least 250, at least 500, at least 750, at least 1000, at least 2000, at least 3000, at least 4000, at least 5000, at least 6000, at least 7000, at least 8000, at least 9000, at least 10000, at least 11000, at least 12000, at least 13000, at least 14000, at least 15000, or at least 20000 base pairs long. Preferably, the linear portion of the double-stranded DNA molecule (i.e. amplified DNA product) is at least 100 base pairs long. The linear portion of the double-stranded DNA molecule (i.e. amplified DNA product) may be 50 to 20000, 100 to 15000, 250 to 14000, 500 to 13000, 750 to 12000, 1000 to 11000, 2000 to 10000, 3000 to 9000, 4000 to 8000, or 5000 to 7000 base pairs long.

As used herein the term "linear double-stranded region" refers to a double stranded DNA. The linear double-stranded region may comprise the digested double-stranded DNA molecule.

The linear double-stranded region may comprise a DNA cassette (i.e. a complete DNA cassette).

The linear portion of each of the double-stranded DNA molecules (i.e. each of the amplified DNA products) may be ligated to the linear portion of another of the n double-stranded DNA molecules (i.e. amplified DNA products) to generate the linear double-stranded region.

The linear portion of the double-stranded DNA molecule (i.e. the amplified DNA product) may have an overhang. For example, the linear portion of the double-stranded DNA molecule (i.e. the amplified DNA product) may comprise a 5' overhang at a first end and a 3' overhang at a second end. Each overhang may have at least 3 nucleotides (preferably from 4 to 6 nucleotides).

Alternatively, the linear portion of the double-stranded DNA molecule (i.e. amplified DNA product) may have blunt ends.

The first end and the second end of the linear double-stranded region may be resistant to nuclease digestion. Preferably, the first end and the second end of the linear double-stranded region are resistant to exonucleases that cleave the 3'-end nucleotides (e.g. exonuclease III) and/or exonucleases that cleave the 5'-end nucleotides (e.g. exonuclease VIII).

The linear double-stranded region may comprise an overhang. For example, the linear double-stranded region may comprise a 5' overhang or a 3' overhang. The linear double-stranded region may comprise a blunt end or blunt ends. The linear double-stranded region may comprise: a 5' overhang and a blunt end, two 5' overhangs, a 3' overhang and a blunt end, two 3' overhangs, or a 5' overhang and a 3' overhang. The overhang may have at least 3 nucleotides (preferably from 4 to 6 nucleotides). The overhang may be in the sense strand or the antisense strand of the linear double-stranded region.

The linear double-stranded region (or a linear portion of a double-stranded DNA molecule (i.e. an amplified DNA product)) may comprise a plurality of nuclease-resistant nucleotides (e.g. phosphorothioated nucleotides) at internal positions in each strand. For example, the linear double-stranded region (or a linear portion of a double-stranded DNA molecule (i.e. an amplified DNA product)) may comprise at least 2, at least 4, at least 6, at least 8, at least 10, at least 12, at least 14, at least 16, at least 18, at least 20, at least 30, at least 40, at least 50, at least 60, at least 70, at least 80, at least 90, at least 100, at least 125, at least 150, at least 175, at least 200, at least 250, at least 300, at least 350, at least 400, at least 450, at least or 500 nuclease-resistant nucleotides (e.g. phosphorothioated nucleotides) at internal positions in each strand. Preferably, the linear double-stranded region (or a linear portion of a double-stranded DNA molecule (i.e. an amplified DNA product)) comprises at least 2 nuclease-resistant nucleotides (e.g. phosphorothioated nucleotides) at internal positions in each strand. The internal positions may not be located between the second and penultimate nucleotide of the linear double-stranded region (or DNA cassette).

The first end of the linear double-stranded region may be complementary to a portion of the first adaptor molecule. The second end of the linear double-stranded region may be complementary to a portion of the second adaptor molecule. The first end and/or the second end of the linear double-stranded region may be generated by endonuclease digestion.

### Adaptor molecules

The linear DNA product produced by the methods described herein may have enhanced resistance to exonuclease digestion (e.g. exonuclease III digestion). The enhanced resistance to exonuclease digestion may extend the life of the linear DNA product in a cell (i.e. the linear DNA product has enhanced resistance to intracellular exonucleases) and in a cell-free system (i.e. the linear DNA product has enhanced resistance to extracellular exonucleases). The enhanced resistance to exonuclease digestion may be provided by appending (e.g. ligating) a first adaptor molecule to a first end of the linear double-stranded region and a second adaptor to a second end of the linear double stranded region. In this regard, the entire content of WO2023/006978 A1 is incorporated herein by reference.

The adaptor molecule (e.g. the first adaptor molecule and/or second adaptor molecule) may be a double-stranded DNA. The double-stranded DNA may be open-ended at both ends. The double-stranded DNA may have a hairpin or a stem-loop at one end and be open-ended at the other end. The double-stranded DNA may have a hairpin or a stem-loop at one end and a hairpin or a stem-loop at the other end.

The adaptor molecule (e.g. the first adaptor molecule and/or second adaptor molecule) may comprise a homopolymeric sequence. The adaptor molecule (e.g. the first adaptor molecule and/or second adaptor molecule) may comprise a homopolymeric sequence at a 5'-end or a 3'-end or both a 5'-end and a 3'-end. The homopolymeric sequence may be a polyA, a polyC, a polyG, or a polyT sequence. The homopolymeric sequence may be 3-20 nucleotides, 4-12 nucleotides, 5-10 nucleotides, 10-20 nucleotides, 20-30 nucleotides, 30-40 nucleotides, 40-50 nucleotides, 50-60 nucleotides, 60-70 nucleotides, 70-80 nucleotides, 80-90 nucleotides, 90-100 nucleotides, 100-110 nucleotides, 110-120 nucleotides, or 3-120 nucleotides. The homopolymeric sequence may be at least 3 nucleotides, at least 4 nucleotides, at least 5 nucleotides, at least 10 nucleotides, at least 20 nucleotides, at least 30 nucleotides, at least 40 nucleotides, at least 50 nucleotides, at least 60 nucleotides, at least 70 nucleotides, at least 80 nucleotides, at least 90 nucleotides, at least 100 nucleotides, at least 110 nucleotides, at least 120 nucleotides. The homopolymeric sequence may be less than 5 nucleotides, less than 10 nucleotides, less than 20 nucleotides, less than 30 nucleotides, less than 40 nucleotides, less than 50 nucleotides, less than 60 nucleotides, less than 70 nucleotides, less than 80 nucleotides, less than 90 nucleotides, less than 100 nucleotides, less than 110 nucleotides, or less than 120 nucleotides.

The homopolymeric sequence may be used to facilitate purification of the linear DNA product. The homopolymeric sequence may be used to improve mRNA expression.

The double-stranded DNA of the adaptor molecule (e.g. the first adaptor molecule and/or second adaptor molecule) may have a Tm (i.e. melting temperature at which the DNA duplex dissociates into single-stranded DNA) above the temperature used for the step of incubating the single contiguous aqueous volume to generate the linear DNA product. The adaptor molecule (e.g. the first adaptor molecule and/or second adaptor molecule) may comprise one or more locked nucleic acids (LNAs). The adaptor molecule (e.g. the first adaptor molecule and/or second adaptor molecule) may comprise one or more DNA base modifications e.g. one or more cytosine base modifications. The adaptor molecule (e.g. the first adaptor molecule and/or the second adaptor molecule) may comprise one or more C5 substituted pyrimidine nucleotide(s), optionally wherein the C5 substituted pyrimidine nucleotide(s) is/are C5 alkenyl, C5 alkynyl, or C5 aryl pyrimidine nucleotide(s). The adaptor molecule (e.g. the first adaptor molecule and/or second adaptor molecule) may comprise one or more 5-methyl deoxycytosine (5-Me-dC) or one or more 5-Propynyl-2'-deoxyuridine (5-propynyl-dU) nucleotides. The adaptor molecule (e.g. the first adaptor molecule and/or second adaptor molecule) may comprise one or more 2-aminoadenine nucleotides.

The adaptor molecule may comprise a sequence of the DNA cassette (i.e. a cassette sequence). For example, the adaptor molecule may comprise a promoter or a portion thereof, or the adaptor may comprise a poly(A) tail or a portion thereof.

The first adaptor molecule may comprise a sequence of the DNA cassette (i.e. a cassette sequence). For example, the first adaptor molecule may comprise a promoter or a portion thereof. The second adaptor molecule may comprise a sequence of the DNA cassette (i.e. a cassette sequence). The second adaptor molecule may comprise a poly(A) tail or a portion thereof.

The adaptor molecule may comprise a GC content of at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%. The adaptor molecule may comprise a GC content of less than 30%, less than 20%, less than 15% or less than 10%. The adaptor molecule may comprise a GC content of 5-20%, 5-15% or 10-20%. GC content may refer to the percentage of bases that are either guanine or cytosine.

The adaptor molecule may comprise an enhancer or a terminator. The adaptor molecule may comprise a promoter. The adaptor molecule may comprise a promoter and an enhancer or a terminator.

The first adaptor molecule and/or the second adaptor molecule may comprise an enhancer or a terminator. The first adaptor molecule and/or the second adaptor molecule may comprise a promoter, The first adaptor molecule and/or the second adaptor molecule may comprise a promoter and an enhancer or a terminator.

The adaptor molecule may comprise a long terminal repeat (LTR) or a repeat sequence thereof.

The first adaptor molecule and/or the second adaptor molecule may comprise a long terminal repeat (LTR) or a repeat sequence thereof.

The adaptor molecule may be a double-stranded DNA molecule.

The first adaptor molecule and/or the second adaptor molecule may be a double-stranded DNA molecule.

The first and second adaptor molecules may be identical molecules, or they may be different molecules. For example, the first adaptor molecule and/or the second adaptor molecule may comprise a hairpin. The first adaptor molecule and/or the second adaptor molecule may be double-stranded linear nucleic acid molecules comprising one or more nuclease-resistant nucleotides. The first adaptor molecule may comprise a hairpin and the second adaptor molecule may be a double-stranded linear nucleic acid molecule comprising one or more nuclease-resistant nucleotides. The linear DNA product produced by the methods described herein may be resistant to nuclease (e.g. exonuclease) digestion.

The adaptor molecule may be a nucleic acid adaptor molecule (e.g. DNA). The adaptor molecule may be a synthetic adaptor molecule. The adaptor molecule may not be a plasmid or vector DNA. The adaptor molecule may be a nucleic acid/amino acid conjugated hybrid.

The first adaptor molecule may be a nucleic acid adaptor molecule (e.g. DNA). The second adaptor molecule may be a nucleic acid adaptor molecule (e.g. DNA). The first adaptor molecule and/or the second adaptor molecule may be a synthetic adaptor molecule. The first adaptor molecule and/or the second adaptor molecule may not be a plasmid or a vector DNA. The first adaptor molecule and/or the second adaptor molecule may be a nucleic acid/amino acid conjugated hybrid.

The adaptor molecule may comprise an overhang. For example, the adaptor molecule may comprise a 5' overhang or a 3' overhang. The adaptor molecule may comprise a blunt end or blunt ends. The overhang may have at least 3 nucleotides (preferably from 4 to 6 nucleotides). The overhang of the adaptor molecule may be complementary to an end of the linear double-stranded region. The overhang of the adaptor molecule may be complementary to a first end of the linear double-stranded region. The overhang of the adaptor molecule may anneal to an end of the linear double-stranded region. The overhang of the adaptor molecule may anneal to a first end of the linear double-stranded region.

The first adaptor molecule and/or the second adaptor molecule may comprise an overhang. For example, the first adaptor molecule and/or the second adaptor molecule may comprise a 5' overhang or a 3' overhang. The first adaptor molecule and/or the second adaptor molecule may comprise a blunt end or blunt ends. The overhang may have at least 3 nucleotides (preferably from 4 to 6 nucleotides). The overhang of the first adaptor molecule and/or the second adaptor molecule may be complementary to the first and/or second end of the linear double-stranded region. The overhang of the first adaptor molecule and/or the second adaptor molecule may anneal to the first and/or second end of the linear double-stranded region.

The adaptor molecule may comprise a 5' overhang and a 3' overhang. The adaptor molecule may comprise a 5' overhang and a 3' overhang, wherein the 3' overhang anneals to an end of the linear double-stranded region. The adaptor molecule may comprise a 5' overhang and a 3' overhang, wherein the 3' overhang anneals to a first end of the linear double-stranded region. The adaptor molecule may comprise a 5' overhang and a 3' overhang, wherein the 5' overhang anneals to an end of the linear doble-stranded region. The adaptor molecule may comprise a 5' overhang and a 3' overhang, wherein the 5' overhang anneals to a first end of the linear double-stranded region.

The first adaptor molecule and/or the second adaptor molecule may comprise a 5' overhang and a 3' overhang. The first adaptor molecule may comprise a 5' overhang and a 3' overhang, wherein the 3' overhang anneals to a first end of the linear double-stranded region. The second adaptor molecule may comprise a 5' overhang and a 3' overhang, wherein the 5' overhang anneals to a second end of the linear double-stranded region.

The adaptor molecule may comprise: a 5' overhang and a blunt end, two 5' overhangs, a 3' overhang and a blunt end, two 3' overhangs, or a 5' overhang and a 3' overhang. The overhang may have at least 3 nucleotides (preferably from 4 to 6 nucleotides). The overhang may be in the sense strand or the antisense strand of the linear double-stranded region.

The first adaptor molecule and/or the second adaptor molecule may comprise: a 5' overhang and a blunt end, two 5' overhangs, a 3' overhang and a blunt end, two 3' overhangs, or a 5' overhang and a 3' overhang. The overhang may have at least 3 nucleotides (preferably from 4 to 6 nucleotides). The overhang may be in the sense strand or the antisense strand of the linear double-stranded region.

The adaptor molecule may comprise a self-complementary element which creates a loop, such as a hairpin loop or a stem-loop. Thus, the adaptor molecule may comprise a hairpin or a stem-loop. The adaptor molecule may comprise a double-stranded portion comprising a sense strand and an antisense strand, wherein the sense strand and the antisense strand are linked together in a hairpin such that the sense strand is hybridized to the antisense strand. The double-stranded portion of an adaptor may comprise a 3' overhang or a 5' overhang of at least 1, at least 2, at least 3, at least 4, or at least 5 nucleotides. Preferably the 3' overhang or the 5' overhang is 4 to 6 nucleotides. Each end of the linear double-stranded region may comprise a 3' or a 5' overhang.

The first adaptor molecule and/or the second adaptor molecule may comprise a self-complementary element which creates a loop, such as a hairpin loop or a stem-loop. Thus, the first adaptor molecule may comprise a hairpin or a stem-loop. The second adaptor molecule may comprise a hairpin or a stem-loop. Both the first and second adaptor molecules may comprise a hairpin or a stem-loop. The adaptor molecules may each comprise a double-stranded portion comprising a sense strand and an antisense strand, wherein the sense strand and the antisense strand are linked together in a hairpin such that the sense strand is hybridized to the antisense strand. The double-stranded portion of an adaptor may comprise a 3' overhang or a 5' overhang of at least 1, at least 2, at least 3, at least 4, or at least 5 nucleotides. Preferably the 3' overhang or the 5' overhang is 4 to 6 nucleotides. Each end of the linear double-stranded region may comprise a 3' or a 5' overhang.

The adaptor molecule may comprise a single-stranded portion. The single-stranded portion may form a hairpin or a stem-loop. Thus, the adaptor molecule may comprise a loop portion. The single-stranded portion may comprise at least 2, at least 3, at least 4, at least 5, at least 6, at least 7, at least 8, at least 9, at least 10, at least 15, at least 20, at least 25, at least 30, at least 35, at least 40, at least 45 or at least 50 nucleotides. The single-stranded portion may comprise 2-50, 3-45, 4-40, 5-35, 6-30, 7-25, 8-20 or 9-15 nucleotides.

The first adaptor molecule and/or the second adaptor molecule may comprise a single-stranded portion. The single-stranded portion may form a hairpin or a stem-loop. Thus, the first adaptor molecule and/or the second adaptor molecule may comprise a loop portion. The single-stranded portion may comprise at least 2, at least 3, at least 4, at least 5, at least 6, at least 7, at least 8, at least 9, at least 10, at least 15, at least 20, at least 25, at least 30, at least 35, at least 40, at least 45 or at least 50 nucleotides. The single-stranded portion may comprise 2-50, 3-45, 4-40, 5-35, 6-30, 7-25, 8-20 or 9-15 nucleotides.

The adaptor molecule may comprise a double-stranded portion. The double-stranded portion may comprise less than 50, less than 45, less than 40, less than 35, less than 30, less than 25, less than 20, less than 15, or less than 10 base pairs. The double-stranded portion may comprise at least 5, at least 6, at least 7, at least 8, at least 9, at least 10, at least 11, at least 12, at least 13, at least 14, or at least 15 base pairs.

The first adaptor molecule and/or the second adaptor molecule may comprise a double-stranded portion. The double-stranded portion may comprise less than 50, less than 45, less than 40, less than 35, less than 30, less than 25, less than 20, less than 15, or less than 10 base pairs. The double-stranded portion may comprise at least 5, at least 6, at least 7, at least 8, at least 9, at least 10, at least 11, at least 12, at least 13, at least 14, or at least 15 base pairs.

The adaptor molecule may comprise an endonuclease target sequence. The first adaptor molecule and/or the second adaptor molecule may comprise an endonuclease target sequence. The first adaptor molecule may comprise an endonuclease target sequence and the second adaptor molecule may not comprise an endonuclease target sequence. The first adaptor molecule may not comprise an endonuclease target sequence and the second adaptor molecule may comprise an endonuclease target sequence. The first adaptor molecule and/or the second adaptor molecule may comprise the same endonuclease target sequences. The first adaptor molecule and/or the second adaptor molecule may comprise different endonuclease target sequences.

The first adaptor molecule and/or the second adaptor molecule may comprise a Type IIS endonuclease target sequence. The first adaptor molecule and/or the second adaptor molecule may comprise Bbsl, Bsal, BsmBI, BspQl, BtgZl, Esp3I,SapI, Aarl, Acc36I, AclWI, Acul, Ajul, Alol, Alw26I, Alwl, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse1I, Bse3DI, BseGI, BseMI, BseMll, BseNI, BseRl, BseXI, Bsgl, BsIFI, BsmAl, BsmFl, Bsml, Bso31I, BspCNI, BspMI, BspPI, BspQl, BspTNI, BsrDl, Bsrl, Bst6I, BstF5I, BstMAI, BstV1I, BstV2I, Bsul, BtgZl, BtsCl, Btsl-v2, BtsMutl, Bvel, Csel, CspCl, Eam1104I, Earl, Ecil, Eco31I, Eco57I, Esp3I, Faql, Faul, Fokl, Gsul, Hgal, Hphl, HpyAV, Lgul, Lmnl, Lsp1109I, Lwel, Mboll, Mlyl, Mmel, Mnll, Mva1269I, NmeAlll, PaqCl, PciSI, Pctl, Plel, Ppsl, Psrl, Schl, SfaNl, Taqll, TspDTI and/or TspGWI target sequences. The first adaptor molecule and/or the second adaptor molecule may comprise the same or different endonuclease target sequences.

The adaptor molecule (e.g. the first adaptor molecule and/or the second adaptor molecule) may comprise one or more nuclease-resistant nucleotides (i.e. protected nucleotides), such as phosphorothioated nucleotides. The nuclease-resistant nucleotides may be located in the single-stranded portion (e.g. hairpin portion) or the double-stranded portion of the adaptor molecules. The nuclease-resistant nucleotides may be located in the overhang portion of the adaptor molecule(s).

The adaptor molecule may confer resistance to 3'-end exonuclease digestion (e.g. by exonuclease III) and/or 5'-end exonuclease digestion (e.g. by exonuclease VIII).

The closing of the linear double-stranded region at the first end may generate a first closed end of the closed linear DNA product. The closing of the linear double-stranded region at the second end may generate a second closed end of the closed linear DNA product. The first closed end and the second closed end of the closed linear DNA product may be resistant to nuclease digestion. The nuclease digestion may be exonuclease digestion. Preferably, the first closed end and the second closed end of the closed linear DNA product confer resistance to 3'-end exonuclease digestion (e.g. by exonuclease III) and/or 5'-end exonuclease digestion (e.g. by exonuclease VIII).

The first and second adaptor molecules may comprise one or more nuclease-resistant nucleotides (e.g. phosphorothioated nucleotides), such that, once the adaptor molecules are appended (e.g. ligated) to the linear double-stranded region, the linear DNA product is resistant to nuclease digestion or has improved or enhanced resistance to nuclease digestion. The linear DNA product may be resistant to 3'-end exonuclease digestion (e.g. by exonuclease III) and/or 5'-end exonuclease digestion (e.g. by exonuclease VIII).

The adaptor molecule (e.g. the first adaptor molecule and/or the second adaptor molecule) may comprise a plurality of phosphorothioated nucleotides. For example, the adaptor molecule may comprise at least 2, at least 3, at least 4, at least 5, at least 6, at least 7, at least 8, at least 9, at least 10, at least 11, at least 12, at least 13, at least 14, at least 15 or at least 16 phosphorothioated nucleotides.

Each adaptor molecule may comprise a plurality of phosphorothioated nucleotides. For example, each adaptor molecule may comprise at least 2, at least 3, at least 4, at least 5, at least 6, at least 7, at least 8, at least 9, at least 10, at least 11, at least 12, at least 13, at least 14, at least 15 or at least 16 phosphorothioated nucleotides.

The adaptor may have a sense and antisense strands. The phosphorothioated nucleotides may be present in the sense or antisense strand of the adaptor molecule.

The adaptor molecule (e.g. the first adaptor molecule and/or the second adaptor molecule) may comprise one or more locked nucleic acid(s) and one or more phosphorothioated nucleotides. The adaptor molecule (e.g. the first adaptor molecule and/or the second adaptor molecule) may comprise one LNA and 5 phosphorothioated nucleotides. The adaptor molecule (e.g. the first adaptor molecule and/or the second adaptor molecule) may comprise one LNA in the sense strand of the adaptor and 5 phosphorothioated nucleotides in the antisense strand of the adaptor.

The adaptor molecule may be double-stranded (e.g. double-stranded DNA). The adaptor molecule may comprise a portion that is double-stranded (e.g. double-stranded DNA).

Each adaptor molecule may be double-stranded (e.g. double-stranded DNA). Each adaptor molecule may comprise a portion that is double-stranded (e.g. double-stranded DNA).

The adaptor molecule may comprise at least 3, at least 4, at least 5, at least 6, at least 7, at least 8, at least 9, at least 10, at least 11, at least 12, at least 13, at least 14, at least 15, at least 16, at least 20, at least 25, at least 30, at least 35, at least 40, at least 45, or at least 50 base pairs.

The first and/or second adaptor molecules may each comprise at least 3, at least 4, at least 5, at least 6, at least 7, at least 8, at least 9, at least 10, at least 11, at least 12, at least 13, at least 14, at least 15, at least 16, at least 20, at least 25, at least 30, at least 35, at least 40, at least 45, or at least 50 base pairs.

The adaptor molecule may comprise a plurality of phosphorothioated nucleotides in each strand. For example, the adaptor molecule may comprise at least 2, at least 3, at least 4, at least 5, at least 6, at least 7, at least 8, at least 9, at least 10, at least 11, at least 12, at least 13, at least 14, at least 15 or at least 16 phosphorothioated nucleotides in each strand.

Each adaptor molecule may comprise a plurality of phosphorothioated nucleotides in each strand. For example, each adaptor molecule may comprise at least 2, at least 3, at least 4, at least 5, at least 6, at least 7, at least 8, at least 9, at least 10, at least 11, at least 12, at least 13, at least 14, at least 15 or at least 16 phosphorothioated nucleotides in each strand.

The adaptor molecule may comprise a plurality of phosphorothioated nucleotides at internal positions in each strand. For example, the adaptor molecule may comprise at least 1, at least 2, at least 3, at least 4, at least 5, at least 6, at least 7, at least 8, at least 9, at least 10, at least 11, at least 12, at least 13, at least 14, at least 15 or at least 16 phosphorothioated nucleotides at internal positions in each strand. Preferably, the adaptor molecule comprises at least 2 phosphorothioated nucleotides at internal positions in each strand.

Each adaptor molecule may comprise a plurality of phosphorothioated nucleotides at internal positions in each strand. For example, each adaptor molecule may comprise at least 1, at least 2, at least 3, at least 4, at least 5, at least 6, at least 7, at least 8, at least 9, at least 10, at least 11, at least 12, at least 13, at least 14, at least 15 or at least 16 phosphorothioated nucleotides at internal positions in each strand. Preferably, each adaptor molecule comprises at least 2 phosphorothioated nucleotides at internal positions in each strand.

The internal positions may not be located between the second and penultimate nucleotide of the adaptor molecule. The internal positions may be any position in the adaptor molecules other than the last nucleotide at the end of each strand.

The adaptor molecule may comprise at least 5%, at least 10%, at least 20%, at least 30%, at least 40%, at least 50%, at least 60%, at least 70%, at least 80%, at least 90%, at least 95%, or at least 100% of nuclease-resistant nucleotides.

Each adaptor molecule may comprise at least 5%, at least 10%, at least 20%, at least 30%, at least 40%, at least 50%, at least 60%, at least 70%, at least 80%, at least 90%, at least 95%, or at least 100% of nuclease-resistant nucleotides.

The nuclease-resistant nucleotides (i.e. nucleotides resistant to exonuclease digestion) may be phosphorothioated nucleotides of at least one type. For example, the at least one type of phosphorothioated nucleotides is α-S-dATP (i.e. 2'-deoxyadenosine-5'-(α-thio)-triphosphate), α-S-dCTP (i.e. 2'-deoxycytidine-5'-(α-thio)-triphosphate), α-S-dGTP (i.e. 2'-deoxyguanosine-5'-(α-thio)-triphosphate), α-S-dTTP (i.e. 2'-deoxythymidine-5'-(α-thio)-triphosphate), α-S-dUTP (i.e. 2'-deoxyuridine-5'-(α-thio)-triphosphate), and/or uridine 2', 3'-cyclophosphorothioate.

The adaptor molecule(s) may comprise at least two types of phosphorothioated nucleotides. For example, the at least two types of phosphorothioated nucleotides are: α-S-dATP and α-S-dCTP, α-S-dATP and α-S-dGTP, α-S-dATP and α-S-dTTP, α-S-dCTP and α-S-dGTP, α-S-dCTP and α-S-dTTP, or α-S-dGTP and α-S-dTTP.

The adaptor molecule(s) may comprise at least three types of phosphorothioated nucleotides. For example, the at least three types of phosphorothioated nucleotides are:
(a) α-S-dATP, α-S-dCTP and α-S-dGTP;
(b) α-S-dATP, α-S-dCTP and α-S-dTTP;
(c) α-S-dATP, α-S-dGTP and α-S-dTTP; or
(d) α-S-dCTP, α-S-dGTP and α-S-dTTP.

The adaptor molecule(s) may comprise at least four types of phosphorothioated nucleotides. For example, the at least four types of nuclease-resistant nucleotides are α-S-dATP, α-S-dCTP, α-S-dGTP and α-S-dTTP.

Although the adaptor molecule(s) are described herein as comprising phosphorothioated nucleotides, the skilled person would appreciate that the adaptor molecule(s) may instead comprise any molecules that provide resistance to nuclease digestion (e.g. exonuclease digestion). For example, the adaptor molecule(s) may comprise nuclease-resistant nucleotides i.e. modified nucleotides that provide or increase resistance to nucleases (e.g. exonucleases). The adaptor molecule(s) may comprise a peptide, polypeptide or protein that provides or increases resistance to nucleases (e.g. exonucleases) digestion. The adaptor molecule(s) may comprise 2'-O-methyl nucleotides or 2'-O-methoxyethyl (MOE) nucleotides. The adaptor molecule(s) may comprise one or more locked nucleic acid(s).

The adaptor molecule may comprise a functional portion. The functional portion may be a binding molecule, a targeting sequence, or a probe.

The first adaptor molecule and/or the second adaptor molecule may comprise a functional portion. The functional portion may be a binding molecule, a targeting sequence, or a probe.

The functional portion may be a probe. As used herein, the term "probe" refers to a fragment of DNA, RNA or DNA/RNA chimera of variable length (e.g. 3-1000 bases long), which is used to detect the presence of target nucleotide sequences that are complementary to the sequence in the probe. Typically, the probe hybridizes to single-stranded nucleic acid whose base sequence allows probe-target base pairing due to complementarity between the probe and target. Thus, the functional portion may be a DNA sequence, a RNA sequence or a DNA/RNA chimera sequence.

The functional portion may be a binding molecule. The term "binding molecule" refers to any molecule capable of binding to the linear DNA product described herein and/or that is capable of binding to a further molecule or target. The binding molecule may be a protein, a polypeptide, or a peptide. The binding molecule may be an antibody, such as a monoclonal antibody or a polyclonal antibody. The binding molecule may be an antibody fragment.

The functional portion may facilitate detection of the linear DNA product by binding to capture molecules (e.g. capture antibodies bound by protein-protein interactions). The functional portion may bind to a cell target, for example, a cell receptor.

The functional portion may be a label. The 'label' can be any chemical entity which enables the detection of the double-stranded nucleic acid molecule via physical, chemical and/or biological means. The label may be a chromophore, a fluorophore and/or a radioactive molecule.

The functional portion may be a targeting sequence. The targeting sequence may be a fragment of DNA or RNA of variable length, which is used to target the linear DNA product to a specific location in a cell. The targeting sequence may be used to increase transfection efficiency of non-viral gene delivery by virtue of enhanced nuclear import of the linear DNA product. For example, the targeting sequence may be a DNA nuclear targeting sequence (i.e. a recognition sequence for endogenous DNA-binding proteins), such as a SV40 enhancer sequence (preferably downstream from the DNA cassette).

To facilitate detection and/or quantification of the linear DNA product, the functional portion may comprise a fluorophore, a radioactive compound or a barcode.

The functional portion may also facilitate DNA sequencing. For example, the functional portion may be a sequencing adapter. The term "sequencing adapter" is intended to encompass one or more nucleic acid domains that include at least a portion of a nucleic acid sequence (or complement thereof) utilized by a sequencing platform of interest, such as a sequencing platform provided by Illumina^{®} (e.g. the HiSeq^{™}, MiSeq^{™} and/or Genome Analyzer^{™} sequencing systems), Oxford Nanopore^{™} Technologies (e.g. the MinION sequencing system), Ion Torrent^{™} (e.g. the Ion PGM^{™} and/or Ion Proton^{™} sequencing systems), Pacific Biosciences (e.g. the PACBIO RS II sequencing system); Life Technologies^{™} (e.g. a SOLiD sequencing system), Roche (e.g. the 454 GS FLX+ and/or GS Junior sequencing systems), or any other sequencing platform of interest.

The adaptor molecule may comprise an inverted terminal repeat (ITR) sequence. An inverted terminal repeat sequence may comprise a terminal resolution site and a Rep binding site.

The first adaptor molecule and/or the second adaptor molecule may comprise an inverted terminal repeat (ITR) sequence. The inverted terminal repeat sequences of the first adaptor molecule and the second adaptor molecule may be symmetrical (i.e. have the same symmetrical three-dimensional organization with respect to each other) or asymmetrical (i.e. have different three-dimensional organization with respect to each other). The inverted terminal repeat sequences of the first adaptor molecule and the second adaptor molecule may be from the same or different (viral) serotypes. An inverted terminal repeat sequence may comprise a terminal resolution site and a Rep binding site.

The adaptor molecule may comprise an aptamer.

The first adaptor molecule and/or the second adaptor molecule may comprise an aptamer.

### Endonuclease and ligase

The endonuclease may be a restriction endonuclease (such as a Type II restriction endonuclease), RNA-guided DNA endonuclease, meganuclease or homing endonuclease. The Type II restriction endonuclease may be a Type IIP restriction endonuclease (e.g. EcoRI, Hindlll, BamHI or Notl) or a Type IIS restriction endonuclease (e.g. Bsal, Bbvl, Fokl or Sapl). The RNA-guided DNA endonuclease may be an endonuclease of Class 2 e.g. Class 2 Type V. The RNA-guided DNA endonuclease may be Cas12a. The RNA-guided DNA endonuclease may be Cpf1 or Mad7. Preferably, the RNA-guided DNA endonuclease is Cpf1. The homing endonuclease may be I-Ceul, I-Scel, PI-Pspl or PI-Scel.

The endonuclease may be a restriction enzyme endonuclease. The endonuclease may be a Type IIS restriction enzyme. The endonuclease may be any enzyme that recognizes a DNA sequence and cleaves outside of the recognition sequence. For example, the endonuclease may be a Bbsl, Bsal, BsmBl, BspQl, BtgZl, Esp3I, Sapl, Aarl, Acc36I, AclWI, Acul, Ajul, Alol, Alw26I, Alwl, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse1I, Bse3DI, BseGI, BseMI, BseMll, BseNI, BseRI, BseXl, Bsgl, BsIFI, BsmAl, BsmFl, Bsml, Bso31I, BspCNI, BspMI, BspPI, BspQl, BspTNI, BsrDI, Bsrl, Bst6I, BstF5I, BstMAI, BstV1I, BstV2I, Bsul, BtgZl, BtsCl, Btsl-v2, BtsMutl, Bvel, Csel, CspCl, Eam1104I, Earl, Ecil, Eco31I, Eco57I, Esp3I, Faql, Faul, Fokl, Gsul, Hgal, Hphl, HpyAV, Lgul, Lmnl, Lsp1109I, Lwel, Mboll, Mlyl, Mmel, Mnll, Mva1269I, NmeAlll, PaqCl, PciSl, Pctl, Plel, Ppsl, Psrl, Schl, SfaNI, Taqll, TspDTI and/or TspGWI restriction enzyme.

Type IIS restriction endonucleases cleave the double-stranded DNA molecule (i.e. amplified DNA product) outside of the recognition sequence (i.e. an endonuclease target sequence), which means that the recognition sequence (i.e. endonuclease target sequence) is not included in the linear DNA product.

The endonuclease may be at least 2, at least 3, at least 4 or at least 5 different endonucleases.

The ligase may be a DNA ligase, such as a T4 DNA ligase, T7 DNA ligase, mammalian DNA ligase I, III and IV; Taq DNA ligase, Tth DNA ligase, or *E*. *coli* DNA ligase.

### Incubating the single contiguous aqueous volume

The step of incubating the single contiguous aqueous volume may be performed under conditions that promote appending (or linking) of the first and/or second adaptor molecule(s) to the linear double-stranded region to produce the linear DNA product. The appending may be performed by creating a covalent link between the first and/or second adaptor molecule and the first and/or second end of the linear double-stranded region.

The ligation of the adaptor molecule may be performed by both hybridization and ligation of the adaptor molecule to an end of the linear double-stranded region. Thus, the adaptor molecule may be hybridized and ligated to an end of the linear double-stranded region. The ligation of the adaptor molecule may be performed by both hybridization and ligation of the adaptor molecule to a first end of the linear double-stranded region. Thus, the adaptor molecule may be hybridized and ligated to a first end of the linear double-stranded region.

The appending may be performed via a linker or spacer molecule which facilitates joining of the first and/or second adaptor molecules to the first and/or second end of the linear double-stranded region.

The linker or spacer may be at least 10, at least 20, at least 30, at least 40, at least 50, at least 60, at least 70, at least 80, at least 90, at least 100, at least 125, at least 150, at least 175, or at least 200 base pairs long.

The step of incubating the single contiguous aqueous volume may be performed under conditions that promote digestion of the double-stranded DNA molecule (i.e. the amplified DNA product) to produce a linear double-stranded region. The step of incubating the single contiguous aqueous volume may be performed under conditions that promote digestion of the double-stranded DNA molecules (i.e. the amplified DNA products) to produce the linear portions of the double-stranded DNA molecules (i.e. the amplified DNA products). The digestion of the double-stranded DNA molecule (i.e. the amplified DNA product) to produce the linear double-stranded region may be performed at a first temperature of 1°C-100°C, 1°C -80°C, 5°C-70°C, 10°C-60°C, 15°C-55°C, 20°C-50°C, 25°C -45°C, 30°C-40°C, 35°C-39°C, 36°C-38°C, or at about 37°C. The digestion of the double-stranded DNA molecules (i.e. the amplified DNA products) to produce the linear portions of the double-stranded DNA molecules (i.e. the amplified DNA products) may be performed at a first temperature of 1°C-100°C, 1°C -80°C, 5°C-70°C, 10°C-60°C, 15°C-55°C, 20°C-50°C, 25°C -45°C, 30°C-40°C, 35°C-39°C, 36°C-38°C, or at about 37°C. The digestion may be endonuclease digestion, preferably Type IIS endonuclease digestion.

The step of incubating the single contiguous aqueous volume may be performed under conditions that promote ligation of the linear double-stranded region and the adaptor molecule.

The step of incubating the single contiguous aqueous volume may be performed under conditions that promote ligation of the linear portions of the double-stranded DNA molecules (i.e. the amplified DNA products) (and optionally the first and second adaptor molecules). The ligation may be at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 82%, at least 85%, at least 90%, or at least 95% efficient. For example, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 82%, at least 85%, at least 90%, or at least 95% of the linear portions of the amplified DNA products may be incorporated into linear DNA products. Preferably, the ligation is at least 15% efficient.

Ligation efficiency may be established based on DNA quantification values before and after the digestion/ligation reaction. Thus, ligation efficiency may be established based on the equation: (starting amplified DNA amount) / (final linear DNA amount) x 100%.

Ligation efficiency may also be established based on DNA quantification values before and after the digestion/ligation reaction and the subsequent exonuclease treatment to remove remaining unprotected DNA molecules and adaptor molecules excess.

For example, the double-stranded DNA molecule(s) (i.e. the amplified DNA product(s)) generated by rolling circle amplification is first quantified so that the amount of the double-stranded DNA molecule(s) (i.e. the amplified DNA product(s)) used as the starting material during the digestion/ligation reaction is known. After all the enzymatic reactions, the linear DNA product is quantified to calculate the ligation efficiency as per the equation above.

DNA quantification methods are known to a person skilled in the art. For example, DNA quantifications may be carried out using the Qubit dsDNA BR assay from ThermoFisher (https://www.thermofisher.com/order/catalog/product/Q32850#/Q32850).

The step of ligation of the linear double-stranded region and the adaptor molecule may be performed at a second temperature of 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C. The step of ligation of the linear double-stranded region and the adaptor molecule may be performed at a second temperature of 1°C - 90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C.

The step of ligation of the linear double-stranded region and the first and/or second adaptor molecules may be performed at a second temperature of 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C. The step of ligation of the linear double-stranded region and the first and/or second adaptor molecules may be performed at a second temperature of 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C. The step of ligation of the linear portions of the double-stranded DNA molecule(s) (i.e. the amplified DNA product(s)) (and, optionally, the first and second adaptor molecules) may be performed at a second temperature of 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C.

The step of incubating the single contiguous aqueous volume may comprise incubating at a first temperature and then incubating at a second temperature. The first temperature may be 1°C-100°C, 1°C-80°C, 5°C-70°C, 10°C-60°C, 15°C-55°C, 20°C-50°C, 25°C-45°C, 30°C-40°C, 35°C-39°C, 36°C-38°C, or about 37°C. The second temperature may be 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C. Preferably, the first temperature is 35°C-39°C and the second temperature is 14°C-18°C. Using these conditions the endonuclease may be a Type IIS restriction endonuclease (e.g. Bsal) and the ligase may be T4 DNA ligase, T7 DNA ligase, mammalian DNA ligase I, III and IV; Taq DNA ligase, Tth DNA ligase, or *E*. *coli* DNA ligase.

The step of incubating the single contiguous aqueous volume may be performed isothermally. The step of incubating the single contiguous aqueous volume may comprise incubating at a constant temperature. The constant temperature may be 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, 40°C, 41°C, 42°C, 43°C, 44°C, 45°C, 46°C, 47°C, 48°C, 49°C, 50°C, 51°C, 52°C, 53°C, 54°C, or 55°C. Preferably, the constant temperature is 37°C. The constant temperature is intended to mean that the temperature does not significantly change during the reaction. The constant temperature is intended to mean that the temperature variation during the step of incubating the single contiguous aqueous volume is less than 10°C, less than 9°C, less than 8°C, less than 7°C, less than 6°C, less than 5°C, less than 4°C, less than 3°C, less than 2°C, or less than 1°C. In a preferred embodiment the temperature during the step of incubating the single contiguous aqueous volume does not deviate by more than 5°C, preferably by not more than 3°C, even more preferably not more than 1°C. Thus, the constant temperature may be a temperature in a range of 20°C-30°C, 22°C-32°C, 24°C-34°C, 26°C-36°C, 28°C-38°C, 30°C-40°C, 22°C-28°C, 32°C-38°C, 25°C-35°C, 26°C-34°C, 27°C-33°C, 27.5°C-32.5°C, 28°C-32°C, 28.5°C-31.5°C, 29°C-31°C, 29.5°C-30.5°C, 40°C-50°C, or 50°C-55°C. Preferably, the constant temperature is a temperature in a range of 27.5°C-32.5°C. Alternatively, the constant temperature may be a temperature in a range of 32°C-42°C, 33°C-41°C, 34°C-40°C, 35°C-39°C, 36°C-38°C. Preferably, the constant temperature is a temperature in a range of 34.5°C-39.5°C.

The step of incubating the single contiguous aqueous volume may comprise cycling between the first temperature and the second temperature. The step of incubating the single contiguous aqueous volume may comprise cycling between the first temperature and the second temperature at least 2, at least 3, at least 4, at least 5, at least 6, at least 7, at least 8, at least 9, at least 10, at least 15, at least 20, at least 25, at least 30, at least 35, at least 40, at least 45, at least 50, at least 55, at least 60, at least 65, at least 70, at least 80, at least 90, or at least 100 times, preferably at least 20 times. The step of incubating the single contiguous aqueous volume may comprise cycling between the first temperature and the second temperature less than 40, less than 35, less than 30 times, less than 29, less than 25 times. The step of incubating the single contiguous aqueous volume may comprise cycling between the first temperature and the second temperature 2-100, 5-80, 10-70, 20-60, or 30-60 times. The step of incubating the single contiguous aqueous volume may comprise cycling between the first temperature and the second temperature 2-20, 5-29, 61-100, or 65-80 times.

### 3. Optional steps of the methods

The method may further comprise (after the steps of amplification and before the step of forming a single contiguous aqueous volume (i.e. the step of contacting the double-stranded DNA molecule with an endonuclease, a ligase and first and second adaptor molecules)) a step of heat-deactivation. The step of heat-deactivation may be performed under conditions sufficient to inactivate the reagents used during the amplification reaction. The step of heat-deactivation may be performed at a temperature of at least 50°C, at least 55°C, at least 60°C, at least 65°C, at least 70°C, at least 75°C, at least 80°C, at least 85°C, at least 90°C, at least 95°C, or at least 100°C. The step of heat-deactivation may be performed for at least 1 min, at least 3 mins, at least 5 mins, at least 10 mins, at least 15 mins, or at least 20 mins.

The inventors discovered that large concatemeric products of rolling circle amplification reactions can be used to produce linear DNA products. This is surprising as the product of the rolling circle amplification has high viscosity and typically has to undergo purification steps before it can be utilized for downstream applications.

In the method described herein, after the step of amplification, the step of forming a single contiguous aqueous volume may be performed without purifying the double-stranded DNA molecule (i.e. the amplified DNA product). That is to say that the step of forming a single contiguous aqueous volume (may be performed directly after the step of amplification.

The method may further comprise, after the step of incubating the single contiguous aqueous volume, a step of purifying the linear DNA product.

The method may further comprise, after the step of incubating the single contiguous aqueous volume, a step of nuclease digestion. The nuclease digestion may be exonuclease digestion, such as exonuclease I and/or exonuclease III digestion. The step of nuclease digestion may take place before or after the step of purifying the linear DNA product. The step of nuclease digestion may allow for removal of any double-stranded DNA molecules and/or adaptor molecules that have not been used to produce linear DNA products. The method may comprise incubating the single contiguous aqueous volume with a nuclease (e.g. an exonuclease).

The method may comprise (i) incubating the single contiguous aqueous volume with a nuclease (e.g. an exonuclease); and (ii) purifying the linear DNA product. The method may further comprise (i) purifying the linear DNA product; and (ii) incubating the DNA product with a nuclease (e.g. an exonuclease).

The step of incubating with a nuclease may be performed at a temperature of 5-90°C, 10-80°C, 15-70°C, 20-60°C, 25-50°C, 30-45°C or 35-40°C. The step of incubating with a nuclease may be performed for at least 10, at least 20, at least 30, at least 40, at least 50, or at least 60 min. The step of incubating with a nuclease may be performed at two different temperatures. For example, the step of incubating with a nuclease may be performed at 15-40°C for 10-60 minutes followed by a temperature of 60-90°C for 10-30 min. The higher temperature typically inactivates the nuclease (e.g. exonuclease). Thus, the method may further comprise a step of inactivating the nuclease (e.g. exonuclease). The step of incubating with a nuclease may be performed at 37°C for 30 min and 80°C for 20 min. Preferably, the step of inactivating the nuclease (e.g. exonuclease) is performed at a temperature of 70-80°C. The step of inactivating the nuclease (e.g. exonuclease) may be performed for at least 1, at least 5, at least 10, at least 20 or at least 30 minutes. Preferably, the step of inactivating the nuclease (e.g. exonuclease) is performed for at least 5 minutes.

The method may further comprise contacting the single contiguous aqueous volume (or the product of any of the above steps) with Proteinase K.

### 4. General definitions

As used herein, the term "complementary" refers to the pairing of nucleotide sequences according to Watson/Crick pairing rules. For example, a sequence 5'-GCGGTCCCA-3' has the complementary sequence of 5'-TGGGACCGC-3'. A complement sequence can also be a sequence of RNA complementary to a DNA sequence.

As used herein the term "nuclease-resistant nucleotide" or "protected nucleotide" is intended to encompass any type of nucleotide that provides or enhances resistance to nuclease digestion (especially exonuclease digestion).

The nuclease-resistant nucleotides (i.e. nucleotides resistant to exonuclease digestion) may be phosphorothioated nucleotides. As used herein, the term "phosphorothioated nucleotide" refers to a nucleotide that has an altered phosphate backbone, wherein the sugar moieties are linked by a phosphorothioate bond. In the phosphate backbone of an oligonucleotide sequence, the phosphorothioate bond contains a sulphur atom as a substitute for a non-bridging oxygen atom. This modification renders the internucleotide linkage resistant to nuclease degradation.

The phosphorothioated nucleotides may be α-S-dATP (i.e. 2'-deoxyadenosine-5'-(α-thio)-triphosphate), α-S-dCTP (i.e. 2'-deoxycytidine-5'-(α-thio)-triphosphate), α-S-dGTP (i.e. 2'-deoxyguanosine-5'-(α-thio)-triphosphate), α-S-dTTP (i.e. 2'-deoxythymidine-5'-(α-thio)-triphosphate), α-S-dUTP (i.e. 2'-deoxyuridine-5'-(α-thio)-triphosphate), and/or uridine 2', 3'-cyclophosphorothioate.

The phosphorothioated nucleotides may be Sp-isomers, Rp-isomers or a mixture of both Sp- and Rp-isomers.

The nuclease-resistant nucleotides (i.e. nucleotides resistant to exonuclease digestion) may be 2'-O-methyl nucleotides or 2'-O-methoxyethyl (MOE) nucleotides. The nuclease-resistant nucleotides may be MOE nucleotides of at least one type, or at least two, three or four types. For example, the MOE nucleotides may be 2'-O-methoxy-ethyl guanosine, 2'-O-methoxy-ethyl cytidine, 2'-O-methoxy-ethyl adenosine, and/or 2'-O-methoxy-ethyl thymidine.

### 5. Linear DNA products

The invention provides a linear DNA product (e.g. an open linear DNA product, a closed linear DNA product or partially closed linear DNA product) as described herein.

The invention provides a linear DNA product (e.g. an open linear DNA product, a closed linear DNA product or partially closed linear DNA product) produced or obtainable by the methods for producing a linear DNA product as described herein.

The linear DNA product may comprise an overhang. The linear DNA product may comprise two overhangs. The linear DNA product may comprise an overhang of at least 2, at least 3, at least 4, at least 5, at least 6, at least 7, at least 8, at least 9, at least 10, at least 11, at least 12, at least 13, at least 14, at least 15, at least 16, at least 17, at least 18, at least 19 or at least 20 nucleotides in length. The linear DNA product may comprise an overhang of 2, 3, 4, 5, 6, 7, 8, 9, 10, 11, 12, 13, 14, 15, 16, 17, 18, 19 or 20 nucleotides in length. The linear DNA product may comprise two overhangs, wherein each overhang is at least 2, at least 3, at least 4, at least 5, at least 6, at least 7, at least 8, at least 9, at least 10, at least 11, at least 12, at least 13, at least 14, at least 15, at least 16, at least 17, at least 18, at least 19 or at least 20 nucleotides in length. The linear DNA product may comprise two overhangs, wherein each overhang is 1, 2, 3, 4, 5, 6, 7, 8, 9, 10, 11, 12, 13, 14, 15, 16, 17, 18, 19 or 20 nucleotides in length.

The linear DNA product may comprise an overhang at the 5' end of the linear DNA product and/or at the 3' end of the linear DNA product. The linear DNA product may comprise a 5' overhang of at least 2, at least 3, at least 4, at least 5, at least 6, at least 7, at least 8, at least 9, at least 10, at least 11, at least 12, at least 13, at least 14, at least 15, at least 16, at least 17, at least 18, at least 19 or at least 20 nucleotides in length. The linear DNA product may comprise a 5' overhang of 2, 3, 4, 5, 6, 7, 8, 9, 10, 11, 12, 13, 14, 15, 16, 17, 18, 19 or 20 nucleotides in length. The linear DNA product may comprise a 3' overhang of at least 2, at least 3, at least 4, at least 5, at least 6, at least 7, at least 8, at least 9, at least 10, at least 11, at least 12, at least 13, at least 14, at least 15, at least 16, at least 17, at least 18, at least 19 or at least 20 nucleotides in length. The linear DNA product may comprise a 3' overhang of 2, 3, 4, 5, 6, 7, 8, 9, 10, 11, 12, 13, 14, 15, 16, 17, 18, 19 or 20 nucleotides in length.

The overhang(s) may be present in either the template strand or the coding strand of the linear DNA product.

Preferably, the linear DNA product comprises a 5' overhang of 4-nucleotides. Preferably, the linear DNA product comprises a 4-nucleotide overhang at the 5' end of the template strand of the linear DNA product.

The overhang(s) of the linear DNA product may comprise one or more nuclease-resistant nucleotides (i.e. protected nucleotides), such as phosphorothioated nucleotides. The overhang(s) of the linear DNA product may comprise at least 1, at least 2, at least 3, at least 4 or at least 5 nuclease-resistant nucleotides (i.e. protected nucleotides), such as phosphorothioated nucleotides.

The linear DNA product may comprise a nuclease-resistant nucleotide at the 5' end of the DNA cassette or 5' of the DNA cassette, and a nuclease-resistant nucleotide at the 3'-end of the DNA cassette or 3' of the DNA cassette. The nuclease-resistant nucleotides may be incorporated into the linear DNA product during the RCA reaction.

The location of the nuclease-resistant nucleotides in the linear double-stranded DNA product may be such that the cassette is protected from nuclease (e.g. exonuclease III) digestion. Thus, the one or more nuclease-resistant nucleotides at the internal positions in each strand may be selected from:
(a) a 5'-end nucleotide of the cassette;
(b) a 3'-end nucleotide of the cassette; and
(c) one or more nucleotides outside of the cassette.

The term *"5'-end nucleotide of the cassette"* as used herein is intended to encompass the 5'-end nucleotide of each strand of the cassette. Thus, in the linear double-stranded DNA product, the cassette typically comprises a 5'-end nucleotide of the sense strand and a 5'-end nucleotide of the antisense strand.

The term *"3'-end nucleotide of the cassette"* as used herein is intended to encompass the 3'-end nucleotide of each strand of the cassette. Thus, in the linear double-stranded DNA product, the cassette typically comprises a 3'-end nucleotide of the sense strand and a 3'-end nucleotide of the antisense strand.

The term *"outside of the cassette"* as used herein is intended to encompass any nucleotides which are not part of the cassette. This includes any nucleotides that are comprised in the linear double-stranded DNA product and which also do not form part of the cassette.

The internal positions may not be located between the second and penultimate nucleotide of the cassette. The internal positions may be any position in the linear double-stranded DNA product other than the last nucleotide on the 3'-end and the 5'-end of the sense and antisense strands and other than nucleotides located between the second and penultimate nucleotide of the cassette.

The linear double-stranded DNA product may comprise at least 2, 3, 4, 5, 6, 7, 8, 9 or 10 nuclease-resistant nucleotides at internal positions in each strand. Preferably, the linear double-stranded DNA product comprises at least 2 nuclease-resistant nucleotides at internal positions in each strand.

### 6. Methods for transcription and protein expression

The invention provides a method for in vitro transcription of a linear DNA product, wherein the method comprises contacting the linear DNA product produced or obtainable by the methods described herein with a (RNA) polymerase and producing a transcription product encoded by the linear DNA product.

The invention provides a method for *in vitro* transcription of a linear DNA product, the method comprising:
(a) providing a linear DNA product, wherein the linear DNA product is produced by any of the methods described herein;
(b) contacting the linear DNA product with an RNA polymerase; and
(c) producing a transcription product.

The invention provides a method for in vitro transcription of a linear DNA product, wherein the method comprises:
(a) producing a linear DNA product by any of the methods described herein;
(b) contacting the linear DNA product with a (RNA) polymerase; and
(c) producing a transcription product encoded by the linear DNA product.

The invention provides a method for producing a protein, wherein the method comprises introducing the linear DNA product, produced or obtainable by the methods described herein, into a cell (e.g. a prokaryotic cell or a eukaryotic cell) or a cell-free expression system to generate a protein encoded by the linear DNA product.

The invention provides a method for producing a protein, wherein the method comprises:
(a) producing a linear DNA product by any of the methods described herein; and
(b) introducing the linear DNA product into a cell (e.g. a prokaryotic cell or a eukaryotic cell) or a cell-free expression system to generate a protein encoded by the linear DNA product.

The invention provides a method for producing a protein, wherein the method comprises:
(a) producing a linear deoxyribonucleic acid (DNA) product by any of the methods described herein; and
(b) introducing the linear DNA product into a cell or a cell-free expression system to generate a protein encoded by the linear DNA product; and
wherein step (b) is performed *in vitro.*

The cell-free expression system may originate from (or be derived from) a prokaryotic cell or eukaryotic cell. For example, the cell-free expression system may originate from (or be derived from) rabbit reticulocytes, wheat germ or *Escherichia coli.*

The cell may be a prokaryotic cell or a eukaryotic cell. The cell may be an animal cell, such as mammal cell (e.g. a human cell), a fungal cell, a cell of a micro-organism (e.g. a prokaryotic cell or a eukaryotic cell), or a plant cell. Preferably, the cell is a human cell. The step of introducing the linear DNA product into a cell may be performed in vivo or in vitro.

The linear DNA product may comprise a DNA cassette. The desired protein might be encoded by the DNA cassette. The step of introducing the linear DNA product into a cell may be performed in vivo or in vitro.

The nuclease-resistant nucleotides may be phosphorothioated nucleotides or any other nuclease-resistant nucleotides described herein.

### 7. Methods for cell transfection and cell transfection compositions

The invention provides a method for cell transfection of a linear DNA product produced or obtainable by any of the methods described herein, into a cell.

The invention provides a method for cell transfection of a linear DNA product into a cell, wherein the method comprises:
(a) producing a linear DNA product by any of the methods described herein;
(b) contacting a cell with the linear DNA product; and
(c) transfecting the linear DNA product into the cytosol of the cell.

The invention provides a method for cell transfection of linear deoxyribonucleic acid (DNA) product into a cell, wherein the method comprises:
(a) producing a linear DNA product by any of the methods described herein;
(b) contacting a cell with the linear DNA product; and
(c) transfecting the linear DNA product into the cytosol of the cell; and
wherein steps (b) and (c) are performed *in vitro.*

In the methods, the step of transfecting the linear DNA product into the cytosol of the cell may be performed by electroporation.

The invention provides a cell transfection composition comprising a linear DNA product produced by (or obtainable by) the methods described herein.

The cell transfection composition may or may not comprise a carrier e.g. an agent or a formulation. Preferably, the carrier promotes accumulation of the linear DNA product at a target site and/or protects the linear DNA product from undesirable interactions with biological milieu components and/or protects the linear DNA product from metabolism and/or degradation.

The invention further provides a cell transfection method comprising contacting (in vitro) a cell to be transfected with a linear DNA product produced or obtainable by the methods of the invention, and wherein the linear DNA product is transfected into the cytosol of the cell.

The cell or cells to be transfected may be provided in a cell culture medium (e.g. in a Petri dish, culture vessel or well, etc). The linear DNA product may be added directly to the cell culture medium or the cells may be added to a solution, such as saline, a buffered solution or a cell culture medium, comprising the linear DNA product.

The carrier may be a viral carrier or a non-viral carrier. Viral carriers include a lentiviral vector, an adenoviral vector, a retroviral vector, or an adeno associated viral vector for delivery of the linear DNA product. Non-viral carriers (or vectors) include complexing the linear DNA product with a cationic agent such as a cationic cell penetrating peptide (CPP); a DNA-binding cationic component, such as a polylysine chain; a cationic polymer or dendrimer e.g. polyethylenimine (PEI), poly-D,L-lactide-co-glycolide (PLGA), and a block copolymer of PEG and polylysine, and/or a cationic lipid (e.g. lipofectamine).

The carrier may be a small molecule (e.g., cholesterol, bile acid, and/or lipid), polymer, protein (e.g. an antibody), and/or aptamer (e.g. RNA) that is conjugated to the linear DNA product. The carrier may be a nanoparticulate formulation used to encapsulate the linear DNA product.

The carrier may be a modification of the linear DNA product with a targeting ligand (e.g. an antibody), a peptide, a small molecule (e.g. folic acid and/or biotin), or a polymer present in extracellular matrix (e.g. hyaluronic acid and/or chondroitin sulphate), or a hydrophobic modification of the double-stranded nucleic acid molecule (e.g. using cholesterol and/or α-tocopherol).

The cell transfection composition may or may not comprise an agent selected from a photosensitizing agent and/or a radical initiator e.g. a photoinitiator. Preferably, this agent improves the function of the linear DNA product at a target site and/or protects the linear DNA product from metabolism and/or degradation.

The invention further provides a cell obtainable by the methods of the invention. Thus, the cell may comprise a linear DNA product produced or obtainable by the methods of the invention.

The invention further provides a cell transfected with a linear DNA product produced or obtainable by the methods of the invention.

The cell may be an animal cell, such as mammal cell (e.g. a human cell), a fungal cell, a cell of a micro-organism (e.g. a prokaryotic cell or a eukaryotic cell), or a plant cell. Preferably, the cell is a human cell.

The step of contacting the cell with a linear DNA product may be performed in vivo or in vitro. For example, the linear DNA product may be administered to an organism (e.g. a subject) in need thereof. The organism (e.g. the subject) may be an animal, such as mammal (e.g. a human), a fungus, a micro-organism, or a plant.

Any or all of the linear DNA products described herein may be delivered to a cell via delivery particles such as liposomes, nanoparticles, exosomes, macrovesicles, viral or non-viral vectors. Any or all of the linear DNA products described herein may be delivered to a cell using a gene-gun. Any or all of the linear DNA products described herein may be delivered to a cell by electroporation. Any or all of the linear DNA products described herein may be delivered to a cell by hydrodynamic needle. The linear DNA products described herein may be delivered to a cell without a carrier.

The nanoparticle is preferably a self-assembled nanoparticle. The nanoparticle may be a nanoparticle which is produced by a process in which pre-existing components (e.g. a lipid component, a DNA product described herein) form an organized structure as a consequence of specific, local interactions among the components themselves, without external direction.

The linear DNA product and the lipid component may reversibly interact to form a self-assembled nanoparticle. The linear DNA product and the lipid component may reversibly interact in the self-assembled nanoparticle through intermolecular forces. The linear DNA product and the lipid component may reversibly interact in the self-assembled nanoparticle through non-covalent interactions. The linear DNA product and the lipid component may reversibly interact in the self-assembled nanoparticle through hydrogen bonds, van der Waals, hydrophobic, and/or electrostatic interactions. The linear DNA product and the lipid component may not be conjugated or linked by forces other than inter-molecular forces in the self-assembled nanoparticle.

### 8. Pharmaceutical compositions and methods for producing pharmaceutical compositions

The invention provides a pharmaceutical composition comprising a linear DNA product described herein, and a pharmaceutically acceptable carrier or excipient.

The invention provides a pharmaceutical composition comprising a linear DNA product produced or obtainable by the methods described herein, and a pharmaceutically acceptable carrier or excipient.

The invention provides a method for producing a pharmaceutical composition comprising the linear DNA product, wherein the method comprises performing the methods described herein to produce the linear DNA product and formulating the resulting linear DNA product with a pharmaceutically acceptable carrier or excipient.

Thus, the invention provides a method for producing a pharmaceutical composition, wherein the method comprises:
(a) producing a linear DNA product by any of the methods described herein;
(b) formulating the linear DNA product with a pharmaceutically acceptable carrier or excipient.

The pharmaceutical composition may be formulated as pills, tablets or capsules combined with one or more pharmaceutically acceptable solid carriers or as a solution in one or more pharmaceutically acceptable solvents, or as an emulsion, suspension or dispersion in one or more pharmaceutically acceptable solvents or carriers. The formulation may also include other pharmaceutically acceptable excipients such as stabilizers, anti-oxidants, binders, colouring agents or emulsifying or taste-modifying agents and extended release formulations.

The pharmaceutical composition may be administered orally, topically, parenterally or transdermally or by inhalation. The pharmaceutical composition may be administered by injection or intravenous infusion using suitable sterile solutions. Topical dosage forms may be creams, ointments, patches, or similar vehicles suitable for transdermal and topical dosage forms.

The pharmaceutical composition may be dissolved or suspended in a liquid vehicle or formulated as a granule (a small particle or grain), a pellet (a small sterile solid mass consisting of a highly purified composition, with or without excipients, made by the formation of granules, or by compression and moulding), or a pellet coated extended release (a solid dosage form in which the composition itself is in the form of granules to which varying amounts of coating have been applied, and which releases the composition in such a manner to allow a reduction in dosing frequency as compared to that composition presented as a conventional dosage form).

Other forms of pharmaceutical compositions include pills (a small, round solid dosage form containing the composition intended for oral administration), powder (an intimate mixture of dry, finely divided composition with one or more pharmaceutically acceptable additives that may be intended for internal or external use), elixir (a clear, pleasantly flavoured, sweetened hydroalcoholic liquid containing dissolved composition; it is intended for oral use), chewing gum (a sweetened and flavoured insoluble plastic material of various shapes which when chewed, releases the composition into the oral cavity), syrup (an oral solution containing the composition and high concentrations of sucrose or other sugars; the term has also been used to include any other liquid dosage form prepared in a sweet and viscid vehicle, including oral suspensions), tablet (a solid dosage form containing the composition with or without suitable diluents), tablet chewable (a solid dosage form containing the composition with or without suitable diluents that is intended to be chewed, producing a pleasant tasting residue in the oral cavity that is easily swallowed and does not leave a bitter or unpleasant after-taste), tablet coated or tablet delayed release, tablet dispersible, tablet effervescent, tablet extended release, tablet film coated, or tablet film coated extended release where the tablet is formulated in such manner as to make the contained composition available over an extended period of time following ingestion.

In other forms of pharmaceutical compositions, a tablet for solution, tablet for suspension, tablet multilayer, tablet multilayer extended release may be provided, where the tablet is formulated in such manner as to allow at least a reduction in dosing frequency as compared to that composition presented as a conventional dosage form. A tablet orally disintegrating, tablet orally disintegrating delayed release, tablet soluble, tablet sugar coated, osmotic, and the like are also suitable.

The oral dosage form pharmaceutical composition may contain, in addition to the composition, one or more inactive pharmaceutical ingredients such as diluents, solubilizers, alcohols, binders, controlled release polymers, enteric polymers, disintegrants, excipients, colorants, flavorants, sweeteners, antioxidants, preservatives, pigments, additives, fillers, suspension agents, surfactants (e.g., anionic, cationic, amphoteric and nonionic), and the like. Various FDA-approved topical inactive ingredients are found at the FDA's "The Inactive Ingredients Database" that contains inactive ingredients specifically intended as such by the manufacturer.

As used herein, injectable and infusion dosage forms include, but are not limited to, a liposomal injectable, which either consists of or forms liposomes (a lipid bilayer vesicle usually composed of phospholipids which is used to encapsulate the composition); an injection, which includes a sterile preparation intended for parenteral use; an emulsion injection, which includes an emulsion consisting of a sterile, pyrogen-free preparation intended to be administered parenterally; or a lipid complex injection.

For example, the linear DNA product can be administered by intratympanic injection (e.g. into the middle ear) and/or injections into the outer, middle, and/or inner ear. Such methods are routinely used in the art, for example, for the administration of steroids and antibiotics into human ears. Injection can be, for example, through the round window of the ear or through the cochlear capsule.

Other forms of pharmaceutical composition include a powder for solution injection, which is a sterile preparation intended for reconstitution to form a solution for parenteral use; a powder for suspension injection that is a sterile preparation intended for reconstitution to form a suspension for parenteral use; a powder lyophilized for liposomal suspension injection, which is a sterile freeze dried preparation intended for reconstitution for parenteral use which has been formulated in a manner that would allow liposomes (a lipid bilayer vesicle usually composed of phospholipids which is used to encapsulate the composition, either within a lipid bilayer or in an aqueous space) to be formed upon reconstitution; or a powder lyophilized for solution injection, wherein lyophilization ("freeze drying") is a process which involves the removal of water from products in the frozen state at extremely low pressures.

A suspension injection comprises a liquid preparation, suitable for injection, which consists of solid particles dispersed throughout a liquid phase in which the particles are not soluble that can also consist of an oil phase dispersed throughout an aqueous phase, or vice-versa. A suspension liposomal injection comprises a liquid preparation, suitable for injection, which consists of an oil phase dispersed throughout an aqueous phase in such a manner that liposomes (a lipid bilayer vesicle usually composed of phospholipids which is used to encapsulate the composition, either within a lipid bilayer or in an aqueous space) are formed. A suspension sonicated injection comprises a liquid preparation, suitable for injection, which consists of solid particles dispersed throughout a liquid phase in which the particles are not soluble. In addition, the product is sonicated while a gas is bubbled through the suspension, and this results in the formation of microspheres by the solid particles.

In another mode of administration, the pharmaceutical composition can be administered in situ, via a catheter or pump. A catheter or pump can, for example, direct the composition into the target location.

The parenteral carrier system may include one or more pharmaceutically suitable excipients, such as solvents and co-solvents, solubilizing agents, wetting agents, suspending agents, thickening agents, emulsifying agents, chelating agents, buffers, pH adjusters, antioxidants, reducing agents, antimicrobial preservatives, bulking agents, protectants, tonicity adjusters, and special additives. Formulations suitable for parenteral administration conveniently comprise a sterile oily or aqueous preparation of the composition which is preferably isotonic with the blood of the recipient but this is not essential.

As used herein, inhalation dosage forms include, but are not limited to, an aerosol (a product that is packaged under pressure and contains the composition which is released upon activation of an appropriate valve system intended for topical application to the skin as well as local application into the nose (nasal aerosols), mouth (lingual and sublingual aerosols), or lungs (inhalation aerosols)). A foam aerosol is a dosage form containing the composition, surfactants, aqueous or nonaqueous liquids, and propellants, whereby if the propellant is in the internal (discontinuous) phase (i.e., of the oil-in-water type), a stable foam is discharged, and if the propellant is in the external (continuous) phase (i.e., of the water-in-oil type), a spray or a quick-breaking foam is discharged. A metered aerosol is a pressurized dosage form consisting of metered dose valves which allow for the delivery of a uniform quantity of spray upon each activation. A powder aerosol is a product that is packaged under pressure and contains the composition, in the form of a powder that is released upon activation of an appropriate valve system. An aerosol spray is an aerosol product which utilizes a compressed gas as the propellant to provide the force necessary to expel the product as a wet spray and being applicable to solutions of the composition in aqueous solvent(s).

A transdermal dosage form may include, but is not limited to, a patch (a drug delivery system that often contains an adhesive backing that is usually applied to an external site on the body, whereby the ingredients (including the composition) either passively diffuse from, or are actively transported from, some portion of the patch, and whereby depending upon the patch, the ingredients (including the composition are either delivered to the outer surface of the body or into the body. Various types of transdermal patches such as matrix, reservoir and others are known in the art.

A topical dosage form may include various dosage forms known in the art such as lotions (an emulsion, liquid dosage form, whereby this dosage form is generally for external application to the skin), lotion augmented (a lotion dosage form that enhances composition delivery, whereby augmentation does not refer to the strength of the composition in the dosage form), gels (a semisolid dosage form that contains a gelling composition to provide stiffness to a solution or a colloidal dispersion, whereby the gel may contain suspended particles) and ointments (a semisolid dosage form, usually containing less than 20% water and volatiles and greater than 50% hydrocarbons, waxes, or polyols as the vehicle, whereby this dosage form is generally for external application to the skin or mucous membranes). Further embodiments include ointment augmented (an ointment dosage form that enhances composition delivery, whereby augmentation does not refer to the strength of the composition in the dosage form), creams (an emulsion, semisolid dosage form, usually containing greater than 20% water and volatiles and/or less than 50% hydrocarbons, waxes, or polyols may also be used as the vehicle, whereby this dosage form is generally for external application to the skin or mucous membranes) and cream augmented (a cream dosage form that enhances composition delivery, whereby augmentation does not refer to the strength of the composition in the dosage form). As used herein, an "emulsion" refers to a dosage form consisting of a two-phase system comprised of at least two immiscible liquids, one of which is dispersed as droplets, internal or dispersed phase, within the other liquid, external or continuous phase, generally stabilized with one or more emulsifying agents, whereby emulsion is used as a dosage form term unless a more specific term is applicable (e.g. cream, lotion, ointment). Further embodiments include suspensions (a liquid dosage form that contains solid particles dispersed in a liquid vehicle), suspension extended release, pastes (a semisolid dosage form, containing a large proportion, 20-50%, of solids finely dispersed in a fatty vehicle, whereby this dosage form is generally for external application to the skin or mucous membranes), solutions (a clear, homogeneous liquid dosage form that contains one or more chemical substances dissolved in a solvent or mixture of mutually miscible solvents), and powders.

The topical dosage form composition contains the composition and one or more inactive pharmaceutical ingredients such as excipients, colorants, pigments, additives, fillers, emollients, surfactants (e.g., anionic, cationic, amphoteric and nonionic), penetration enhancers (e.g., alcohols, fatty alcohols, fatty acids, fatty acid esters and polyols), and the like. Various FDA-approved topical inactive ingredients are found at the FDA's "The Inactive Ingredients Database" that contains inactive ingredients specifically intended as such by the manufacturer.

### 9. Uses and Applications

The invention provides a use of a linear DNA product as described herein in the production of a viral or non-viral delivery system.

The invention provides for use of a linear DNA product in the production of a viral or non-viral delivery system, wherein the linear DNA product is produced by any of the methods described herein.

The invention provides a use of a linear DNA product in the production of a viral or non-viral delivery system, wherein the linear DNA product is produced or obtainable by performing the method described herein.

The invention provides a viral or non-viral delivery system comprising a linear DNA product as described herein. The invention provides a viral or non-viral delivery system comprising a linear DNA product, wherein the linear DNA product is produced or obtainable by performing the method described herein.

### Viral vectors

Methods of producing viral vectors, such as AAV vectors, are known in the art. The most widely used method involves the co-transfection of HEK293 by three bacterial plasmids. The first plasmid encodes the *Rep* and *Cap* element, the second plasmid is a helper plasmid, while the third plasmid encodes the genetic payload of interest with inverted terminal repeats (ITRs). There are several issues surrounding the use of plasmids for viral preparation (e.g. AAV), namely: 1) the production of the plasmids is time consuming and costly, 2) there is a difficulty in the propagation of ITR sequences in *E.coli;* 3) the incorporation of the plasmid backbone into the viral capsid (e.g. AAV capsid) might be challenging (e.g. issues with antibiotic resistance markers). Methods of producing viral vectors may be performed using other cell lines. For example, a cell line suitable for production of viral vectors may be a Vero cell, or any other stable cell line. Together these represent the main bottleneck in viral vector production (e.g. AAV production). Thus, the methods described herein provide a linear DNA product suitable for use in production of viral vectors. The linear DNA product overcomes the above issues with plasmid vectors.

The invention provides a method of producing a viral vector, the method comprising introducing the linear DNA product produced or obtainable by the methods described herein into a cell under conditions such that the viral vector is produced. The linear DNA product may encode at least one element required for the production of the viral vector. For example, the linear DNA product may encode Rep and/or Cap elements. The linear DNA product may encode the helper plasmid elements. The linear DNA product may encode Rep, Cap and helper plasmid elements. The linear DNA product may encode a transgene. The method may be an in vivo or in vitro method. The cell may be an animal cell, preferably mammal cell, such as human cell (e.g. HEK293T, HEK293, CAP, CAP-T, or CHO). The cell may be a cell cultured in vitro in a tissue culture cell line.

Preferably, the vector is an AAV vector or lentivirus vector.

The invention also provides a method of delivering the viral vector (e.g. the linear DNA product) to a cell, comprising contacting the viral vector produced by the methods described herein with the cell. The cell may be an animal cell, preferably mammal cell, such as human cell.

The invention also provides a cell obtainable by the methods described herein.

### Non-viral vectors

Methods of producing non-viral vectors, are known in the art. Use of non-viral vectors over viral vectors has several advantages, including the opportunity of repeat dosing due to their non-immunogenicity, an unlimited packaging capacity, and low associated toxicity. Most methods for producing non-viral vectors use plasmid DNA. Thus, the linear DNA product produced or obtainable by the methods described herein is suitable for use in production of non-viral vectors. The linear DNA product produced or obtainable by the methods of the invention overcomes the issues of using plasmid vectors in non-viral vector preparation. For example, the linear DNA product, unlike plasmid DNA, does not comprise a bacterial backbone, allowing more transgene copies per mg of DNA. In addition, the linear DNA product does not comprise antibiotic resistant genes and bacterial contaminants. Moreover, the linear DNA product provides a prolonged transgene expression (due to the presence of exonuclease-resistant nucleotides), and a more cost-effective process of non-viral vector production.

The invention provides a method of delivering the non-viral vector to a cell, comprising contacting the non-viral vector comprising the linear DNA product with the cell. The cell may be an animal cell, preferably mammal cell, such as human cell.

The invention also provides a cell obtainable by the methods described herein.

### General therapeutic and diagnostic uses

The linear DNA product produced by the methods described herein is particularly suitable for use in therapy. The invention provides a linear DNA product as described herein for use in therapy. The invention provides a linear DNA product obtainable by the method described herein for use in therapy. The linear DNA product may encode a sequence of a therapeutic protein, a part of a vaccine, or an element of a genetic engineering mechanism, which is used to treat a disease or infection in a subject.

The invention further provides the linear DNA product as described herein for use as a medicament. The invention further provides the linear DNA produced by the methods described herein for use as a medicament. The invention further provides the linear DNA product obtainable by the methods described herein for use as a medicament. The invention also provides the use of a linear DNA product as described herein for the manufacture of a medicament for treating a disease. The invention also provides the use of a linear DNA product produced by the methods described herein for the manufacture of a medicament for treating a disease. The invention also provides the use of a linear DNA product obtainable by the methods described herein for the manufacture of a medicament for treating a disease.

The linear DNA product may encode a sequence of a therapeutic protein, a part of a vaccine, or an element of a genetic engineering mechanism, which is used to treat a disease or infection in a subject.

The invention further provides the linear DNA product produced by the methods described herein for use in treating a disease.

The invention also provides a method of treating a disease in a subject comprising administering to the subject a linear DNA product described herein. The invention also provides a method of treating a disease in a subject comprising administering to the subject the linear DNA product produced by the methods described herein. The invention also provides a method of treating a disease in a subject comprising administering to the subject the linear DNA product obtainable by the methods described herein. Preferably, the amount of the linear DNA product administered to the subject is a therapeutically active amount.

The linear DNA product described herein may be used to treat any disease or disorder. For example, the linear DNA product may be used to treat one or more of the diseases and/or disorders selected from genetic disorders (e.g. monogenic disorders), cancer, HIV, other viral infections (e.g. infection caused by coronavirus (e.g. COVID-19), hepatitis A, hepatitis B, herpes simplex virus type 2, influenza, measles and/or respiratory syncytial virus), neurodegenerative diseases (e.g. Parkinson's disease and/or polyglutamine diseases such as Huntington's disease), ocular diseases (e.g. macular degeneration) and liver failure. Preferably, the linear DNA product is used to treat a genetic disorder. More preferably still, the linear DNA product is used to treat a monogenic disorder. For example, the linear DNA product, may be used to treat sickle cell anaemia, cystic fibrosis, Huntington disease, and Duchenne's Muscular Dystrophy, Haemophilia A, α1-antitrypsin deficiency, primary ciliary dyskinesia, or respiratory distress syndrome of prematurity.

A subject treated with the linear DNA product may receive the linear DNA product in the form of any of the pharmaceutical compositions described herein.

A subject treated with the linear DNA product may receive the linear DNA product in combination with other forms of treatment for the disorder concerned, including treatment with drugs generally used for the treatment of the disorder. The drugs may be administered in one or several dosage units. The skilled person (e.g. a medical practitioner) is well able to determine an appropriate dosage regimen for the subject according to the subject's specific circumstances.

As used herein, "administering" means introducing the linear DNA product into the subject's body as described in more detail above (see "Pharmaceutical compositions and methods for producing pharmaceutical compositions"). Examples include but are not limited to oral, topical, buccal, sublingual, pulmonary, transdermal, transmucosal, as well as subcutaneous, intraperitoneal, intravenous, and intramuscular injection or in the form of liquid or solid doses via the alimentary canal.

As used herein, the phrase "a therapeutically active amount" means an amount of the linear DNA product that, when administered to a subject for treating a disease, is sufficient to affect such treatment of the disease. A "therapeutically active amount" will vary depending, for instance, on factors such as the specific product used, the severity of subject's disease, the age and relative health of the subject and the route and form of administration. Determining the relevant therapeutically active amount for a specific subject based on such factors is routine for the person skilled in the art (e.g. an attending medical practitioner). Treatment of a disease as described herein should be understood to mean an improvement in one of more of the symptoms of a disease.

The invention also provides the use of the linear DNA product as described herein in a method of diagnosing a disease and/or a disorder. The invention also provides the use of the linear DNA product produced or obtainable by the methods described herein in a method of diagnosing a disease and/or a disorder.

The invention provides the use of the linear DNA product in the "in vitro" diagnosis of a disease. The invention provides the use of the linear DNA product obtainable by the methods described herein in the "in vitro" diagnosis of a disease.

The invention also provides the linear DNA product for use in a method of diagnosis "in vivo" of a disease.

The method may be used to diagnose any disease and/or disorder. For example, the diseases and/or disorders may be selected from genetic disorders (e.g. monogenic disorders), cancer, HIV, other viral infections (e.g. infection caused by coronavirus (e.g. COVID-19), hepatitis A, hepatitis B, herpes simplex virus type 2, influenza, measles and/or respiratory syncytial virus), neurodegenerative diseases (e.g. Parkinson's disease and/or polyglutamine diseases such as Huntington's disease), ocular diseases (e.g. macular degeneration) and liver failure. Preferably, the linear DNA product is used to diagnose a genetic disorder. More preferably still, the linear DNA product is used to diagnose a monogenic disorder. For example, the linear DNA product may be used to diagnose sickle cell anaemia, cystic fibrosis, Huntington disease, and Duchenne's Muscular Dystrophy, Haemophilia A, α1-antitrypsin deficiency, primary ciliary dyskinesia, or respiratory distress syndrome of prematurity.

The diagnostic and treatment methods described herein may be in vitro methods or in vivo methods.

The method of diagnosis may rely on the detection and/or quantification of the linear DNA product.

To facilitate detection and/or quantification of the linear DNA product, the linear DNA product may be attached or bound to a functional portion. The functional portion may be any functional portion described herein. For example, the functional portion may be a probe. The functional portion may comprise a fluorophore, a radioactive compound or a barcode. The functional portion may be a protein, for example, an antibody.

The diagnosis may rely on the detection of a signal corresponding to the presence, absence and/or level of the linear DNA product. For example, the signal may be measured by flow cytometry and/or fluorescence-activated cell sorting of a linear DNA product attached to a fluorescent probe.

The linear DNA product may be detected by being bound to a capture moiety, for example in a lateral flow assay. In this example, the functional portion is a protein, for example, an antibody specific for the capture moiety. The capture of the antibody attached to the linear DNA product may produce a visual signal (e.g. a band of a different colour).

The invention also provides a method that combines diagnosis of a disease with a treatment of the disease.

### Cell therapy

The products produced by the methods of the invention may be substantially less contaminated than plasmid DNA. In addition, the products produced by the methods of the invention are very simple in nature (e.g. no bacterial backbone), which means that they are typically easy to work with. The pure and simple nature of the products described herein makes them particularly suitable for use in cell therapy. For example, a cell comprising the linear DNA product may be injected or otherwise transplanted into a patient to cause a desired effect. The cell (or cells) may be capable of fighting cancer cells, for example, via cell-mediated immunity in the course of immunotherapy. The cell (or cells) may be grafted to regenerate diseased tissues.

The invention provides the linear DNA product described herein for use in cell therapy. The invention provides the linear DNA product produced or obtainable by the methods described herein for use in cell therapy.

Preferably, cell therapy is ex-vivo cell therapy. The cell may be an animal cell, preferably mammal cell, such as human cell.

The invention also provides a cell obtainable by any methods described herein. The cell may be suitable for use in cell therapy.

### Vaccines

The linear DNA products produced by the methods described herein are particularly suitable for use in vaccine production. A vaccine may comprise a linear DNA product described herein. A vaccine may comprise a linear DNA product produced or obtainable by the methods described herein. Alternatively, the linear DNA product may be used to produce a vaccine, preferably an mRNA-based vaccine. For example, vaccines such as the BioNTech and Moderna mRNA vaccines against severe acute respiratory syndrome coronavirus 2 (SARS-CoV-2). The linear DNA product may be used to produce a seasonal, pandemic, prophylactic or therapeutic vaccine.

Thus, the invention provides the use of the linear DNA product described herein in the production of a vaccine. The invention also provides the use of the linear DNA product produced or obtainable by the methods described herein in the production of a vaccine.

The linear DNA product may encode an antigen, which may cause an immune response in a subject. The subject may be human. Preferably, the antigen is encoded on the DNA cassette. The linear DNA product may encode a neoantigen, which may cause an immune response in a subject. Preferably, the neoantigen is encoded on the DNA cassette.

### CAR-T cells

The invention provides the use of a linear DNA product described herein in the production of a CAR-T cell. The invention provides the use of the linear DNA product produced or obtainable by the methods described herein in the production of a CAR-T cell.

The invention provides a method for producing a genetically engineered CAR-T cell comprising: (a) introducing the linear DNA product described herein into a T cell; and (b) expressing a gene of interest encoded by the linear DNA product. Preferably, the gene of interest is a tumour-specific CAR.

The invention provides a method for producing a genetically engineered CAR-T cell comprising: (a) introducing the linear DNA product obtainable by the methods described herein into a T cell; and (b) expressing a gene of interest encoded by the linear DNA product. Preferably, the gene of interest is a tumour-specific CAR.

The method may further comprise, before step (a) (i.e. introducing the linear DNA product into a T cell), a step of removing mononuclear cells from a patient. Preferably, the step of removing is performed using leukapheresis. Preferably the mononuclear cells are T cells. The method may further comprise, after step (b) (i.e. expressing a gene of interest), a step of returning the genetically engineered cells to the patient.

The invention also provides an engineered T cell obtainable by any methods described herein. The engineered T cell may be suitable for use in CAR T-cell therapy.

### CRISPR delivery

The products produced by the methods described herein are particularly suitable for use in delivery of CRISPR machinery to cells, for example in cell therapy or in vivo therapy.

Various different cargos and delivery vehicles are used commonly for delivery of CRISPR machinery, including physical delivery methods (e.g. microinjection; electroporation), viral delivery methods (e.g. adeno-associated virus (AAV); full-sized adenovirus and lentivirus), and non-viral delivery methods (e.g. liposomes; polyplexes; gold particles).

The linear DNA product may comprise a gene sequence encoding any component of the CRISPR machinery. The linear DNA product may encode all components of the CRISPR machinery.

The linear DNA product may comprise (or encode) a repair template (or editing template). The repair template (or editing template) may be for editing genomes, for example, using the CRISPR-Cas system. The repair template (or editing template) may comprise or consist of a homology region (e.g. homology arm) which is homologous to the desired DNA region (i.e. target molecule). The repair template (or editing template) may be for use in CRISPR-Cas mediated homology directed repair (HDR). The repair template (or editing template) may be used to repair a target molecule having a strand break (e.g. a single stranded break or a double stranded break). The strand break may be created by a nuclease of the CRISPR system (e.g. Cpf1 or Mad7). The repair template (or editing template) may introduce at least one mutation (e.g. an insertion, deletion, and/or substitution) in the desired DNA region (i.e. target molecule). The repair template (or editing template) may be at least 10, 20, 30, 40, 50, 100, 200, 300, 400, 500, 600, 700, 800, 900, 1000, 1500, 2000, 2500, 3000, 3500, 4000, 4500, 5000, 5500, 6000, 6500, 7000, 7500, 8000, 8500, 9000, 9500, or 10000 base pairs long. The linear DNA product encoding the repair template may be delivered to a cell by a nanoparticle, a non-viral vector or a viral vector, or without the aid of any carrier. The linear DNA product encoding the repair template may be delivered to a cell by electroporation. The linear DNA product encoding the repair template may be delivered to a cell by hydrodynamic needle.

The linear DNA product may comprise (or further comprise) a gene sequence encoding a nuclease protein of a CRISPR system (e.g. Cas9, Cpf1 or Mad7), and/or a guide RNA. The linear DNA product may comprise (or further comprise) a gene sequence encoding a nuclease protein of a CRISPR system (e.g. Cas9, Cpf1, or Mad7). The linear DNA product may comprise (or further comprise) a gene sequence encoding a guide RNA. The linear DNA product may comprise (or further comprise) a gene sequence encoding a nuclease protein of a CRISPR system (e.g. Cas9, Cpf1, or Mad7) and a guide RNA. The linear DNA product may comprise (or further comprise) a gene sequence encoding a genomic target to be modified (e.g. a spacer). The linear DNA product may be ligated to a vector. The vector may comprise a sequence of some of the components of the CRISPR system. The vector may comprise a sequence of guide RNA or a sequence of a part of the guide RNA. If the vector comprises a sequence of a part of the guide RNA, the linear DNA product may comprise the missing sequence part of the guide RNA, so that upon ligation, the ligated vector comprises a full sequence of a guide RNA. The nuclease of the CRISPR system and guide RNA may be encoded on a single vector or on two different vectors. The linear DNA product may encode the nuclease of the CRISPR system and guide RNA. One linear DNA product may encode the nuclease of the CRISPR system, and the other linear DNA product may encode guide RNA.

The linear DNA product may comprise (or further comprise) a gene sequence encoding a nuclease protein of a CRISPR system (e.g. Cas9, Cpf1 or Mad7), and/or a gene sequence encoding a DNA polymerase, a reverse transcriptase, or an exonuclease. The linear DNA product may comprise (or further comprise) a gene sequence encoding a DNA polymerase, a reverse transcriptase or an exonuclease. The linear DNA product may comprise (or further comprise) a gene sequence encoding a nuclease protein of a CRISPR system (e.g. Cas9, Cpf1, or Mad7) and a gene sequence encoding a DNA polymerase, a reverse transcriptase or an exonuclease.

The linear DNA product may be for use in CRISPR-Cas mediated repair by recombination, homology-directed repair, or non-homologous end joining.

If the nuclease of the CRISPR system and the guide RNA are encoded by a different linear DNA product, they may be part of a different or the same delivery mechanism. For example, the linear DNA product encoding the nuclease of the CRISPR system may be delivered to a cell by a first nanoparticle, non-viral vector or viral vector, whereas the linear DNA product encoding the guide RNA may be delivered to a cell by a second nanoparticle, a non-viral vector, or viral vector. For example, the linear DNA product encoding the nuclease of the CRISPR system and the linear DNA product encoding the guide RNA may be delivered to a cell by the same nanoparticle, non-viral vector or viral vector.

If the nuclease of the CRISPR system and the guide RNA are encoded by the same linear DNA product (or by a vector that comprises the linear DNA product) they may be part of the same delivery mechanism. For example, the linear DNA product, or the vector, encoding the nuclease of the CRISPR system and the guide RNA may be delivered to a cell by a nanoparticle, a non-viral vector or a viral vector.

The linear DNA product encoding the nuclease of the CRISPR system and the linear DNA product encoding the guide RNA may be delivered to a cell by electroporation. The linear DNA product encoding the nuclease of the CRISPR system and the linear DNA product encoding the guide RNA may be delivered to a cell by hydrodynamic needle.

Thus, the invention provides the linear DNA product described herein for use in CRISPR system delivery to a cell. The invention provides a method of delivering the CRISPR system to a cell, comprising contacting the linear DNA product described herein with a cell. The invention also provides the linear DNA product obtainable by the methods described herein for use in CRISPR system delivery to a cell. The invention provides a method of delivering the CRISPR system to a cell, comprising contacting the linear DNA product obtainable by the methods described herein with a cell.

The cell may be an animal cell, preferably mammal cell, such as human cell.

The invention also provides a cell obtainable by the methods described herein. The cell is particularly suitable for use in cell therapy and/or in vivo therapy.

### mRNA generation

The linear DNA produced or obtainable by the methods described herein may be used as a template for in vitro transcription (IVT).

The linear DNA product produced or obtainable by the methods described herein may be used in transcription, to generate RNA, preferably mRNA, in vitro or in vivo.

The linear DNA product produced or obtainable by the methods described herein may be used in an in vitro transcription (IVT) reaction to generate mRNA which encodes a gene editing nuclease (e.g. Cas9, Cpf1, or Mad7).

A first linear DNA product may encode a nuclease of the CRISPR system (e.g. Cas9, Cpf1, or Mad7) and second linear DNA product may encode a guide RNA. The first and second DNA products may be used as templates for an IVT reaction to generate an mRNA encoding the nuclease of the CRISPR system and a gRNA.

The linear DNA product may comprise (or further comprise) a gene sequence encoding for therapeutic target and a T7 or SP6 promoter. The therapeutic target may comprise or consist of functional gene, vaccine antigen or neoantigen. The therapeutic target may be at least 30, at least 40, at least 50, at least 100, at least 200, at least 300, at least 400, at least 500, at least 600, at least 700, at least 800, at least 900, at least 1000, at least 1500, at least 2000, at least 2500, at least 3000, at least 3500, at least 4000, at least 4500, at least 5000, at least 5500, at least 6000, at least 6500, at least 7000, at least 7500, at least 8000, at least 8500, at least 9000, at least 9500, or at least 10000 base pairs long.

The linear DNA product may comprise (or further comprise) a gene sequence encoding for any component of self-amplifying mRNA system. The linear DNA product may comprise (or further comprise) a gene sequence encoding for all the components of a self-amplifying mRNA system.

The linear DNA product may comprise (or further comprise) a sequence encoding for tRNA or siRNA.

The linear DNA product encoding the nuclease of the CRISPR system and the linear DNA product encoding the guide RNA may be used to treat any disease or disorder. For example, the linear DNA product encoding the nuclease of the CRISPR system and the linear DNA product encoding the guide RNA may be used to treat one or more of the diseases and/or disorders selected from genetic disorders (e.g. monogenic disorders), cancer, HIV, other viral infections (e.g. infection caused by coronavirus (e.g. COVID-19), hepatitis A, hepatitis B, herpes simplex virus type 2, influenza, measles and/or respiratory syncytial virus), neurodegenerative diseases (e.g. Parkinson's disease and/or polyglutamine diseases such as Huntington's disease), ocular diseases (e.g. macular degeneration) and liver failure. Preferably, the linear DNA product is used to treat a genetic disorder. More preferably still, the linear DNA product encoding the nuclease of the CRISPR system and the linear DNA product encoding the guide RNA is used to treat a monogenic disorder. For example, the linear DNA product encoding the nuclease of the CRISPR system and the linear DNA product encoding the guide RNA, may be used to treat sickle cell anaemia, cystic fibrosis, Huntington disease, and Duchenne's Muscular Dystrophy, Haemophilia A, α1-antitrypsin deficiency, primary ciliary dyskinesia, or respiratory distress syndrome of prematurity.

The linear DNA product may comprise (or further comprise) a sequence encoding for self-amplifying mRNA (SAM). The linear DNA product may comprise (or further comprise) a sequence encoding for tRNA. The linear DNA product may comprise (or further comprise) a sequence encoding for circular mRNA (or circRNA).

### Kits

The invention provides a kit comprising components required to carry out the methods described herein.

The kit may comprise:
(a) a primase/polymerase (e.g. *Tth*PrimPol),
(b) a DNA polymerase (e.g. a strand displacing polymerase),
(c) a single-stranded DNA binding protein (e.g. gp32).

The kit may comprise:
(a) a primase/polymerase (e.g. *Tth*PrimPol),
(b) a DNA polymerase (e.g. a strand displacing polymerase),
(c) a single-stranded DNA binding protein (e.g. gp32);
(d) an endonuclease; and
(e) a ligase.

The kit may comprise:
(a) a primase/polymerase (e.g. *Tth*PrimPol),
(b) a DNA polymerase (e.g. a strand displacing polymerase),
(c) a single-stranded DNA binding protein (e.g. gp32);
(d) an endonuclease;
(e) a ligase, and
(f) adaptor molecules.

The kit may further comprise a double-stranded DNA molecule.

The strand-displacing polymerase may be Phi29 DNA polymerase or a mutant thereof which retains functionality (e.g. QualiPhi^{®}, a chimeric form of Phi29 DNA polymerase from 4basebio).

The kit may further comprise an exonuclease e.g. exonuclease I, exonuclease III and/or exonuclease VIII.mThe endonuclease may be any endonuclease described herein. Preferably, the endonuclease is a Type IIS restriction enzyme. The ligase may a DNA ligase, such as a T4 DNA ligase, T7 DNA ligase, mammalian DNA ligase I, III and IV; Taq DNA ligase, Tth DNA ligase, or *E*. *coli* DNA ligase.

Each aspect or embodiment as defined herein may be combined with any other aspect(s) or embodiment(s) unless clearly indicated to the contrary. In particular, any feature indicated as being preferred or advantageous may be combined with any other feature or features indicated as being preferred or advantageous.

The foregoing detailed description has been provided by way of explanation and illustration, and is not intended to limit the scope of the appended claims. Many variations in the presently preferred embodiments illustrated herein will be apparent to one of ordinary skill in the art, and remain within the scope of the appended claims and their equivalents.

Various publications are cited herein, the disclosures of which are incorporated by reference in their entireties.

### BRIEF DESCRIPTION OF THE DRAWINGS

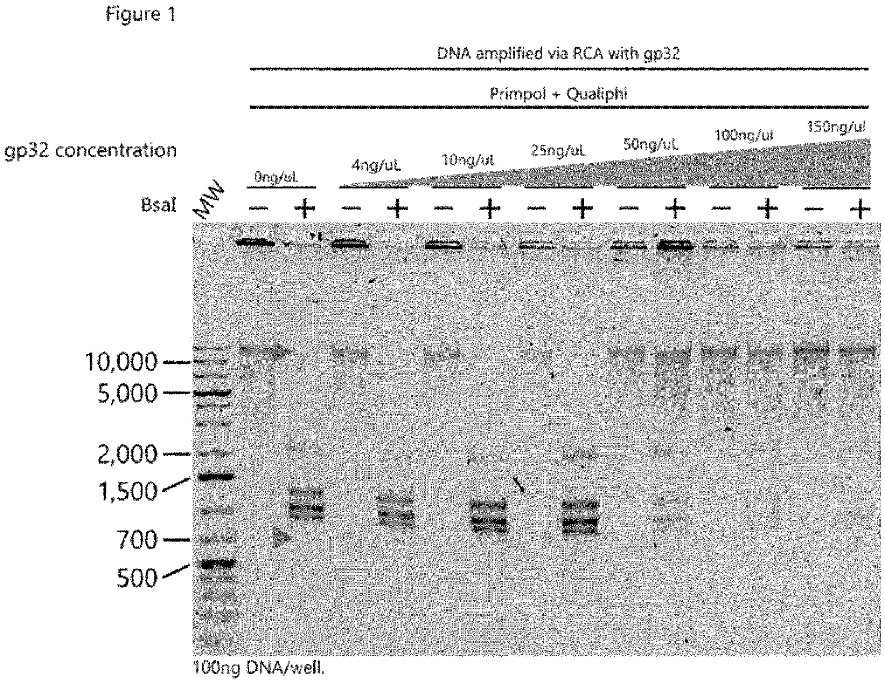

Figure 1 illustrates an agarose gel showing the results of a RCA reaction in the presence of TthPrimPol, QualiPhi DNA polymerase and increasing concentrations of bacteriophage T4 Gene 32 Protein (gp32). The double-stranded DNA molecule produced during the RCA reaction was subjected to a restriction endonuclease (Bsal) to produce a linear DNA product of 1346 base pairs. Red arrows indicate undesired DNA smears (i.e. DNA molecules which were either incorrectly digested or undigested) of over 10,000 base pairs, and under 1,000 base pairs.

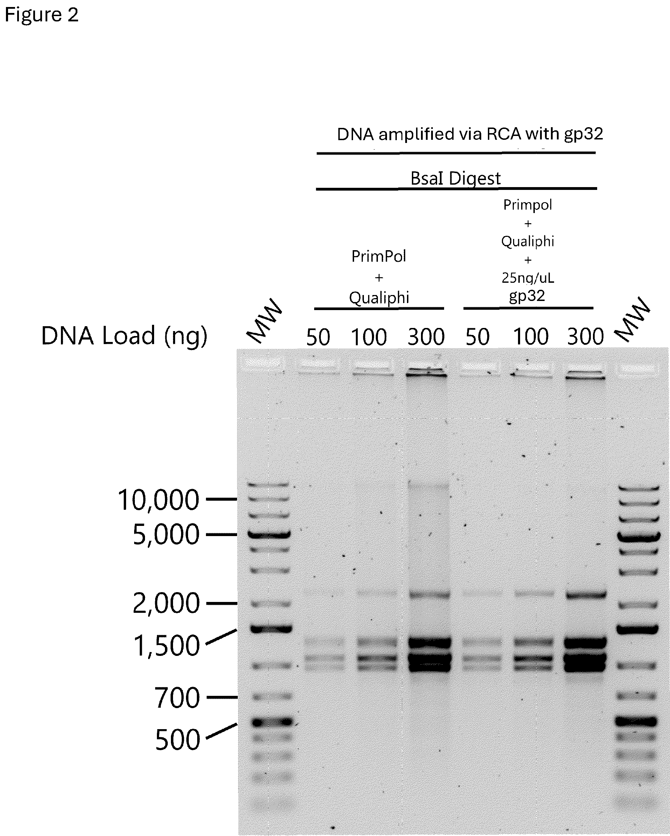

Figure 2 illustrates an agarose gel showing the results of a RCA reaction in the presence of *Tth*PrimPol, QualiPhi DNA polymerase and, optionally, bacteriophage T4 Gene 32 Protein (gp32) at a concentration of 25 ng/ µL. The double-stranded DNA molecule produced during the RCA reaction was subjected to a restriction endonuclease (Bsal) to produce a linear DNA product of 1346 base pairs. The wells of the agarose gel were loaded with 50ng, 100ng and 300ng of the linear DNA product per well, respectively, to visualize any undesired DNA smears.

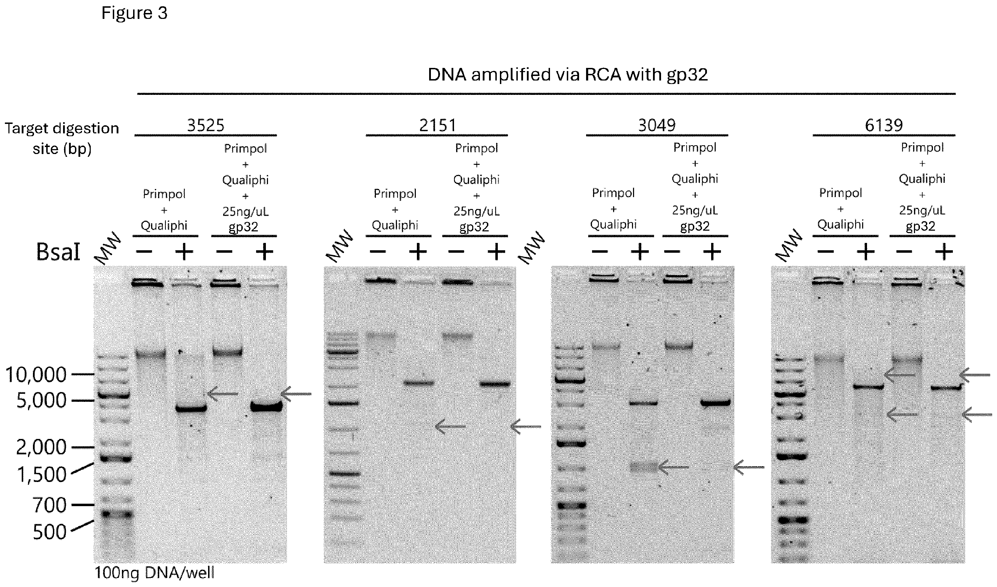

Figure 3 illustrates an agarose gel showing the results of RCA reactions in the presence of *Tth*PrimPol, QualiPhi DNA polymerase and, optionally, bacteriophage T4 Gene 32 Protein (gp32) at a concentration of 25 ng/ µL for four different template molecules with target digestion size of 3525 bp, 2151 bp, 3049 bp, and 6139 bp, respectively. The double-stranded DNA molecules produced during the RCA reaction was subjected to a restriction endonuclease (Bsal) to produce linear DNA products of the target digestion size. Red arrows indicate undesired DNA smears (i.e. DNA molecules which were either incorrectly digested or undigested) of varied sizes (bp).

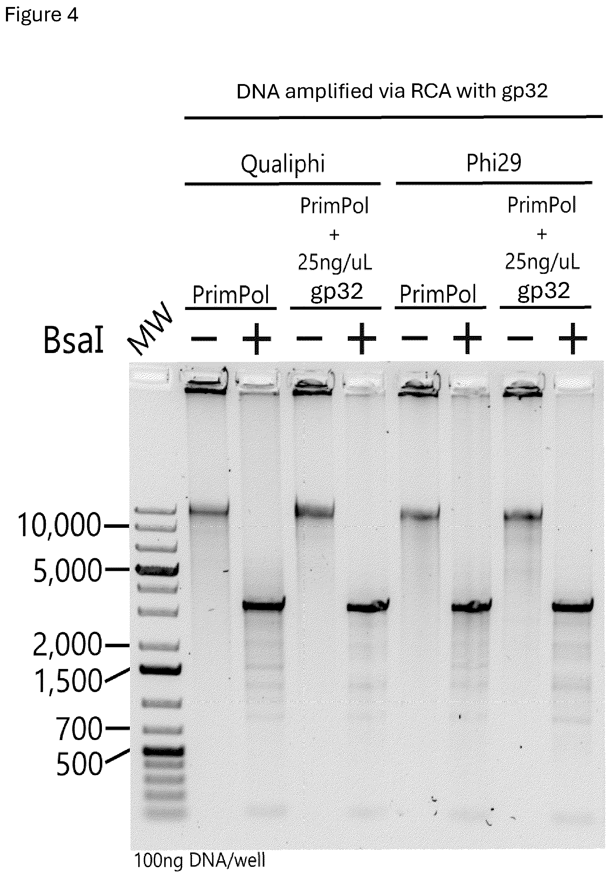

Figure 4 illustrates an agarose gel showing the results of a RCA reaction in the presence of TthPrimPol, different DNA polymerases and, optionally, bacteriophage T4 Gene 32 Protein (gp32) at a concentration of 25 ng/ µL. The double-stranded DNA molecule produced during the RCA reaction was subjected to a restriction endonuclease (Bsal) to produce a linear DNA product of 3525 base pairs.

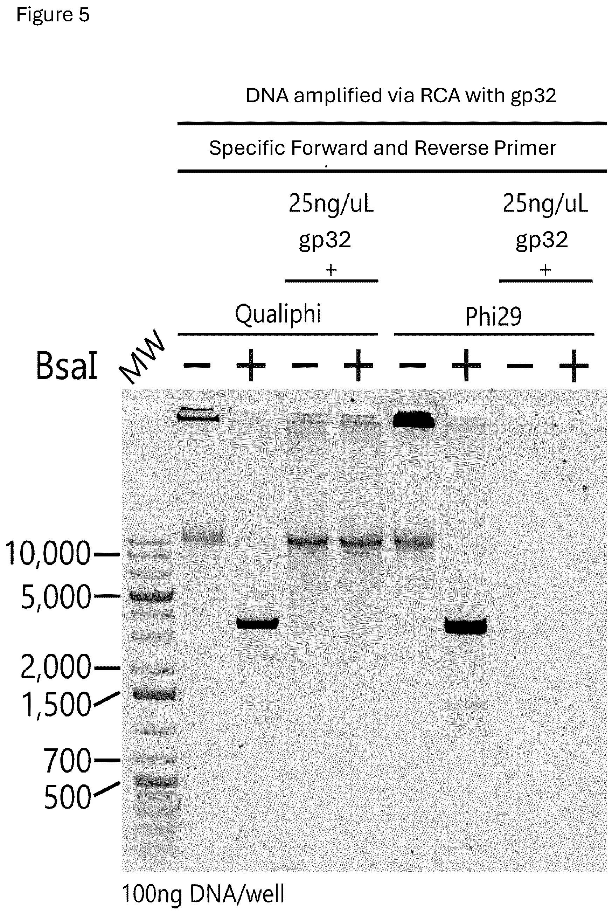

Figure 5 illustrates an agarose gel showing the results of a RCA reaction in the presence of specific forward and reverse primers, different DNA polymerases and, optionally, bacteriophage T4 Gene 32 Protein (gp32) at a concentration of 25 ng/ µL. The double-stranded DNA molecule produced during the RCA reaction was subjected to a restriction endonuclease (Bsal) to produce a linear DNA product of 3525 base pairs.

### CLAUSES

The invention is further defined with reference to the following clauses.
1. A method for amplifying a DNA template molecule, wherein the method comprises:
   amplifying a DNA template molecule to generate a double-stranded DNA molecule, wherein the DNA template molecule is amplified by rolling circle amplification in the presence of (i) a single-stranded DNA binding protein that enhances the efficiency of amplification, and (ii) a primase/polymerase.
2. The method of clause 1, wherein the template molecule comprises at least one endonuclease target sequence.
3. A method for producing a linear deoxyribonucleic acid (DNA) product, wherein the method comprises:
   (a) amplifying a DNA template molecule comprising at least one endonuclease target sequence to generate a double-stranded DNA molecule, wherein the DNA template molecule is amplified by rolling circle amplification in the presence of (i) a single-stranded DNA binding protein that enhances the efficiency of amplification, and (ii) a primase/polymerase; and
   (b) contacting the double-stranded DNA molecule with an endonuclease to generate the linear DNA product.
4. A method for producing a closed linear deoxyribonucleic acid (DNA) product, wherein the method comprises:
   (a) amplifying a DNA template molecule comprising at least one endonuclease target sequence to generate a double-stranded DNA molecule, wherein the DNA template molecule is amplified by rolling circle amplification in the presence of (i) a single-stranded DNA binding protein that enhances the efficiency of amplification, and (ii) a primase/polymerase;
   (b) contacting the double-stranded DNA molecule with an endonuclease, a ligase and first and second adaptor molecules to form a single contiguous aqueous volume; and
   (c) incubating the single contiguous aqueous volume to generate the closed linear DNA product, wherein the closed linear DNA product comprises a linear double-stranded region, wherein the linear double-stranded region comprises a linear portion of the double-stranded DNA molecule, and wherein the linear double-stranded region is closed at a first end by the first adaptor molecule and closed at a second end by the second adaptor molecule.
5. A method for producing a linear deoxyribonucleic acid (DNA) product, wherein the method comprises:
   (a) amplifying a DNA template molecule comprising at least one endonuclease target sequence to generate a double-stranded DNA molecule, wherein the DNA template molecule is amplified by rolling circle amplification in the presence of (i) a single-stranded DNA binding protein that enhances the efficiency of amplification, and (ii) a primase/polymerase;
   (b) contacting the double-stranded DNA molecule with an endonuclease, a ligase and first and second adaptor molecules to form a single contiguous aqueous volume; and
   (c) incubating the single contiguous aqueous volume to generate the linear DNA product,
   wherein the linear DNA product comprises a linear double-stranded region, wherein the linear double-stranded region comprises a linear portion of the double-stranded DNA molecule, and wherein the first adaptor molecule is ligated to a first end of the linear double-stranded region and the second adaptor molecule is ligated to a second end of the linear double-stranded region, and wherein the first and second adaptor molecules are nucleic acid molecules that comprise one or more nuclease-resistant nucleotides.
6. A method for producing a partially closed deoxyribonucleic acid (DNA) product, wherein the method comprises:
   (a) amplifying a DNA template molecule comprising at least one endonuclease target sequence to generate a double-stranded DNA molecule, wherein the DNA template molecule is amplified by rolling circle amplification in the presence of (i) a single-stranded DNA binding protein that enhances the efficiency of amplification, and (ii) a primase/polymerase;
   (b) contacting the double-stranded DNA molecule with an endonuclease, a ligase and first and second adaptor molecules to form a single contiguous aqueous volume; and
   (c) incubating the single contiguous aqueous volume to generate the partially closed linear DNA product, wherein the partially closed linear DNA product comprises a linear double-stranded region,
   wherein the linear double-stranded region comprises a linear portion of the double-stranded DNA molecule, and wherein the first adaptor molecule is ligated to a first end of the linear double-stranded region and the second adaptor molecule is ligated to a second end of the linear double-stranded region, and wherein the first adaptor molecule is a nucleic acid molecule that comprise one or more nuclease-resistant nucleotides, and wherein the linear double-stranded region is closed at the second end by the second adaptor molecule.
7. A method for producing a linear double-stranded DNA product, wherein the method comprises:
   (a) rolling circle amplification of a DNA template molecule comprising at least one endonuclease target sequence in the presence of: 1) at least one type of a nuclease-resistant nucleotide; and 2) a single-stranded DNA binding protein that enhances the efficiency of amplification, to generate a double-stranded DNA product,
   (b) contacting the double-stranded DNA product with at least one endonuclease to generate the linear double-stranded DNA product, wherein the linear double-stranded DNA product has increased resistance to nuclease digestion.
8. The method of any one of clauses 1-7, wherein the method comprises a step of providing a template molecule before the step of amplifying the DNA template molecule.
9. The method of any one of clauses 1-8, wherein the single-stranded DNA binding protein is bacteriophage T4 Gene 32 Protein (gp32).
10. The method of any one of clauses 1-9, wherein the primase/polymerase is TthPrimPol.
11. The method of any one of clauses 1-10, wherein the single-stranded DNA binding protein is at a concentration of less than 50 ng/µL.
12. The method of any one of clauses 1-11, wherein the single-stranded DNA binding protein is at a concentration of between 1 ng/µL and less than 50 ng/µL.
13. The method of any one of clauses 1-12, wherein the single-stranded DNA binding protein is at a concentration of between 4 ng/µL and 40 ng/µL, between 4 ng/µL and 30 ng/µL, between 4 ng/µL and 25 ng/µL, or between 10 ng/µL and 25 ng/µL.
14. The method of any one of clauses 1-13, wherein the single-stranded DNA binding protein is at a concentration of about 25 ng/µL.
15. The method of any one of clauses 1-14, wherein the step of amplification is carried out at a temperature of between 20°C and 40°C, or between 25°C and 35°C, preferably about 30°C.
16. The method of any one of clauses 1-15, wherein the step of amplification is carried out for at least 10 hours, at least 12 hours, at least 14 hours, at least 16 hours, at least 18 hours, or at least 20 hours, preferably about 20 hours.
17. wherein the step of amplification is carried out for less than 24 hours, or less than 22 hours, preferably about 20 hours.
18. The method of any one of clauses 1-17, wherein the step of amplification is performed in the presence of a DNA polymerase.
19. The method of clause 18, wherein the DNA polymerase is a wild-type DNA polymerase, or a chimeric form of a wild-type DNA polymerase.
20. The method of clause 18 or clause 19, wherein the DNA polymerase is QualiPhi DNA polymerase or Phi29 DNA polymerase.
21. A method of amplifying of a DNA template molecule, wherein the method comprises:
   amplifying a DNA template molecule to generate a double-stranded DNA molecule, wherein the DNA template molecule is amplified by rolling circle amplification in the presence of (i) a single-stranded DNA binding protein that enhances the efficiency of amplification, and (ii) primers.
22. A method for producing a linear deoxyribonucleic acid (DNA) product, wherein the method comprises:
   (a) amplifying a DNA template molecule comprising at least one endonuclease target sequence to generate a double-stranded DNA molecule, wherein the DNA template molecule is amplified by rolling circle amplification in the presence of (i) a single-stranded DNA binding protein that enhances the efficiency of amplification, and (ii) primers; and
   (b) contacting the double-stranded DNA molecule with an endonuclease to generate the linear DNA product.
23. A method for producing a closed linear deoxyribonucleic acid (DNA) product, wherein the method comprises:
   (a) amplifying a DNA template molecule comprising at least one endonuclease target sequence to generate a double-stranded DNA molecule, wherein the DNA template molecule is amplified by rolling circle amplification in the presence of (i) a single-stranded DNA binding protein that enhances the efficiency of amplification, and (ii) primers;
   (b) contacting the double-stranded DNA molecule with an endonuclease, a ligase and first and second adaptor molecules to form a single contiguous aqueous volume; and
   (c) incubating the single contiguous aqueous volume to generate the closed linear DNA product,
   wherein the closed linear DNA product comprises a linear double-stranded region, wherein the linear double-stranded region comprises a linear portion of the double-stranded DNA molecule, and wherein the linear double-stranded region is closed at a first end by the first adaptor molecule and closed at a second end by the second adaptor molecule.
24. A method for producing a linear deoxyribonucleic acid (DNA) product, wherein the method comprises:
   (a) amplifying a DNA template molecule comprising at least one endonuclease target sequence to generate a double-stranded DNA molecule, wherein the DNA template molecule is amplified by rolling circle amplification in the presence of (i) a single-stranded DNA binding protein that enhances the efficiency of amplification, and (ii) primers;
   (b) contacting the double-stranded DNA molecule with an endonuclease, a ligase and first and second adaptor molecules to form a single contiguous aqueous volume; and
   (c) incubating the single contiguous aqueous volume to generate the linear DNA product,
   wherein the linear DNA product comprises a linear double-stranded region, wherein the linear double-stranded region comprises a linear portion of the double-stranded DNA molecule, and wherein the first adaptor molecule is ligated to a first end of the linear double-stranded region and the second adaptor molecule is ligated to a second end of the linear double-stranded region, and wherein the first and second adaptor molecules are nucleic acid molecules that comprise one or more nuclease-resistant nucleotides.
25. A method for producing a partially closed deoxyribonucleic acid (DNA) product, wherein the method comprises:
   (a) amplifying a DNA template molecule comprising at least one endonuclease target sequence to generate a double-stranded DNA molecule, wherein the DNA template molecule is amplified by rolling circle amplification in the presence of (i) a single-stranded DNA binding protein that enhances the efficiency of amplification, and (ii) primers;
   (b) contacting the double-stranded DNA molecule with an endonuclease, a ligase and first and second adaptor molecules to form a single contiguous aqueous volume; and
   (c) incubating the single contiguous aqueous volume to generate the partially closed linear DNA product, wherein the partially closed linear DNA product comprises a linear double-stranded region,
   wherein the linear double-stranded region comprises a linear portion of the double-stranded DNA molecule, and wherein the first adaptor molecule is ligated to a first end of the linear double-stranded region and the second adaptor molecule is ligated to a second end of the linear double-stranded region, and wherein the first adaptor molecule is a nucleic acid molecule that comprise one or more nuclease-resistant nucleotides, and wherein the linear double-stranded region is closed at the second end by the second adaptor molecule.
26. The method of any one of clauses 21-25, wherein the step of amplification is performed in the presence of a DNA polymerase.
27. The method of clause 26, wherein the DNA polymerase is a chimeric form of a wild-type DNA polymerase.
28. The method of clause 26 or clause 27, wherein the DNA polymerase is QualiPhi DNA polymerase.

### SEQUENCES DISCLOSED IN THE APPLICATION

| **SEQ ID NO** | **Sequence 5'-3' or N'-C'** | **name** |
|---|---|---|
| 1 | MRPIEHALSYAAQGYGVLPLRPGGKEPLGKLVPHGLKNASRDP ATLEAWWRSCPRCGVGILPGPEVLVLDFDDPEAWEGLRQEHP ALEAAPRQRTPKGGRHVFLRLPEGVRLSASVRAIPGVDLRGMG RAYVVAAPTRLKDGRTYTWEAPLTPPEELPPVPQALLLKLLPPP PPPRPSWGAVGTASPKRLQALLQAYAAQVARTPEGQRHLTLIR YAVAAGGLIPHGLDPREAEEVLVAAAMSAGLPEWEARDAVRW GLGVGASRPLVLESSSKPPEPRTYRARVYARMRRWV | *Tth*PrimPol |
| 2 | MRPIEHVLGYARLGYAVLPLLPGEKRPHPRLVPHGLKEASRDPA TLEAWWRSCPQAGAGILPAPEVLVLDFDDPEAWERLKEEHPTL LEAPRARTPRGGVHVYLRLPPEAVGRLSASVRAIPGVDLRGLG RSYLVAPPTTLPTGAYVWEVPLRRPEELPPVPAPLLARLLPPPP PPREVWTPVEGVSPKRLQALLQAYAAQVAHTPEGQRHNTLIRY AVAAGGLLPHGLDPREAEEALVAAAMSAGLPEAEARAAARWGL EVGASRPLALEPSLAPSGPRTYRARVYARMRRWA | PrimPol (i.e. *Tth*PrimPol2) |
| 3 | MGSSHHHHHHSSGLVPRGSHMRPIEHALSYAAQGYGVLPLRP GGKEPLGKLVPHGLKNASRDPATLEAWWRSCPRCGVGILPGP EVLVLDFDDPEAWEGLRQEHPALEAAPRQRTPKGGRHVFLRLP EGVRLSASVRAIPGVDLRGMGRAYVVAAPTRLKDGRTYTWEAP LTPPEELPPVPQALLLKLLPPPPPPRPSWGAVGTASPKRLQALL QAYAAQVARTPEGQRHLTLIRYAVAAGGLIPHGLDPREAEEVLV AAAMSAGLPEWEARDAVRWGLGVGASRPLVLESSSKPPEPRT YRARVYARMRRWV | His tag + *Tth*PrimPol |
| 4 | MGSSHHHHHHSSGLVPRGSHMRPIEHVLGYARLGYAVLPLLPG EKRPHPRLVPHGLKEASRDPATLEAWWRSCPQAGAGILPAPEV LVLDFDDPEAWERLKEEHPTLLEAPRARTPRGGVHVYLRLPPE AVGRLSASVRAIPGVDLRGLGRSYLVAPPTTLPTGAYVWEVPLR RPEELPPVPAPLLARLLPPPPPPREVWTPVEGVSPKRLQALLQA YAAQVAHTPEGQRHNTLIRYAVAAGGLLPHGLDPREAEEALVA AAMSAGLPEAEARAAARWGLEVGASRPLALEPSLAPSGPRTYR ARVYARMRRWA | His tag + PrimPol (i.e. *Tth*PrimPol2) |
| 5 | MFKRKSTAELAAQMAKLNGNKGFSSEDKGEWKLKLDNAGNGQ AVIRFLPSKNDEQAPFAILVNHGFKKNGKWYIETCSSTHGDYDS CPVCQYISKNDLYNTDNKEYSLVKRKTSYWANILVVKDPAAPEN EGKVFKYRFGKKIWDKINAMIAVDVEMGETPVDVTCPWEGANF VLKVKQVSGFSNYDESKFLNQSAIPNIDDESFQKELFEQMVDLS EMTSKDKFKSFEELNTKFGQVMGTAVMGGAAATAAKKADKVA DDLDAFNVDDFNTKTEDDFMSSSSGSSSSADDTDLDDLLNDL | gp32 |
| 6 | CAGCCACAACGTCTATATCA | Forward primer |
| 7 | GAACTTGTGGCCGTTTAC | Reverse primer |

### EXAMPLES

### Example 1 - Testing single-stranded DNA binding protein concentrations

A DNA template molecule comprising a 1346 base pairs DNA cassette (flanked by Bsal restriction enzyme sites) underwent rolling circle amplification (RCA) in the presence of TthPrimPol (SEQ ID NO: 1; 4basebio), QualiPhi DNA polymerase (4basebio) and increasing concentrations (0-150 ng/µL) of bacteriophage T4 Gene 32 Protein (gp32) (New England Labs). The double-stranded DNA molecule produced during the RCA reaction was subjected to Bsal digestion to produce a linear DNA product of 1346 base pairs.

Figure 1 illustrates an agarose gel showing the linear DNA product after Bsal digestion. In the control reaction (0ng/µL of gp32), DNA smears ranging in size from 400 bp - 20,000 bp (red arrows) are observed alongside the linear DNA product. The addition of gp32 shows reduction in the smear intensity at the gp32 concentrations ranging from 4ng/µL to 25ng/µL. The most notable improvement in the DNA quality is observed at a gp32 concentration of 25ng/µL. The addition of gp32 at concentrations of or above 50 ng/µL produces some DNA material that is not digestible by the Bsal restriction enzyme. It can be hypothesized that this is likely single stranded DNA, as literature associates gene 32 protein, and other ssDNA-binding proteins, with bias towards ssDNA synthesis during *in vitro* DNA replication processes.

In conclusion, an RCA reaction in the presence of *Tth*PrimPol, QualiPhi DNA polymerase, and bacteriophage T4 Gene 32 Protein (gp32) at a concentration of less than 50ng/µL produces a stable and homogenous linear dsDNA product with low amount of DNA contamination. The best results are observed for gp32 at a concentration of between 4 g/µL and 25 ng/µL.

### Detailed methods:

RCA: The circular DNA template molecule (0.2ng/µL) was denatured in equivolume of Buffer D (400mM KOH [Melford] and 10mM EDTA [Invitrogen]), and neutralised with equivolume of Buffer N, (400mM HCl [Fisher Scientific] and 600mM Tris-HCl [Invitrogen, pH 7.5]). The denatured DNA template molecule was used in the amplification reaction containing 1x RCA Buffer (22.5mM (NH₄)₂SO₄ [Melford], 50mM Tris-HCl pH 7.5 [Invitrogen], 100mM MgCl₂ [Melford]), and 1mM DTT (Merck), 1mM dNTPs (Larova), 0.2ng/µL Pyrophosphatase (4basebio), 3.4ng/µL *Tth*PrimPol I (4basebio), 16ng/µL QualiPhi DNA polymerase (4basebio), and T4 Gene 32 Protein at concentrations ranging from 0ng/µL to 150ng/µL(New England Biolabs). The reaction was then incubated for 20 hours at 30°C, followed by heat inactivation for 10 minutes at 55°C.

The resulting RCA DNA material was digested with Bsal restriction enzyme. The digestion reactions involved digestion of 10µg of DNA from each RCA reaction using 1x CutSmart Buffer (New England Biolabs) and 4U/µg of DNA Bsal-HFv2 (New England Biolabs). The reaction was incubated for 1 hour at 37°C. The unprocessed RCA DNA (Bsal -) and the digested DNA (Bsal +) was resolved by agarose gel electrophoresis with DNA load of 100ng per well.

### Example 2 - Testing single-stranded DNA binding protein at a concentration of 25 ng/µL

A DNA template molecule comprising a 1346 base pairs DNA cassette (flanked by Bsal restriction enzyme sites) underwent rolling circle amplification (RCA) in the presence of *Tth*PrimPol (SEQ ID NO: 1; 4basebio), QualiPhi DNA polymerase (4basebio) and, optionally, bacteriophage T4 Gene 32 Protein (gp32) at a concentration of 25ng/µL (New England Labs). The double-stranded DNA molecule produced during the RCA reaction was subjected to Bsal digestion to produce a linear DNA product of 1346 base pairs.

Figure 2 illustrates an agarose gel showing the linear DNA product after Bsal digestion. The wells of the agarose gel were loaded with 50ng, 100ng and 300ng of the linear DNA product per well, respectively, to visualize any undesired DNA smears (i.e. DNA molecules which were either incorrectly digested or undigested). The use of gp32 at a concentration of 25ng/µL during the RCA reaction shows a significant reduction of undigested or incorrectly digested DNA molecules. This is clearly observed even when a high concentration of the DNA product is loaded on the agarose gel.

In conclusion, an RCA reaction in the presence of *Tth*PrimPol, QualiPhi DNA polymerase, and bacteriophage T4 Gene 32 Protein (gp32) at a concentration of 25ng/µL produces a stable and homogenous linear DNA product with low (if any) amount of DNA contamination.

### Detailed methods

The RCA and endonuclease digestion reaction conditions are as in Example 1, with the exception that gp32 was used only at a concentration of 25ng/µL. The digested DNA was resolved by agarose gel electrophoresis with the DNA load of 50ng, 100ng or 300ng per well.

### Example 3 - Testing different DNA template molecules

Different DNA template molecules comprising 3525 bp, 2151 bp, 3049 bp, or 6139 bp DNA cassettes, respectively (each flanked by Bsal restriction enzyme sites) underwent rolling circle amplification (RCA) in the presence of TthPrimPol (SEQ ID NO: 1; 4basebio), QualiPhi DNA polymerase (4basebio) and, optionally, bacteriophage T4 Gene 32 Protein (gp32) at a concentration of 25ng/µL (New England Labs). The double-stranded DNA molecules produced during the RCA reactions were subjected to Bsal digestion to produce linear DNA products of 3525 bp, 2151 bp, 3049 bp, or 6139 bp, respectively.

Figure 3 illustrates an agarose gel showing the results of RCA reactions in the presence of *Tth*PrimPol, QualiPhi DNA polymerase and, optionally, bacteriophage T4 Gene 32 Protein (gp32) at a concentration of 25 ng/µL for four different DNA template molecules with target digestion size (i.e. the DNA cassette) of 3525 bp, 2151 bp, 3049 bp, and 6139 bp, respectively. The use of gp32 at a concentration of 25ng/µL during the RCA reaction shows a significant reduction of undigested or incorrectly digested DNA molecules, regardless of DNA template molecule size, and the DNA cassette size. The most notable improvements in the reduction of the DNA smear are highlighted in Figure 3 with red arrows.

In conclusion, an RCA reaction in the presence of *Tth*PrimPol, QualiPhi DNA polymerase, and bacteriophage T4 Gene 32 Protein (gp32) at a concentration of 25ng/µL is able to produce stable and homogenous linear DNA products with little (if any) DNA contamination for different DNA template molecules (of different DNA cassette size).

### Detailed methods

The RCA and endonuclease digestion reaction conditions are as in Examples 1 and 2 with the exception that four different DNA template molecules were used (as discussed above).

### Example 4 - Testing different DNA polymerases

A DNA template molecule comprising a 3525 base pairs DNA cassette (flanked by Bsal restriction enzyme sites) underwent rolling circle amplification (RCA) in the presence of *Tth*PrimPol (SEQ ID NO: 1; 4basebio), different DNA polymerases (QualiPhi DNA polymerase, and Phi29 DNA polymerase) and, optionally, bacteriophage T4 Gene 32 Protein (gp32) at a concentration of 25ng/µL (New England Labs). The double-stranded DNA molecule produced during the RCA reaction was subjected to Bsal digestion to produce a linear DNA product of 3525 base pairs.

Figure 4 illustrates an agarose gel showing the results of a RCA reaction in the presence of *Tth*PrimPol, different DNA polymerases and, optionally, bacteriophage T4 Gene 32 Protein (gp32) at a concentration of 25 ng/µL. The RCA reactions in the presence of either QualiPhi DNA polymerase or Phi29 DNA polymerase produced the expected linear DNA product of 3525bp upon digestion with the Bsal restriction enzyme. As shown in previous figures, an improved reduction of the DNA smear surrounding the target DNA band can be observed when T4 Gene 32 Protein at a concentration of 25ng/µL is used in the amplification reaction.

In conclusion, an RCA reaction in the presence of *Tth*PrimPol, either QualiPhi DNA polymerase, or Phi29 DNA polymerase, and bacteriophage T4 Gene 32 Protein (gp32) at a concentration of 25ng/µL produces the most stable and homogenous linear DNA product with the lowest amount of DNA contamination.

### Detailed methods

The RCA and endonuclease digestion reaction conditions are as in Examples 1 and 2 with the exception that either 16ng/µL QualiPhi DNA polymerase (4basebio), or 14ng/µL Phi29 DNA polymerase (4basebio) are used.

### Example 5- Testing primers instead of primase/polymerase

A DNA template molecule comprising a 3525 base pairs DNA cassette (flanked by Bsal restriction enzyme sites) underwent rolling circle amplification (RCA) in the presence of sequence-specific forward and reverse primers, different DNA polymerases (QualiPhi DNA polymerase, or Phi29 DNA polymerase) and, optionally, bacteriophage T4 Gene 32 Protein (gp32) at a concentration of 25ng/µL (New England Labs). The double-stranded DNA molecule produced during the RCA reaction was subjected to Bsal digestion to produce a linear DNA product of 3525 base pairs.

Figure 5 illustrates an agarose gel showing the results of a RCA reaction in the presence of specific forward and reverse primers, different DNA polymerases and, optionally, bacteriophage T4 Gene 32 Protein (gp32) at a concentration of 25 ng/ µL. In some reactions, the double-stranded DNA molecule produced during the RCA reaction was subjected to Bsal digestion to produce a linear DNA product of 3525 base pairs. RCA reactions with specific primers in the absence of T4 Gene 32 Protein using QualiPhi DNA polymerase and Phi29 DNA polymerase resulted in the expected linear DNA product of 3525 bp after Bsal digestion. RCA reactions with specific primers in the presence of T4 Gene 32 Protein did not results in the production of the linear DNA product of 3525 bp after Bsal digestion. The RCA reaction with specific primers and with Phi29 DNA polymerase and in the presence of T4 Gene 32 Protein did not produce any amplification product.

In conclusion, the combination of specific primers and either QualiPhi DNA polymerase or Phi29 DNA polymerase during RCA results in the production of the target DNA product upon endonuclease digestion. The combination of specific primers, T4 Gene 32 Protein, and either QualiPhi DNA polymerase or Phi29 DNA polymerase during RCA does not result in the production of the target DNA product upon endonuclease digestion.

### Methods

The RCA and endonuclease digestion reaction conditions are as in Examples 1 and 2 with the exception that:
(1) either 16ng/µL QualiPhi DNA polymerase (4basebio), or 14ng/µL Phi29 DNA polymerase (4basebio) were used, and
(2) 400nM of a sequence-specific forward primer (IDT) (SEQ ID NO: 6: CAGCCACAACGTCTATATCA), and 400nM of a sequence-specific reverse primer (IDT) were used (SEQ ID NO: 7: GAACTTGTGGCCGTTTAC).

## Claims

1. A method for amplifying a DNA template molecule, wherein the method comprises amplifying a DNA template molecule to generate a double-stranded DNA molecule, wherein the DNA template molecule is amplified by rolling circle amplification in the presence of (i) a single-stranded DNA binding protein that enhances the efficiency of amplification, and (ii) a primase/polymerase.

2. The method of claim 1, wherein the template molecule comprises at least one endonuclease target sequence.

3. A method for producing a linear deoxyribonucleic acid (DNA) product, wherein the method comprises:
(a) amplifying a DNA template molecule comprising at least one endonuclease target sequence to generate a double-stranded DNA molecule, wherein the DNA template molecule is amplified by rolling circle amplification in the presence of (i) a single-stranded DNA binding protein that enhances the efficiency of amplification, and (ii) a primase/polymerase; and
(b) contacting the double-stranded DNA molecule with an endonuclease to generate the linear DNA product.

4. A method for producing a closed linear deoxyribonucleic acid (DNA) product, wherein the method comprises:
(a) amplifying a DNA template molecule comprising at least one endonuclease target sequence to generate a double-stranded DNA molecule, wherein the DNA template molecule is amplified by rolling circle amplification in the presence of (i) a single-stranded DNA binding protein that enhances the efficiency of amplification, and (ii) a primase/polymerase;
(b) contacting the double-stranded DNA molecule with an endonuclease, a ligase and first and second adaptor molecules to form a single contiguous aqueous volume; and
(c) incubating the single contiguous aqueous volume to generate the closed linear DNA product, wherein the closed linear DNA product comprises a linear double-stranded region, wherein the linear double-stranded region comprises a linear portion of the double-stranded DNA molecule, and wherein the linear double-stranded region is closed at a first end by the first adaptor molecule and closed at a second end by the second adaptor molecule.

5. A method for producing a linear deoxyribonucleic acid (DNA) product, wherein the method comprises:
(a) amplifying a DNA template molecule comprising at least one endonuclease target sequence to generate a double-stranded DNA molecule, wherein the DNA template molecule is amplified by rolling circle amplification in the presence of (i) a single-stranded DNA binding protein that enhances the efficiency of amplification, and (ii) a primase/polymerase;
(b) contacting the double-stranded DNA molecule with an endonuclease, a ligase and first and second adaptor molecules to form a single contiguous aqueous volume; and
(c) incubating the single contiguous aqueous volume to generate the linear DNA product,
wherein the linear DNA product comprises a linear double-stranded region, wherein the linear double-stranded region comprises a linear portion of the double-stranded DNA molecule, and wherein the first adaptor molecule is ligated to a first end of the linear double-stranded region and the second adaptor molecule is ligated to a second end of the linear double-stranded region, and wherein the first and second adaptor molecules are nucleic acid molecules that comprise one or more nuclease-resistant nucleotides.

6. A method for producing a partially closed deoxyribonucleic acid (DNA) product, wherein the method comprises:
(a) amplifying a DNA template molecule comprising at least one endonuclease target sequence to generate a double-stranded DNA molecule, wherein the DNA template molecule is amplified by rolling circle amplification in the presence of (i) a single-stranded DNA binding protein that enhances the efficiency of amplification, and (ii) a primase/polymerase;
(b) contacting the double-stranded DNA molecule with an endonuclease, a ligase and first and second adaptor molecules to form a single contiguous aqueous volume; and
(c) incubating the single contiguous aqueous volume to generate the partially closed linear DNA product, wherein the partially closed linear DNA product comprises a linear double-stranded region, wherein the linear double-stranded region comprises a linear portion of the double-stranded DNA molecule, and wherein the first adaptor molecule is ligated to a first end of the linear double-stranded region and the second adaptor molecule is ligated to a second end of the linear double-stranded region, and wherein the first adaptor molecule is a nucleic acid molecule that comprise one or more nuclease-resistant nucleotides, and wherein the linear double-stranded region is closed at the second end by the second adaptor molecule.

7. A method for producing a linear double-stranded DNA product, wherein the method comprises:
(a) rolling circle amplification of a DNA template molecule comprising at least one endonuclease target sequence in the presence of: (i) at least one type of a nuclease-resistant nucleotide; and (ii) a single-stranded DNA binding protein that enhances the efficiency of amplification, to generate a double-stranded DNA product,
(b) contacting the double-stranded DNA product with at least one endonuclease to generate the linear double-stranded DNA product, wherein the linear double-stranded DNA product has increased resistance to nuclease digestion.

8. The method of any one of claims 1-7, wherein the method comprises a step of providing a DNA template molecule before the step of amplifying the DNA template molecule.

9. The method of any one of claims 1-8, wherein the single-stranded DNA binding protein is bacteriophage T4 Gene 32 Protein (gp32).

10. The method of any one of claims 1-9, wherein the primase/polymerase is TthPrimPol.

11. The method of any one of claims 1-10, wherein the single-stranded DNA binding protein is at a concentration of less than 50 ng/µL.

12. The method of any one of claims 1-11, wherein the single-stranded DNA binding protein is at a concentration of between 1 ng/µL and less than 50 ng/µL.

13. The method of any one of claims 1-12, wherein the single-stranded DNA binding protein is at a concentration of between 4 ng/µL and 40 ng/µL, between 4 ng/µL and 30 ng/µL, between 4 ng/µL and 25 ng/µL, or between 10 ng/µL and 25 ng/µL.

14. The method of any one of claims 1-13, wherein the single-stranded DNA binding protein is at a concentration of about 25 ng/µL.

15. The method of any one of claims 1-14, wherein the step of amplification is performed in the presence of a highly processive DNA polymerase, preferably wherein the highly processive DNA polymerase is Phi29 DNA polymerase or a chimeric form of Phi29 DNA polymerase, optionally wherein the chimeric form of Phi29 DNA polymerase is QualiPhi DNA polymerase.
